# EUROPEAN PATENT APPLICATION

(11) **EP 2 305 807 A1**
(43) Date of publication of application: **06.04.2011**
(21) Application number: 09762576.8
(22) Date of filing: 11.06.2009
(51) Int. Cl.: C12N 15/09, C12Q 1/25, C12Q 1/68, G01N 33/53

(54) **METHOD FOR DETECTING OR QUANTIFYING DNA**

(30) Priority: 11.06.2008 JP 2008152619
(71) Applicant: Sumitomo Chemical Company, Limited, Tokyo 104-8260 (JP)
(72) Inventor: TOMIGAHARA, Yoshitaka, Toyonaka-shi Osaka 560-0013 (JP); SATOH, Hideo, Ibaraki-shi Osaka 567-0826 (JP); TARUI, Hirokazu, Toyonaka-shi Osaka 561-0802 (JP)
(74) Representative: Duckworth, Timothy John
(86) International application number: PCT/JP2009/061068
(87) International publication number: WO 2009/151150

(57) **Abstract**

The present invention relates to a method for quantifying or detecting DNA having a target DNA region, and so on.

SEQUENCE LISTING

<110> Sumitomo Chemical Co., Ltd.

<120> Method for determining or detecting DNA

<130> S21066WO01

<150> JP2008-152619
<151> 2008-06-11

<160> 63

<210> 1
<211> 2661
<212> DNA
<213> Homo sapiens

<400> 1
acagacatgt gccaccatgc ccagctaatt ttttgtttgt ttgtttgttt gtttgtattt 60
ttagtagaga tggggttttg ccatgttggc caggctggtc tcgaactcct gacctcgaat 120
gataatgatc cgccgcttgg cctccaaagt gctaggatta caggtgtgag ccactgcgcc 180
aggcctgggc actttcttta gtagtttgag gagcaacatt tttgacagtg tccttctgct 240
caagattcaa gatcccagat aaaattaaac catctagaga gatggcttga ttggccaaac 300
ctggatctca tgaccacttc ttgaagtggg taagtctcat aaatgctcag tccttccact 360
atgcaactga gtggggtggg tgggaagccc ctcaaaggaa aatccggttg ttcttactag 420
aaagaaaagg aaaatggatg tgaggcagtc aaaatcagca gaggtccacc acaccaccaa 480
aatgtggtga ttaaatatgg agagacagag actaacagag gtatgtgaat attgaagtat 540
gtctggacaa tagcccaatg atgagaccaa taaaatggtt accaaaatct ggttttgagt 600
agtagtgtta aatcagacca tttagtaacc atttttttgtt gcaaagtttc tagcactgcc 660
caaaccctga gtggtatatg aataactcgt ccattatgta tctctttcca gtcagcataa 720
tttatccccc acctatattc ttttctgacc actcctactt ccttctcttt accaaaatct 780
aaactctaag gctgtttctt cagcaacttc tttgtttaga ttggaagata aattaaacag 840
catgcgatgt tttactgact ttcagtattt aacagaggtg atttaatttt tttttaaatc 900
caaagtcaaa cttctttata agatgaagga gaaaaatgtc ttataaaatg catatgtgaa 960
gatgccttct gagtgctttc tcatgcagac ttgttctagt ctttaatgaa tcttccttgt 1020
agacactgtg gagatgaaag atggttctcc acttctactc aaagtacaaa tcaggccggc 1080
attttgaaaa agagacaggt ttattcatag ctgcagcgtt agctggcttt gttccctgta 1140
caatttcact tttggttatt aaaatattca ctgtaggaaa taaatttgta acccatttct 1200
catattacct acacacagaa aaacaaaatt tgatatcctg ggtttatttt gctgagggcg 1260
cttcccataa aagcgagaga gtgtgcgttg ggaaatgtgt ctggttaact cttttatgga 1320
taaactttag tcacaatcct cccccgcccc cctctcaccc ccagcaccct cccaacctcc 1380
cgacttcccg cctctcaagg gctggtgacc taatagcatt tttcttcgtg catattttgg 1440
cgtcgcccca tggcctggct gccttcgcct gtctgagttt tttgaaattc ctgcatgttc 1500
gccccagatt aagccagtgt gtctcaggat gtgtgttccg ttttgttctt tccccttaac 1560
gctccctgtg caacgtgtct gggggggagga gggcagggac gggagagagg gaggggcaga 1620
ggcgaggagc tgtccgcctt gcacgtttcc aatcgcatta cgtgaacaaa tagctgaggg 1680
gcggccgggc cagaacggct tgtgtaactt tgcaaacgtg ccagaaagtt taaatctctc 1740
ctccttcctt cactccagac actgcccgct ctccgggact gccgcgcggc tccccgttgc 1800
cttccaggac tgagaaaggg gaaagggaag ggtgccacgt ccgagcagcc gccttgactg 1860
gggaagggtc tgaatcccac ccttggcatt gcttggtgga gactgagata cccgtgctcc 1920
gctcgcctcc ttggttgaag atttctcctt ccctcacgtg atttgagccc cgtttttatt 1980
ttctgtgagc cacgtcctcc tcgagcgggg tcaatctggc aaaaggagtg atgcgcttcg 2040
cctggaccgt gctcctgctc gggcctttgc agctctgcgc gctagtgcac tgcgcccctc 2100
ccgccgccgg ccaacagcag cccccgcgcg agccgccggc ggctccgggc gcctggcgcc 2160
agcagatcca atgggagaac aacgggcagg tgttcagctt gctgagcctg ggctcacagt 2220
accagcctca gcgccgccgg gacccgggcg ccgccgtccc tggtgcagcc aacgcctccg 2280
cccagcagcc ccgcactccg atcctgctga tccgcgacaa ccgcaccgcc gcggcgcgaa 2340
cgcggacggc cggctcatct ggagtcaccg ctggccgccc caggcccacc gcccgtcact 2400
ggttccaagc tggctactcg acatctagag cccgcgaacc tggcgcctcg cgcgcggaga 2460
accagacagc gccgggagaa gttcctgcgc tcagtaacct gcggccgccc agccgcgtgg 2520
acggcatggt gggcgacgac ccttacaacc cctacaagta ctctgacgac aacccttatt 2580
acaactacta cgatacttat gaaaggccca gacctggggg caggtaccgg cccggatacg 2640
gcactggcta cttccagtac g 2661

<210> 2
<211> 1953
<212> DNA
<213> Homo sapiens

<400> 2
   tataaattcc acgcaggcat tgaattgaat ttgttcttaa ccaaatgcgt tttatctata 60
   cctggcagga atctagaagt gaaattacaa gatttatttc attttaattc tattatgaag 120
   catttaatca caaataccct gaaaatgaaa agataattta tcattttacc ttgactgagc 180
   aactctcctc acttcacatt catgaatcca taacgcagag aggagactgg atgattaagt 240
   gtttgattag agaaaacaga ttaacctagc aaacataata aatttggctc ataagcagga 300
   tggctttata aatgctcaca atacctctcc tgtataaaat catgaaccac ttcctacagt 360
   gatgactcca tcgaaatagt tgagaaacat aaagcaaatg catgtttatg gctttctctt 420
   tgagacatta aaagggtatt gaaaggcata tctgattcag cttataactc tggatatata 480
   ttaaggaaca tgtaagaaaa tattaatgca taaaaaaagc tacaacttct caagtgttct 540
   agtttccact ttgtcaataa ttacgttttc aatgtccttc tgtggactgt ttccaaaggt 600
   gccaatccag acccaaagtt tcagatcact cagattcacc cttaaccttc ataacacaac 660
   ccaatagctt tacgaaaaaa gttgcatatt taggtagttg ttatcccatt atgacaaaat 720
   acataaaatt agcgagatat tttttagcct tcaaataagt gggaaaaaat ccttttagct 780
   gagattccat ttacatcaga ataaaaatct aagttatgac taggttgaag caacgtcctg 840
   tgcagcgctc cataaagttc acttagtctt caagggttcc ttacttagct aggttagtat 900
   tcctggcctc tttttttagc agtgagaaaa aggatactct ccctgcccca gctttatttt 960
   taaactcaca gccatatcct ggaggtctct gctggctatt tggcgcgtgg gggcggaggg 1020
   gggccggggg aggggggcgg ggcggggtct ggaggtctgt gctggctatc tggcgtgtgt 1080
   gtgtgtgtgt gtgtgtgtgt gtgtggttgg aggtctctgc tggctatctg gcgtgtgtgt 1140
   gtgtgtggtg tggtgtgtgt aagcagtgag gttgttttag ggccagtcct tcctccgcca 1200
   ctttgctgac tcaaagaccc agaggctttc ttggggtgca ggtaccatga ttccttgggc 1260
   cctaagggaa tttttgttag gctagaagag tgggtgtact catgatgggt gtacccgaac 1320
   attcctgggc tcaacaaaac cgattatctt tataaccgcg gcgcctagca cagcgcctgg 1380
   tgccctaaac gttggctgcg ggaacgtccg agacgcgggt gcggagccgg gggcggaata 1440
   actggttgcg cggcgctttg accgtaggcg ctggagcgcg tgcgttgcgt gcgcgcgcgg 1500
   aggcggctgc gtcggggcgc gagaaggtgc agttccccgg cgggcgggcg ggcgggcggg 1560
   cgaagctggg ctcggggcca agcgaggtct agccggagcg actgtgcccc gcctcctggg 1620
   cggagcgggc ggctccccat ggtcagagcc tcgtgccggc tcggcagcgc ccggacgccg 1680
   agcccagcgc gtcggccccc cggcgtgcgg gcgtctcaga gccgcggagg ggccgccggg 1740
   accgtttcag cgtggcggcg ctggtgctgg cgttggccct ggaggacggc cccgagtgat 1800
   ggctggcgcc tgcctcccgg gtgtctcccg ggtacagatg gagtcgtccc gcggccgccg 1860
   gcggcaaggt cggcagctgc gaggccaaga gagaccccag gacacacaca gctgcctccc 1920
   ggtgcgagaa gaagaccccg gcttgagagt gag 1953

<210> 3
<211> 889
<212> DNA
<213> Homo sapiens

<400> 3
cggccccatg gctccgtgtc gtgtccaagg gatgggctgg cacctcttgg accaggctta 60
ccaccagggc ccttctctga agccccagtc tgaccggcct gctgctggga atccccctct 120
gcccccacac taacctctgc tggggctgag ccagggcgcg tcggacagtc agggcgaccc 180
agccagggcg accgttggcc ccgctcctat ggggcagcag ggaccgacgt cagcagggtg 240
gggcgggcac ccgagtggta tgccccgccc tgccccgcct gcccgccctg gtggccgtct 300
gggggcgaca agtcctgaga gaaccagacg gaagcgcgct gggactgaca cgtggacttg 360
ggcggtgctg cccgggtggg tcagcctggg ctgggaggca gccccgggac acagctgtgc 420
ccacgccgtc tgagcacccc aagcccgatg cagccacccc cagacgaggc ccgcagggac 480
atggccgggg acacccagtg gtccaggtgt ggcgggggtg aggggagggg gggtgggagc 540
ggtggagatg gggccgtggg gagggagctg agatactgcc acgtgggacg atgctaggtg 600
gggagggctg agctgggcgg gctcctctgg ctgtggggcc ccctgtgttc cttgtgggag 660
gtggaaggaa gtgagtgccc tgtccttcct ccctgccatg agattccagg accggacctg 720
gcaagtgccc tatcccagcc agtgttcctg gggctcttcc aggcagggct atgttcccca 780
ggccaggggc attgtcctgg acagtcagga ggcatacccc tcgccaggtg gaaccaccct 840
gtgtatgcat gaccctgaca agcaggcgcc aggacagtca ggaggccag 889

<210> 4
<211> 863
<212> DNA
<213> Homo sapiens

<400> 4
gttgttgggt gtgaatggag aactgtgggc cctccccgac accttccagc gggacggcaa 60
cgggggccca gggggtgggc gccatcaacc ccgtcccacc gccaggacgg cgcgggggag 120
ggccggcggg ggcggggcgt cctgtaaggc gcggccccca cccgcgggcg gggcggcatt 180
cctgggaggc cggcgctctg acgtggaccc gggggccgcg ggcacggcgg gggggcggcg 240
gtccgggggc ttcttaaacc ccccgccccg gcccagcccg cacttcccga gcaccgctcc 300
gaccctggag ggagagagag ccagagagcg gccgagcgcc taggaggccc gccgagcctc 360
gccgagcccc gccagccccg gcgcgagaga agttggagag gagagcagcg cagcgcagcg 420

agtcccgtgg tcgcgcccca acagcgcccg acagcccccg atagcccaaa ccgcggccct 480
agccccggcc gcacccccag cccgcgccag catgatgaac aacagcggct actcagacgc 540
cggcctcggc ctgggcgatg agacagacga gatgccgtcc acggagaagg acctggcgga 600
ggacgcgccg tggaagaaga tccagcagaa cacattcacg cgctggtgca atgagcacct 660
caagtgcgtg ggcaagcgcc tgaccgacct gcagcgcgac ctcagcgacg ggctccggct 720
catcgcgctg ctcgaggtgc tcagccagaa gcgcatgtac cgcaagttcc atccgcgccc 780
caacttccgc caaatgaagc tggagaacgt gtccgtggcc ctcgagttcc tcgagcgcga 840
gcacatcaag ctcgtgtcca tag 863

<210> 5
<211> 2198
<212> DNA
<213> Homo sapiens

<400> 5
aagagaggca cactccctct accacaccga gggagggggc gttgagctga gaaaggttga 60
gagaatgagg gacccaggta ggtggacatc ggccaagaaa ggaaccacag cgggaggtaa 120
gaccgagagt ccccagcttg aagcgtcacc actccgggat tcccagattc caacgcgagc 180
ctggggaaag cccacagtgg agagagtccg gctggcaggg aatggcccta cccccggggt 240
gaaatctcgg agggtcgtgc agccgagtcg cgcctctgcg ctgatgcgtg agagatgccg 300
gacgtcgcgt ttgcctgtgc gagcctcgcg gatgctgtgc agtcttggtc ccctctgcgt 360
gtgtctaacg ccgaatgctg gtgtctcgag gtgtgagctt cggggccggt gtctttaaag 420
aaccaaagat tcttaaggag tgatgatctg ggtagagcgg cccgacgtag ccgcgctccc 480
aggtctcggt gcgagtcctg cggacagacc agaggagacc tgctggccag atgccccggg 540
cccaaggcgg acgccagact gtctctgcgc cagccgggct ggccttcgga atggatcagg 600
cacccgggag gccggagtgg atctcagacc ctcaagccgg gaacaaaccc gtcgatgccc 660
gtgggcctgg agtccgcctc ctccttcccg ccccacccct acccctgcct ccgaaaggct 720
tcttcgctgg tcagtagctg cgtgcccgtc tgcctgaggc tgggtcagaa ttggcgggct 780
ggtaacgacc ccgtgcacaa gcggctccca gtctctccag aaagggccga tgactaaggg 840
gtgggggtgg gggcggaggg ctggaaggtg ttagggaaga acgttagcgg cctatcctgt 900
cttcagcagc gccctctcat cttctagctc tgacgccgag cagagcagtt ggagctcggg 960
actgggaact gctggaattc ctatttagac ttctagacag tctagaaaca agaacctttc 1020
tttccctggg cctcagtttc cttgtctgta aaatcaaaag gcgggctcta ggtgtaggcc 1080
ttcttttcgc ttggtgattc tggattcctt tccttggatc cgtggggagg gggtggcagc 1140
aacagtccag ggcgttggcc gtcctgtgcc tcaagtacgt agtccccgtg cccgccccct 1200
caacaccccc agcagcccgc ccccctaagc ccgcagagca gggagctgag tgggaggggc 1260
agaggcgggg ccggttccca gtccctgctg gcggactaga gtggcgcggg ctgagcgtaa 1320
aacctgggat agccactccc ccttttcctt atccccgccc ccctgccatt ggctcccggg 1380
agaggttgac atcaaagccg cggtcttata taagccagat ccgcagggga gtccgcagaa 1440
gggttaaaca ggtctttggg cttcggcgac ctcgcccgcg gcagaaaccg gtaagaagac 1500
agtgggctgc gcgtctcatt ttcagccttg cccggactct cccaaagccg gcgcccagta 1560
gtggctccag agcccacagg tggcccccgg cagtctctgg ggcgcatgga gcggcgttaa 1620
tagggctggc ggcgcaggcc agtagccgct ccaacatgaa cctcgtgggc agctacgcac 1680
accatcacca ccatcaccac ccgcaccctg cgcaccccat gctccacgaa cccttcctct 1740
tcggtccggc ctcgcgctgt catcaggaaa ggccctactt ccagagctgg ctgctgagcc 1800
cggctgacgc tgccccggac ttccctgcgg gcgggccgcc gcccgcggcc gctgcagccg 1860
ccaccgccta tggtcctgac gccaggcctg ggcagagccc cgggcggctg gaggcgcttg 1920
gcggccgtct tggccggcgg aaaggctcag gacccaagaa ggagcggaga cgcactgaga 1980
gcattaacag cgcattcgcg gagttgcgcg agtgcatccc caacgtgccg gccgacacca 2040
agctctccaa gatcaagact ctgcgcctag ccaccagcta catcgcctac ctgatggacg 2100
tgctggccaa ggatgcacag tctggcgatc ccgaggcctt caaggctgaa ctcaagaagg 2160
cggatggcgg ccgtgagagc aagcggaaaa gggagctg 2198

<210> 6
<211> 1945
<212> DNA
<213> Homo sapiens

<400> 6
ctggatgaca gagtgagact ccgtctcaaa aaaaaagctc catttgggag gccgaggagg 60
gtggattacc tgaggtcagg agtttgagac cagcctggcc cacataggga aaccccatct 120
ctactaaaaa tacaaaaatt agtcaggtgt ggtggctgac acctataatc ccagctactt 180
gggaagctga ggcagggaga atcacttgaa ccggggaggt ggaggttgca gtgagctgag 240
atcatgccac tgcactccag cctgggcgac agggtgagat tctgtctcaa acaaacaaat 300
ttaaaagctc cgaatcctcc aaaaatacca agattttcct gtcggtaact agagatgggt 360
actgatgatt atttttaata ggtgattttc aaagatgtga acgttatcca tggagattta 420
agtctccaaa aggaaaaaaa atgcatacct ttatactaaa acttcatcac cagtcaaatt 480
tggatcatca ctaaattggc ttctacacct ctctcctaat ataaggtact tgtgtaagtt 540
tgcagttgtg agacacttat ttcctcattt ttaatgtctt ctcagtaggg ccactgatat 600
agtcactatt tgactgacca gaatggttgg cactggtgat tggctcataa agtgccctcg 660

atttaggggg ctcaattatc aaaggtttaa atcctagccc aaaccattgc tgtgatgggg 720
gttaatcaat gaaccactca gcttcacttg caaaagcggg atcacaatag ccgctttcgt 780
catgacccag cctaggtgag atttagtact taagtacact gccaggcaca caaggttaat 840
ttaacaattt aacacatttg tttcctcatc catttctcca aaccttccaa ctaatcctaa 900
cgttcgttcg gccaaatggg ccaggaattc acttaaacaa aaacaaaaaa caaaacaaac 960
aaaaaaacac tccctggggc ttggggaagg aggcaccgcc gcccatgtcg cagtctgggg 1020
gtggctcagt cctcagcacc cagatctacg gccataatgc tcttcgaggc caaggagccc 1080
ggatgcgggg cgttgccgaa ggcgtcttgc tcaggctgcg ggaaaggaga ggggtgggag 1140
cggggtgggg gcatcgcgac ccagggcaag gcggcgagtc gccgtcttcg agtcccacct 1200
gtccgaagcg gggtgagaaa aggcaaaaca tggcaaagcc atgcacctcc cagggtgggc 1260
aactcacggc cggtgaacgc cggaccctta gcagtttcca gacctttgga accggaagcg 1320
gagcctgaga gcgcgcccga gagggcgtga acgggaccgc tttcccggaa gtgcttgcgg 1380
cctctgccca gcgagctgcc ccggggtctc tctggtttcc taatcagggc aacgccgcgg 1440
gagagaacct ttaccttggc tgcactaagt tctcggtgcc actccctggc agggcgggac 1500
cttgtttagg ccctgtgatc gcgcggttcg tagtagcgca aggcgcagag tggaccttga 1560
cccgcctagg gcgggaagag tttggcccgc cgggtcccaa agggcagaat ggacgggctc 1620
ctaaatccca gggaatcctc taaattcatt gcagaaaaca gtcgggatgt gtttattgac 1680
agcggaggcg tacggagggt ggcagagctg ctgctggcca aggcggcggg gccagagctg 1740
cgcgtggagg ggtggaaagc ccttcatgag ctgaacccca gggcggccga cgaggccgcg 1800
gtcaactggg tgttcgtgac agacacgctc aacttctcct tttggtcgga gcaggacgag 1860
cacaagtgtg tggtgaggta cagagggaaa acatacagtg ggtactggtc cctgtgcgcc 1920
gccgtcaaca gagccctcga cgaag 1945

<210> 7
<211> 2379
<212> DNA
<213> Homo sapiens

<400> 7
aagcttgtgg tttacttgga cctctgcctc atctttcttc ttttgcgctt cagcctgcgc 60
attcgcttcc tccactaggc tctcatggtg cagaggtttc caagaagatg gtgtgaaggc 120
cgagatcatt tggttatatt ataaaataga atgcaaattc acacaagttt ttgtttttta 180
tttatttatt tttttagaga tgaggtcttg ctatgttgtt tagtctggtc tcgaactcct 240
ggcctcgtga tcctcccacc ttgacctccc aaagtgctgg gattacaggc ctgaggcctg 300
agccactaca cccaactgaa ttcacatttt tttttttctt ttctgagacg gagtctcact 360
ctgtcaccca gtatggagtg cagtggcgcg actgcggctc actgcaagct ccgtctctcg 420
ggttcaagtg attctcatgc ctcagccccc caagtagctg gaattacagg ggtgcactac 480
cacacctggc taatttttct gttttagtag agatggggtt tcaccatgtt gcctggtctc 540
aaactcctga ctttaagtga tccacacacc tcagcctccc aaagtgctgg gattacaggt 600
gtgagcctcc acacccggcc gaattcacat gaattttaaa gtgatgtctt caaagtggtt 660
tcactgtggg gatgggcagc tttttgttat acatctagaa cgttcctctt ctgtttctat 720
gaatactcgg ttggaaaggg ctgaaaaacg gtcttaagag attatctgat tcgtttccca 780
gtttatttac tcacatatca gctgtaattt gagcacgttt tctgattgag acaagactca 840
gatggtatta aacattacta caacacatcc gggcacggtg gctcacgcct gtaatcccag 900
cactttggga ggccgaggcg ggcggatcac gaggtcagga gatcgagacc atcctggcta 960
acacggtgaa gccctgtctc tactaaaaat acaaaaaatt aggcgggcat ggtggcgggc 1020
gcctgtagtc ccagctactc gggaggctga ggcaggagaa tggcgtgaac ccgggaggcg 1080
gagcttgcag tgagccgaga tcgcgccact gcactccagc ctgggcgaca gagcaagact 1140
ccatctcaaa aaaaaaaaaa aaaaaaaaaa actacaacac tataaattca tatctattat 1200
aatagtactt tgtgcagggc cctaccctaa gtccttaacc gaacccggaa gcgagaagat 1260
gacttttgtt tgtttttaga gatgggcgcc tggctctgtc gccagcctgg agtgtggggg 1320
cgcgatctcg actcacagca gcctccacct cccgagttca ggcgatcttc ctgcctcagc 1380
ccctcgagga gctgggacca ccggcgcgct ccatcgcgcc cggctaggag ctgactttga 1440
atccgggctc tgcgcctggc cttctgcatc tctataaggg aagacatctg tgacctcggg 1500
gcaaaggtca aattagatcc tgggtaggat cctgttcccg ctgcccctcg ggctggcact 1560
gccaggagta ctcagagctc aaagctggga tctgcagtcc cttacccact cagtgcacgc 1620
cgcctaaggc tttgcgcttc acctttactc acctcgaagc cctggacatc cgcatctgcc 1680
ctaagacttc tcacctcagt agcagaagga agtcgcgtca gctggccaca gcctctctcc 1740
taggagaccg tccgggaaaa gcgagtcagg gtagaccctg aggcccctca gctccggcta 1800
ttttcagatc tgtcgctcct tcaccctcag cctttcaaac aggccactcc aaaaaaaagc 1860
ccaatcacag ccttccttct tctcctggcc ttccggcact gtccaatcaa cgtacgccat 1920
ctatcggtta gtggtgttgc ggggccaccc ttcccgctgg tttccctcgt ggtgtgtaaa 1980
ggcagagagg aaaggcgagg ggtgttgacg ccaggaaggt tccatcttgg ttaagggcag 2040
gagtccctta cggacttgtc tgaggaaaga caggaaagcg ccagcatctc caccttcccc 2100
ggaagcctcc ctttgccagg cagaaagggt ttcccatggg gccgcccctg gcgccgcgcc 2160
cggcccacgt acccggggag gccgggcccc ggaggacgag ggaaagcagg ccgggcgccg 2220
tgagcttcgc ggacgtggcc gtgtacttct ctcccgagga gtgggaatgc ctgcggccag 2280
cgcagagggc cctgtaccgg gacgtgatgc gggagacctt cggccacctg ggcgcgctgg 2340
gtgaggccgg gccctccggc cgggaccccc agtccgtcg 2379

<210> 8
<211> 933
<212> DNA
<213> Homo sapiens

<400> 8
gagacgtact ctggctctgt cgcccaggct ggagcgcaat ggcgccatct cggcgcactg 60
caacctccac ctcccgggtt caagcgattc tactgcctca gcctcccgag tagctgggac 120
tacaggcgcg cactaccaag cccggctaat ttcttttgta tttttagtag agactgggtt 180
tcacgatgtt ggccgggctg gtctggaagt cttgacctca agcgtgcgcc ctctccgcca 240
ctgggtaagg cggggggcgg aatagggggc ttgcaatttc acactagagg cgggcgccgt 300
gggggaaaga agagtcacgt ctcccacggt tcgtagagga aggcctgcct gagcctggag 360
cgggggcggg agagccacag tttggcatcc ccagggcatc ccccagcccg cagactacca 420
ggcctccaga ggacaggacc ccacccccgg ccacaggccc tgcccccagc actccccgca 480
ccccgcctcc aagactcctc cgcccactcc gcacccaact tataaaaacc gtcctcgggc 540
gcggcgggga gaagccgagc tgagcggatc ctcacacgac tgtgatccga ttctttccag 600
cggcttctgc aaccaagcgg gtcttacccc cggtcctccg cgtctccagt cctcgcacct 660
ggaaccccaa cgtccccgag agtccccgaa tccccgctcc caggctacct aagaggatga 720
gcggtgctcc gacggccggg gcagccctga tgctctgcgc cgccaccgcc gtgctactga 780
gcgctcaggg cggacccgtg cagtccaagt cgccgcgctt tgcgtcctgg gacgagatga 840
atgtcctggc gcacggactc ctgcggctcg gccaggggct gcgcgaacac gcggagcgca 900
cccgcagcca gctgagcgcg ctggagcggc gcc 933

<210> 9
<211> 6096
<212> DNA
<213> Homo sapiens

<400> 9
atctgcacct cctcatatag ggttgatcca agtttcacag acatcactga gttcttagtg 60
gactcagcta ttggggctgt tctcacactt tttttttctt tgcaagaatc agcaatgggt 120
gcaagtggac ctgtgtagga cgtccagtga aacattgtgt tggtgaatca gctagaatcc 180
atccaagaac tcagccagcc tggtgtgggg tgagatctga tccttgaatg tccctcagtg 240
gcttttaggg ctggcaggtt cagaagggcc ctctcatcac ccccccaggg cctcattcct 300
tgtttaacac tttgctatca cagtcttgaa tccttgtaat tgaacaatgg accccacatt 360
ttcactttgc actggtttct gattctgtaa ccgatcctgt ccccctctct tgtctcattc 420
actctgggaa ttgtccccac attctgagac ctttcagcag tgccccaacg aggttcctgc 480
ccttatctga agctccaccc tcacccccat ggcggcaccg caggcagccc tgcttttgcg 540
tcccgcgtag gcaggctgtg caccggagtc acgacccccct gattcagcct aggcagccac 600
agcttgactg ctcccgccgg acaagcccta ctgtgctatc tgccgctctt cccttcctct 660
tcccaggggg tccgcgtcag ggaggcgca gctgtgtgca ttccgggagc ttcagacccc 720
cgtgtccagc agctccttcg tttcctgggt gctggggcgg ccttcccagc gaagagctca 780
actcagcggg acgtttggag gctctctgcc ccaaggcgct ggggagtgtg cggcgggaca 840
gtcgtgcttg cctttttcac tttcagagtg tccacgcccc acccgtttgg tcactgcagg 900
tcagtccagt ccagcccggc ccaccccacc ggtgcgtgtc tgtcgcacgt ggcagacgcc 960
atactctctg ttcttgttta aagcccagga tctactgggc cctggaggca agaggtgaac 1020
gcagcggaat ccacgctgag ctgcccggga acggagcttc caaccccaga aggaggactc 1080
tgtgctccta caccttaacc ctttttagcc cgaaacttct ccaacttcct tggctttgtt 1140
tagagctcga cagcgccgcc ccctggcgct cgttgtgagg acagtagagg agagaggcaa 1200
gggtgttttt aaacagtttg cctctcacca ttatgggggc gacccgaggg ggagacccac 1260
tcttccgcat tcccggtaag tgaaccaccg gaagaggtcg aaagtgacgg attcccatgt 1320
cctcctccag cccccccccc accctgccca tccacaggac ggtggctctt cagtgccctt 1380
tgccgagcaa gtggcgtttc tatgcacgtg ggtatcaatt cggactctgg acgaaatgga 1440
aacctcctta gccgacccgg gtgggatcag ctgggatcct gcgcgctccc ctggggggtt 1500
gccagccact ctgttggggt gcaagaagca ccatccttcg gaagctgggc cgaaactggc 1560
caggctgact cgctcccacg cgcccgcccc tacccggcgc cgcagcaatt cacctgccac 1620
cgcctctgag ccgggtccgg acttcggcgc cctgacagtg tccccgcgac ttccccaccc 1680
gatgagatgg ggtctggcgt tggccagtgc gtgtccaggg actcgcgggt ccctggccag 1740
ccatggggca gagggcgctg gtgttaggcc agtcttcccc accctgcccc gtcaccccag 1800
ccacacccac tgtcctgtga ggccaagcgc gctccgctgg tttcctgagc caggcacctt 1860
ggccgcggac aggatccagc tgtctctcct tgcgatcctg tcttcgggga agtccacgtc 1920
ctaggcaggt cctcccaaag tgcccttggt gccgatcacc cctcccagcg tcttgcaggt 1980
cctgtgcacc acctccccca ctccccattc aaagccctct tctctgaagt ctccggttcc 2040
cagagctctt gcaatccagg ctttccttgg aagtggctgt aacatgtatg aaaagaaaga 2100
aaggaggacc aagagatgaa agagggctgc acgcgtgggg gcccgagtgg tgggcgggga 2160
cagtcgtctt gttacagggg tgctggcctt ccctggcgcc tgcccctgtc ggccccgccc 2220
gagaacctcc ctgcgccagg gcagggttta ctcatcccgg cgaggtgatc ccatgcgcga 2280
gggcgggcgc aagggcggcc agagaaccca gcaatccgag tatgcggcat cagcccttcc 2340
caccaggcac ttccttcctt ttcccgaacg tccagggagg gagggccggg cacttataaa 2400
ctcgagccct ggccgatccg catgtcagag gctgcctcgc aggggctgcg cgcagcggca 2460

agaagtgtct gggctgggac ggacaggaga ggctgtcgcc atcggcgtcc tgtgcccctc 2520
tgctccggca cggccctgtc gcagtgcccg cgctttcccc ggcgcctgca cgcggcgcgc 2580
ctgggtaaca tgcttggggt cctggtcctt ggcgcgctgg ccctggccgg cctggggttc 2640
cccgcacccg cagagccgca gccgggtggc agccagtgcg tcgagcacga ctgcttcgcg 2700
ctctacccgg gccccgcgac cttcctcaat gccagtcaga tctgcgacgg actgcggggc 2760
cacctaatga cagtgcgctc ctcggtggct gccgatgtca tttccttgct actgaacggc 2820
gacggcggcg ttggccgccg gcgcctctgg atcggcctgc agctgccacc cggctgcggc 2880
gaccccaagc gcctcgggcc cctgcgcggc ttccagtggg ttacgggaga caacaacacc 2940
agctatagca ggtgggcacg gctcgacctc aatggggctc ccctctgcgg cccgttgtgc 3000
gtcgctgtct ccgctgctga ggccactgtg cccagcgagc cgatctggga ggagcagcag 3060
tgcgaagtga aggccgatgg cttcctctgc gagttccact tcccagccac ctgcaggcca 3120
ctggctgtgg agcccggcgc cgcggctgcc gccgtctcga tcacctacgg caccccgttc 3180
gcggcccgcg gagcggactt ccaggcgctg ccggtgggca gctccgccgc ggtggctccc 3240
ctcggcttac agctaatgtg caccgcgccg cccggagcgg tccaggggca ctgggccagg 3300
gaggcgccgg gcgcttggga ctgcagcgtg gagaacggcg gctgcgagca cgcgtgcaat 3360
gcgatccctg gggctccccg ctgccagtgc ccagccggcg ccgccctgca ggcagacggg 3420
cgctcctgca ccgcatccgc gacgcagtcc tgcaacgacc tctgcgagca cttctgcgtt 3480
cccaaccccg accagccggg ctcctactcg tgcatgtgcg agaccggcta ccggctggcg 3540
gccgaccaac accggtgcga ggacgtggat gactgcatac tggagcccag tccgtgtccg 3600
cagcgctgtg tcaacacaca gggtggcttc gagtgccact gctaccctaa ctacgacctg 3660
gtggacggcg agtgtgtgga gcccgtggac ccgtgcttca gagccaactg cgagtaccag 3720
tgccagcccc tgaaccaaac tagctacctc tgcgtctgcg ccgagggctt cgcgcccatt 3780
ccccacgagc cgcacaggtg ccagatgttt tgcaaccaga ctgcctgtcc agccgactgc 3840
gaccccaaca cccaggctag ctgtgagtgc cctgaaggct acatcctgga cgacggtttc 3900
atctgcacgg acatcgacga gtgcgaaaac ggcggcttct gctccggggt gtgccacaac 3960
ctccccggta ccttcgagtg catctgcggg cccgactcgg cccttgcccg ccacattggc 4020
accgactgtg actccggcaa ggtggacggt ggcgacagcg gctctggcga gcccccgccc 4080
agcccgacgc ccggctccac cttgactcct ccggccgtgg ggctcgtgca ttcgggcttg 4140
ctcataggca tctccatcgc gagcctgtgc ctggtggtgg cgcttttggc gctcctctgc 4200
cacctgcgca agaagcaggg cgccgccagg gccaagatgg agtacaagtg cgcggcccct 4260
tccaaggagg tagtgctgca gcacgtgcgg accgagcgga cgccgcagag actctgagcg 4320
gcctccgtcc aggagcctgg ctccgtccag gagcctgtgc ctcctcaccc ccagctttgc 4380
taccaaagca ccttagctgg cattacagct ggagaagacc ctccccgcac cccccaagct 4440
gttttcttct attccatggc taactggcga gggggtgatt agagggagga gaatgagcct 4500
cggcctcttc cgtgacgtca ctggaccact gggcaatgat ggcaattttg taacgaagac 4560
acagactgcg atttgtccca ggtcctcact accgggcgca ggagggtgag cgttattggt 4620
cggcagcctt ctgggcagac cttgacctcg tgggctaggg atgactaaaa tatttatttt 4680
ttttaagtat ttaggttttt gtttgtttcc tttgttctta cctgtatgtc tccagtatcc 4740
actttgcaca gctctccggt ctctctctct ctacaaactc ccacttgtca tgtgacaggt 4800
aaactatctt ggtgaatttt tttttcctag ccctctcaca tttatgaagc aagccccact 4860
tattccccat tcttcctagt tttctcctcc caggaactgg gccaactcac ctgagtcacc 4920
ctacctgtgc ctgaccctac ttcttttgct cttagctgtc tgctcagaca gaacccctac 4980
atgaaacaga aacaaaaaca ctaaaaataa aaatggccat ttgctttttc accagatttg 5040
ctaatttatc ctgaaatttc agattcccag agcaaaataa ttttaaacaa aggttgagat 5100
gtaaaaggta ttaaattgat gttgctggac tgtcatagaa attacaccca aagaggtatt 5160
tatctttact tttaaacagt gagcctgaat tttgttgctg ttttgatttg tactgaaaaa 5220
tggtaattgt tgctaatctt cttatgcaat ttcctttttt gttattatta cttatttttg 5280
acagtgttga aaatgttcag aaggttgctc tagattgaga gaagagacaa acacctccca 5340
ggagacagtt caagaaagct tcaaactgca tgattcatgc caattagcaa ttgactgtca 5400
ctgttccttg tcactggtag accaaaataa aaccagctct actggtcttg tggaattggg 5460
agcttgggaa tggatcctgg aggatgccca attagggcct agccttaatc aggtcctcag 5520
agaatttcta ccatttcaga gaggcctttt ggaatgtggc ccctgaacaa gaattggaag 5580
ctgccctgcc catgggagct ggttagaaat gcagaatcct aggctccacc ccatccagtt 5640
catgagaatc tatatttaac aagatctgca gggggtgtgt ctgctcagta atttgaggac 5700
aaccattcca gactgcttcc aattttctgg aatacatgaa atatagatca gttataagta 5760
gcaggccaag tcaggccctt attttcaaga aactgaggaa ttttctttgt gtagctttgc 5820
tctttggtag aaaaggctag gtacacagct ctagacactg ccacacaggg tctgcaaggt 5880
ctttggttca gctaagctag gaatgaaatc ctgcttcagt gtatggaaat aaatgtatca 5940
tagaaatgta acttttgtaa gacaaaggtt ttcctcttct attttgtaaa ctcaaaatat 6000
ttgtacatag ttatttattt attggagata atctagaaca caggcaaaat ccttgcttat 6060
gacatcactt gtacaaaata aacaaataac aatgtg 6096

<210> 10
<211> 2500
<212> DNA
<213> Homo sapiens

<400> 10
acccacttct gtgtgtggat agtatcctgc aggagagatg ttgtctgcag tgtgagctgg 60
gcccaccgga gtgtgtgaat aggatcctgc aggagaaatg gaatccggag tgtgagctgc 120

atccgctgta gagggtggat aaaatcctgc aggaaagatg gcatctggaa tgtcagcggg 180
agccaccgac ctctgaggat gcaccccgca ggtgtgatgc ggggccagtt ccaaggctgg 240
gttaggtttt accctggctt ctgtgttgta ctctcattct cttcctcttt cttctaatac 300
ctgctctggg aggcatcagg ccatgtccag tgtgcaggcc atggagaccc acacggcaag 360
gaactggaac cccctgccag cagcctcggg ggtccagtcc ttagatggtg ccctgtggtc 420
agcaatgcac ctgtgacctc cgggctatgt ctcgtggtag ttgcttttgt gttttaacat 480
agcaacagga aactagccta ttacccacca atcccattcc aggctgcttt caaacgcagc 540
tcaggctaga acaccagcac ggggacacag ctgagacttg gggtttgcga cgggaacacg 600
cccatgctgt gcctctgaat ctggcaccgt caccctgtgg cctgggttca gcaacttggc 660
ctcaccttcc ttgtctgtga aattcagact gggtccttgt gagatgattg gagagaatgt 720
atgaactatg tgagaacgcc acctttgtgc gtatctcacg cagtgtcttc cctcctttcc 780
aaagtcttct gctgtctcta gacacacccg acgtgggggg ggggggttcc ctgggtctcc 840
tcctaggtct gtcccaggag ggcacgcact gaaggccgcg agaatcccgg gggctgcatt 900
gcgccgcgcc aaggactcca cacaggacct ttcattttcc caactgtgct gagccaggcg 960
gccggcagag agcaggtggc tgacaggccc cggggagccg gaccgcctgg gtctaatctt 1020
cccgcagact ccccttgctgt gcgctttggg gcttggcct cagtttcctc aaaaggaatg 1080
aggggctttt ttggaacgtt aaataatttc ctacgtggtt gcgggtaggg agaaggagaa 1140
agagaggagc gcgcctgcgc gcctggaatc gtgcccggat cagagcaagc gctctaaaag 1200
tgttacaaac attaaggcgc caactaaaaa acccgtagtg agcgcaggca gaaaccacgg 1260
gtaagagaag tggagaagct tcgcgtaggc cccagggtcc cgagccccga gtctcgagcg 1320
cagaatcagg ggtgccaatg ctctcctccg cgcccccgag cgctcgcctt ggccatgcgg 1380
gccgccccac cgggatgagg gcgctcaggc cggacgctgg ggccccgggt tctcgccccg 1440
ccccgccctc ggggattcag aggggccggg aggagcctcg cgcatgtgca cagctggcgc 1500
cccccgcccc ccgcgcacag ctgggacgtg ggccgcggcc gggcgggcgc agtcgggagc 1560
cggccgtggt ggctccgtgc gtccgagcgt ccgtccgcgc cgtcggccat ggccaagcgc 1620
tccaggggcc ccgggcgccg ctgcctgttg gcgctcgtgc tgttctgcgc ctgggggacg 1680
ctggccgtgg tggcccagaa gccgggcgca gggtgtccga gccgctgcct gtgcttccgc 1740
accaccgtgc gctgcatgca tctgctgctg gaggccgtgc ccgccgtggc gccgcagacc 1800
tccatcctgt gagtgccgcg gggacgccg ggggcgcggg gtccggggct tcgtggagat 1860
ccgggagcgc aggggtgatc ggaggtgggg ggcgcggagg gtggaggggg catcgggcgc 1920
gcgggggcc tggggacttg ggacgcagaa gggaacctcc gaagggggac gtgggggac 1980
ctgggcgcgg ggacccgctg ggcctttgtt cgccctgcgg gagacgccga ggggcggaac 2040
agagcgctgt gcgcgcggcc ttcgtagccg cctttgttcg gaactcggaa tccccgcagg 2100
actgggaagt tgttggagcc tccggggctc cccccgctcg cctcccgccg ccccctctca 2160
tgctccgccg gcctcccgct tcccccctggt tcgcggcccc tcctccgctc acctttcccc 2220
cgctcaggac ccctcggtcc ccctccgctc cccgagcgcg gcgcagcccc ctccgtcctc 2280
ccagccccct ccgccccgtt cctcgtcctg ttcgctcccc tcctccgctc ctcttcctcc 2340
tccccttcct cctcctcctc cccttcctcc tcctcctccc cttcctcctc ctcctcccctt 2400
cccctcctcc tccccccctt ccttctcctc ccccagcctc cgccctctcc ccctcccccg 2460
cccccttggag cgcagtgccc accccatccc cccgcgccgg 2500

<210> 11
<211> 2200
<212> DNA
<213> Homo sapiens

<400> 11
cctcgccccc tccagccggc cccccgggcc cctcctctcg gcgcccggac cttggccctc 60
cctctccttt cccacttctc tctttgccct aacttcgccc ccatcccccg ctcatttcct 120
ctcgcacccg ggctcgccaa tccctctttc caagtccctc ttccagcccg gccttcctct 180
cgggttcgcc cccccttctcc ccaatctccg tcctcttccc tcccttcgcc ctcccccccct 240
tccttcctct tcccctcacc caaccctggt tcccctcgtt cctcagtccc gatctctccc 300
ttactctgtc cccgcccact ctgcgccggc ctctcagtcc gggttgagcc ccacgtgtgg 360
acggccgcgc ccccactgac agccgccgcc cgccggcccg ccccgcgccc cgccgggcct 420
ctaaaacccc cgcgccgcgc cctccaccgc cgcatcttct ccagcgccca gcctcccgcc 480
ctctctcttg ctggccgcac gccccggccc cgcgcacctc cgcccggctc cgcagccgct 540
acccgcgctt cgttgccctg tgggactccg agcgagcccg gagggaaccc tcctcttctt 600
ctgggggcga cttttgtttg cttgcctgtt tctttctggt gacttttgca gctttccaat 660
atccgtcttc ggagcgcacg ggaatccgcc gagctctgcg tgcaggccct tttttctttt 720
gaggttcaca tttttttgaaa ttttacgcca gggcttttgt aatttcctcc cccgcccgct 780
gacggtcctg gagtcgctcg gggctttagg ccggttatgc aacgtgtacc gctcggggct 840
gccggctgca cctccgccgc gcctcgccgc tcactgcgct agacccggcg ccccgcgtct 900
cgcttcgcgg gcagtcaggg ggccggcgct ctgtcgaggt ctccagctag agcagggagc 960
ccgagcccga gggagtcccc ggagccgacg aaggcttat tagaccctga ctcttttctg 1020
aggcgcgcag atttgtcttt tgatcactcc ctctccgcgg gtctacggcc gcgcgctttc 1080
ggcgccggcg atggggagaa gacggaggct gtgtctccag ctctacttcc tgtggctggg 1140
ctgtgtggtg ctctgggcgc agggcacggc cggccagcct cagcctcctc cgcccaagcc 1200
gccccggccc cagccgccgc cgcaacaggt tcggtccgct acagcaggct ctgaaggcgg 1260
gtttctagcg cccgagtatc gcgaggaggg tgccgcagtg gccagccgcg tccgccggcg 1320
aggacagcag gacgtgctcc gagggtaagt gggcaagcgg ctccgcacct agggctccgg 1380

cttgggggag gggggaatcc tcagtttggc ggctttctgg cccactccgt cccagaccct 1440
ttagctggag cctagagctg cagccccctt tgccagaata tccaaagacc cccaggagcg 1500
cgtccccctt ttccttccca accccgcagc tcagcgggcg gaaagccctc tctccggggg 1560
ttgggcggcg ggtggttagg gggtccaggg gtgccgatcg cagagcgtgt gcagagctcg 1620
cgctgcggga acaggttctg aatgtccggc ggcaggcggg cctgggtccg cctgctgcag 1680
gggccagaga agcctgcttg ctccccacgt cggggccgcc gctcgtgagc cttttgtttg 1740
aggacgtgtg cagggttcac agctcacctt ctcatcgtca acccgagcgc tccaccttgc 1800
gacgcgcttt ccttgacacg tcggggccaa agtaacagtt gaccaaggag gaatggattt 1860
gggaaggagg gcaaggattc tttggaacgg aatggtccct ttgttctctg catctggaag 1920
ctagaatagt agcaaattat atgtttccat gcctcttttc gccctttaaa aaggcaggca 1980
agggacgaca gatgaaaggc agtgtttaga catttctgac cctcctgcat tccagcatct 2040
agctcttttg cttccacgtc tgcctcccga tctccaataa tttgaagtgt aattttgatt 2100
tgtttgttgt cctgaaatct actcgctcgg ggcattgctt acgaagaccg tttatatgtt 2160
gctgcatccc tctacctatc tgttacgtga ccgcgcttgt 2200

<210> 12
<211> 2000
<212> DNA
<213> Homo sapiens

<400> 12
ttggaagaaa aggatctccg aggaaggggc tgagagaagg gcagggtgaa ctggactaaa 60
ggccagagta ggaaggagaa gaggggccaa aaaagaaggg gatgaaatta agcacagaag 120
atgggtaaag aaaaaagtat cagggaaagg gcaaaataag agaaagcctt gaggataaga 180
gggtagaagg ctaaagaaca aggggaccac tgggtcgggg aagcgctgcc tgaacggcgg 240
gacagtgaca aagaaagggc gctggcgata ttcgcaccaa gggtgcgaaa cgcaatcggg 300
aggtgagaaa tggaaagaag gcgaatgccc ggctacaagt agcctgggac tgaaagggga 360
cctgggggag gggctgggcc cagggcagaa aagtccaggt tcccatgcgg cctgggccca 420
cgtggagcgg gcgctgaatc accgttcagc cgcccccctc ccctcctccc cgaccggtgc 480
ccgcagtccc cgcctcctcg gccgccgcct ccacggggcg gggccctggc ccgggaccag 540
cgccgcggct ataaatgggc tgcggcgagg ccggcagaac gctgtgacag ccacacgccc 600
caaggcctcc aagatgagct acacgttgga ctcgctgggc aacccgtccg cctaccggcg 660
ggtaaccgag acccgctcga gcttcagccg cgtcagcggc tccccgtcca gtggcttccg 720
ctcgcagtcg tggtcccgcg gctcgcccag caccgtgtcc tcctcctata agcgcagcat 780
gctcgccccg cgcctcgctt acagctcggc catgctcagc tccgccgaga gcagccttga 840
cttcagccag tcctcgtccc tgctcaacgg cggctccgga cccggcggcg actacaagct 900
gtcccgctcc aacgagaagg agcagctgca ggggctgaac gaccgctttg ccggctacat 960
agagaaggtg cactacctgg agcagcagaa taaggagatt gaggcggaga tccaggcgct 1020
gcggcagaag caggcctcgc acgcccagct gggcgacgcg tacgaccagg agatccgcga 1080
gctgcgcgcc accctggaga tggtgaacca cgagaaggct caggtgcagc tggactcgga 1140
ccacctggag gaagacatcc accggctcaa ggagcgcttt gaggaggagg cgcggttgcg 1200
cgacgacact gaggcggcca tccgcgcgct gcgcaaagac atcgaggagg cgtcgctggt 1260
caaggtggag ctggacaaga aggtgcagtc gctgcaggat gaggtggcct tcctgcggag 1320
caaccacgag gaggaggtgg ccgaccttct ggcccagatc caggcatcgc acatcacggt 1380
ggagcgcaaa gactacctga agacagacat ctcgacggcg ctgaaggaaa tccgctccca 1440
gctcgaaagc cactcagacc agaatatgca ccaggccgaa gagtggttca aatgccgcta 1500
cgccaagctc accgaggcgg ccgagcagaa caaggaggcc atccgctccg ccaaggaaga 1560
gatcgccgag taccggcgcc agctgcagtc caagagcatc gagctagagt cggtgcgcgg 1620
caccaaggag tccctggagc ggcagctcag cgacatcgag gagcgccaca accacgacct 1680
cagcagctac caggtaggaa ccgcggctgc gcggccagcc tgcgccagcg ccagcgccgc 1740
gcgcccccga cacttgggct cgtgcccagg cgccctctcc gccgcgctcc ctggtggccg 1800
ctcgctagag cacgcgcgcc gcagacctag ggtatttgcg gatcagcgtc ctcgcccatc 1860
tcatcctcca cactccgccc ccacccacct gccccagctg ctaagggtct tgaccttttt 1920
cagaaacgtg catcttttcc agttctaatt ttgcacgctt gcacgtttaa agcaggaggg 1980
atgaattcgg tagtggataa 2000

<210> 13
<211> 2300
<212> DNA
<213> Homo sapiens

<400> 13
tcagattgtc attgggaggg tgaataaatg aatgcttgca ttatgagagt ttgggggcag 60
aaatatgcca cagactctta tctgaagcca tcagatttag tggctgcgaa cccaccgaag 120
tcagggattt acatttttta cagcaacgag agaaaacttc ccctttcctc tgcagaagtc 180
aggactggat ctcaaaaata gaaatgtgtc ctcctaaatg tgtgcccatc cccgtggttg 240
acaaacaacg gatttcccaa gatagctgcc acacacttgg tttctaatct ctgtattgct 300
tccccgccag aatgtcgaag tccttcccga atatgcccag tcatactttc tgaacttttg 360
agcaaacacc gtccggcttc ttgtgctttc ctcaaagacc ccaggcaccg gcagggagga 420
cacaggccgg ggcagagcgc ccctgcgcgg gggattcctg ccactccgcg ccagcctgcg 480

gcgcaaacgc tcttctcagc cgcagtccca cccgctgctg gcaatctgaa tgaggagccg 540
cgctattttt acctccccgg ctgcaatcct ttatatttac atgcaggaag caaatatata 600
agggattaag aaggagatgc gtggccttag tttatccaga gcaggaagag gttggaatag 660
gagagggtat gtgaagtctg gggtggtgga aaaggcaggt ggacttcggc tggttgtttt 720
ctcccgatca tccctgtctc tggcctggaa acccccgtac tctctttctt ctggcttatc 780
cgtgactgcc ggctccccct ccaccgcccc catcttttga ggtaccaccc gtcacctccg 840
atgctgcttg ggctgctgca tcactctgct gctttacccc cttccccgcc ccccaacaaa 900
gcatgcgcag tgcgttccgg gccaggcaac agcagcagca cagcatccag caacagcatc 960
agcacccgaa gccccgctcg ggcgcgctct cggggggcgg ggcgcacgcc cgctccgcgc 1020
gtccccgcgc cgctcgctcc cgcgcgtccc cgcgccgctc gctcccgcgc gccgcctcag 1080
catcctcagg cccggcggca gcccccgcag tcgctgaagc ggccgcgccc gccgggggag 1140
ggagtagccg ctggggaggc tccaagttgg cggagcggcg aggacccctg gactcctctg 1200
cgtcccgccc cgggagtggc tgcgaggcta ggcgagccgg gaaagggggc gccgcccagc 1260
cccgagcccc gcgccccgtg ccccgagccc ggagccccct gcccgccgcg gcaccatgcg 1320
cgccgagccg gcgtgaccgg ctccgcccgc ggccgccccg cagctagccc ggcgctctcg 1380
ccggccacac ggagcggcgc ccgggagcta tgagccatga agccgcccgg cagcagctcg 1440
cggcagccgc ccctggcggg ctgcagcctt gccggcgctt cctgcggccc ccaacgcggc 1500
cccgccggct cggtgcctgc cagcgccccg gcccgcacgc cgccctgccg cctgcttctc 1560
gtccttctcc tgctgcctcc gctcgccgcc tcgtcccggc cccgcgcctg gggggctgct 1620
gcgcccagcg gtgggtatgg ccccgtgccc tttgcgttgg ctttcccgcg gggccctgca 1680
gaggaaagcg aagggcgcgc gggtccgtgt gctccgggct tgtccccggc tcggcctttc 1740
cttccctccc tgcctgtctt tccacccttc tcgttcccaa acccccattc atcccagttc 1800
acttttggaa gtccatttct gttgcattcg cgaaaaaccc attccaattc ttgttggttc 1860
cactgggagg tgtttagtgg atcctgggtc cctcagcgat ctctgtgcaa cttgcggagg 1920
ggcaaccagt ggatgggaaa tacagcgagg gagcaagttg ctacttgcgt ggtggaacct 1980
taatgtgaat gcggggagga tgtagtgata atagtggtaa tgggctgttt cctcaaattt 2040
cgtatccggc gcattcagtg cggttggaat taaggtgggg gaggcacact tcggggacca 2100
aagaattaag gtgctgaaga catacttcat gcacgacctt tggttctgat ttctcaaagt 2160
gcttgtcatt ataatgaaca attaatataa taccatcttc tatatattga tgattggaag 2220
tcactgaaag cagaaagctg gctttgtcag gaaaataaaa agaaattggg aagctgccag 2280
catctgtatc cctacatggc 2300

<210> 14
<211> 3000
<212> DNA
<213> Homo sapiens

<400> 14
tactgccgac tttaggtctc tctggatctc aggccccctt ctctaagatg catcctagag 60
gaccaaaaat acactttatt tgggcttcgc ctgcttttgt ggaagggtag tttactagag 120
gatataatct cgtgttttaa tttgctctct ctcctaaagg aaatgtggag aaaaaaaaaa 180
agcagaaatt ggaaataacc aatatttagt ttatttcatt cgattcttag gggaactggt 240
gaggagccta agatgatttt cccttcctag agaaagaatc caaagtccag ggaaatagcg 300
acaggggagt tcaagactgc ccctgctagt ccttccttgg ctactctccg ctgcgatcgc 360
aggatagctc tcattagcag gagaatcggg caagtgtgtg gataagtaga gagtgtgttg 420
aacaacttgt aacgttttat gaaatacgca ttgtcatggt tccctaaaag gctttgcgga 480
agccgtttgt ctttactaat caagtcttta cttacacaaa agtagaagta gaagtagttt 540
tagaaaacat actaacaatc ttctatcccc ttgaagacca gagtagcaga aaacaggtga 600
tttgcattat aaaattgcac tcactttttc ctcctttcag atttcacatt acattagccc 660
atttgtgtta cggtgtataa aaaatggaac aggcgcctcc actgcattgt tctcctttaa 720
aaatagatca cttacaccct aactttgttt tccttaaatt cgattcttaa caggagagct 780
ttctattatt tcagatggag tgaggttgca cgactgggat ggaagaaagg aatcccttaa 840
atttggggga atttctgttc tctgttccaa gaccatttta cttggggtgt gggggtgggc 900
gcggcggtca gggcagtgga acgcagtcgc ggctgcgcca tccctgcact tccaggcgcg 960
cgggagggac cggcggggac gcgagctgcg gactctggcg aactcggggg aggcagacag 1020
ggggaggcgg acacccagcc ggcaggcgtc tcagcctccc cgcagccggc gggcttttct 1080
cctgacagct ccaggaaagg cagacccctt ccccagccag ccaggtaagg taaagactgc 1140
tgttgagctt gctgttactg agggcgcaca gaccctgggg agaccgaagc ttgccactgc 1200
gggattctgt ggggtaacct gggtctacgg aagtttcctg aaagagggga gaagggtttg 1260
catttttcct atggaggatt cttctctctc tagcatttcg tttgatgtat tcaactggta 1320
gaagtgagat ttcaacaggt agcagagagc gctcacgtgg aggaggtttg gggcgccgcg 1380
gcgccacccc cacccctcct cgggaccgcg cctatttcta aagttacacg tcgacgaact 1440
aacctatgct ttaaattcct ctttccagcc ccgtgagtcc gcggcgacat tgggccgtgg 1500
ggtggctggg aacggtcccc tcctccggaa aaaccagaga acggcttgga gagctgaaac 1560
gagcgtccgc gagcaggtcc gtgcagaacc gggcttcagg accgctgagc tccgtagggc 1620
gtccttgggg gacgccaggt cgccggctcc tctgccctcg ttgagatgga caacgcctcg 1680
ttctcggagc cctggcccgc caacgcatcg ggcccggacc cggcgctgag ctgctccaac 1740
gcgtcgactc tggcgccgct gccggcgccg ctggcggtgg ctgtaccagt tgtctacgcg 1800
gtgatctgcg ccgtgggtct ggcgggcaac tccgccgtgc tgtacgtgtt gctgcgggcg 1860
ccccgcatga agaccgtcac caacctgttc atcctcaacc tggccatcgc cgacgagctc 1920

ttcacgctgg tgctgcccat caacatcgcc gacttcctgc tgcggcagtg gcccttcggg 1980
gagctcatgt gcaagctcat cgtggctatc gaccagtaca acaccttctc cagcctctac 2040
ttcctcaccg tcatgagcgc cgaccgctac ctggtggtgt tggccactgc ggagtcgcgc 2100
cgggtggccg gccgcaccta cagcgccgcg cgcgcggtga gcctggccgt gtgggggatc 2160
gtcacactcg tcgtgctgcc cttcgcagtc ttcgcccggc tagacgacga gcagggccgg 2220
cgccagtgcg tgctagtctt tccgcagccc gaggccttct ggtggcgcgc gagccgcctc 2280
tacacgctcg tgctgggctt cgccatcccc gtgtccacca tctgtgtcct ctataccacc 2340
ctgctgtgcc ggctgcatgc catgcggctg gacagccacg ccaaggccct ggagcgcgcc 2400
aagaagcggg tgaccttcct ggtggtggca atcctggcgg tgtgcctcct ctgctggacg 2460
ccctaccacc tgagcaccgt ggtggcgctc accaccgacc tcccgcagac gccgctggtc 2520
atcgctatct cctacttcat caccagcctg agctacgcca acagctgcct caaccccttc 2580
ctctacgcct tcctggacgc cagcttccgc aggaacctcc gccagctgat aacttgccgc 2640
gcggcagcct gactccccca gcgtccggct ccgcaactgc ccgccactcc tggccagcga 2700
gggaggagcc ggcgccagag tgcgggacca gacaggccgc ctaggcctcc tggggaaacc 2760
gactcgcgcc ccatacccga cctagcagat cggaagcgct gcgactgtgc ccgcaggttg 2820
accttgccaa gccctccagg tgatgcgcgg ccatgccggg tgaggagaac tgaggctgag 2880
atcgccacac tgagggctcc ctaaagccga ggtggaggaa gaggagggta gaggaggagg 2940
gcggtattgc tgggaaccgc cccctccctg ccctgctccc tgctgcccca cccgagccct 3000

<210> 15
<211> 3000
<212> DNA
<213> Homo sapiens

<400> 15
gaatacatta aagtaggggc aacccttgag cccagacttc tgccatgtga agaccctttg 60
aaaatcctga caaacacagg tactgcgtaa gtggtcagct aattaaagag gggaggtgga 120
gctgtccttt gtgtatccaa taagtaccca ttatctcatt tgagcatgaa aagaggccac 180
tgttattact ttcaagaagg aaagtaagca ggatagctca tatttttaga accattcctc 240
accaaatgga ataattccgg tgaaaagtgg gagtgaggaa gaaagaaaaa aaaaacttct 300
aatcataatg tttgggaata agaaaggaag aagaaactca cgtcaaagcc gactttctcc 360
tgcagctgta aaataaactc ttaagaccct tcctgctgaa actctggaga ggaaaactgg 420
agtggcgggt gggctttgcc tgcagctcaa ctctccctcg cggcgcgggc gcggctgggt 480
tcagcacctc ggaaagcgcc cctcgcggcg ccccgggatt acgcatgctc cttggggccc 540
gccgccttgg ccgtgcaagt gccaccgtaa ctggtgagag ccgctggcaa cccacccgga 600
gttgacaacc gcggagagac gcagacaccc actgacctcc aggaagctga gcgtggtgga 660
tggaactcta cgatctcttt ctctccaagg acggaaacct catccaagca gtcccagagg 720
aaacggataa aggtatttga aagggagcga gcggccccaa atcgcacaat tgagcggctg 780
ggggagttat gcgccagtgc cccagtgacc gcgggacacg gagaggggaa gtctgcgttg 840
tacataagga cctagggact ccgagcttgg cctgagaacc cttggacgcc gagtgcttgc 900
cttacggct gcactcctca actctgctcc aaagcagccg ctgagctcaa ctcctgcgtc 960
cagggcgttc gctgcgcgcc aggacgcgct tagtacccag ttcctgggct ctctcttcag 1020
tagctgcttt gaaagctccc acgcacgtcc cgcaggctag cctggcaaca aaactggggt 1080
aaaccgtgtt atcttaggtc ttgtccccca gaacatgacc tagaggtacc tgcgcatgca 1140
gatggccgat gcagccacga tagccaccat gaataaggca gcaggcgggg acaagctagc 1200
agaactcttc agtctggtcc cggaccttct ggaggcggcc aacacgagtg gtaacgcgtc 1260
gctgcagctt ccggacttgt ggtgggagct ggggctggag ttgccggacg gcgcgccgcc 1320
aggacatccc ccgggcagcg gcggggcaga gagcgcggac acagaggccc gggtgcggat 1380
tctcatcagc gtggtgtact gggtggtgtg cgccctgggg ttggcgggca acctgctggt 1440
tctctacctg atgaagagca tgcagggctg gcgcaagtcc tctatcaacc tcttcgtcac 1500
caacctggcg ctgacggact ttcagtttgt gctcaccctg cccttctggg cggtggagaa 1560
cgctcttgac ttcaaatggc ccttcggcaa ggccatgtgt aagatcgtgt ccatggtgac 1620
gtccatgaac atgtacgcca gcgtgttctt cctcactgcc atgagtgtga cgcgctacca 1680
ttcggtggcc tcggctctga agagccaccg gacccgagga cacggccggg gcgactgctg 1740
cggccggagc ctgggggaca gctgctgctt ctcggccaag gcgctgtgtg tgtggatctg 1800
ggctttggcc gcgctggcct cgctgcccag tgccattttc tccaccacgg tcaaggtgat 1860
gggcgaggag ctgtgcctgg tgcgtttccc ggacaagttg ctgggccgcg acaggcagtt 1920
ctggctgggc ctctaccact cgcagaaggt gctgctgggc ttcgtgctgc cgctgggcat 1980
cattatcttg tgctacctgc tgctggtgcg cttcatcgcc gaccgccgcg cggcggggac 2040
caaaggaggg gccgcggtag ccggaggacg cccgaccgga gccagcgccc ggagactgtc 2100
gaaggtcacc aaatcagtga ccatcgttgt cctgtccttc ttcctgtgtt ggctgcccaa 2160
ccaggcgctc accacctgga gcatcctcat caagttcaac gcggtgccct tcagccagga 2220
gtatttcctg tgccaggtat acgcgttccc tgtgagcgtg tgcctagcgc actccaacag 2280
ctgcctcaac cccgtcctct actgcctcgt gcgccgcgag ttccgcaagg cgctcaagag 2340
cctgctgtgg cgcatcgcgt ctccttcgat caccagcatg cgccccttca ccgccactac 2400
caagccggag cacgaggatc aggggctgca ggccccggcg ccgccccacg cggccgcgga 2460
gccggacctg ctctactacc cacctggcgt cgtggtctac agcgggggcc gctacgacct 2520
gctgcccagc agctctgcct actgacgcag gcctcaggcc caggcgcgcc cgtcggggca 2580
aggtggcctt ccccgggcgg taaagaggtg aaaggatgaa ggagggctgg gggggcccc 2640
atttaagaag taggtgggag gaggatgggc agagcatgga ggaggagcct gtggataggc 2700

cgaggacctt ctctggagag gagatgcttc gaaatcaggt ggagagagga aattggcaaa 2760
gggatagaga cgagccccac gggccagaca gccaacctcc gctccgcacc ccacagcctc 2820
tccttactct tcccacgctg agtagtgtgg gggcgcccag aagcgaagac aagcagcaaa 2880
aatgtagaga aattggcacg gggagcgggg cttagccaaa tgatgcacag acaattgtgc 2940
ccgtttattc cagcgacttc tgcggagagg gcagccgtcg gcacaaacac tcctttgcgt 3000

<210> 16
<211> 2200
<212> DNA
<213> Homo sapiens

<400> 16
gtcccccgat tccctcaccc atcatataac gtgtgtattt attatgtttc ccgtttcctc 60
tgtctccgcc agcagaatgt aaactccatg aggtcaggaa tctccgagtt atgttgcgcc 120
agtgtaatcc aagagcccgg aacagtgcct ggcacacagc gggcatatgg aagaacaaat 180
gtgtgaaggt gtgaatgaat gaataattga aagaataaat agtagttctc agcctcacag 240
aacacgggtc acaacctcaa atgacctgct accctgccca taaataacag agatgcagga 300
gtaagtgctg ggctgtgacc tgtcaacatg ctaagccgct caaacaaaac tgcccaacag 360
cccgctggcc gcctatttgc agcactgggc cctgagccgc acattcccat ttcgttgata 420
aagaaactga ccagatagtt taagtggcct gctgcggaag acagagctgg tgctgcaccg 480
gtcgctgctt ccccagtcct tttttggcct cctttctgac gcgacgcaga ccccagttct 540
ggagagtctg tcactcgctc cccgtggtgg gagatcagag gcctggtgtc cttgggagcg 600
gcgagcggtg ctcggcgcag gatagaaagg gagtgcgcgc ccgagtcccc cagatccctg 660
ggaacccgcg ccaccctccc gcccctgccc atccccggcc gcgctgtcag tctccattag 720
cgctaacagg ctccagacgg agcgggccgg gcgctgggtt aatgcaatcg gcgcgttacc 780
tggggcgcag gctacattac cagcccggcc cccgccaggc acggccagaa ccagtcagcc 840
cgcgccctgc cggccgcccc gcgcctccag ctcttccccg gccccgcccg aacgccacac 900
ggcggagccc agccccagcc cgcgccctag agcctgccaa ggcgccgccg gtcgggggcc 960
ggcagggcgc aaggcaccag ggatcccctc gccgccggac acgtgagtgc gccctgagcg 1020
cgggacaggg ctaggtctgc ctgggaggcc cgggccgaga cgcgccagca gagggctagc 1080
gagtttgtag tgcagtgacg ttaagtgtcc gagaaggctc ctgtggctgt tgaagtgtcg 1140
cggacctgag ctggggaggg ggtcggcacg ctgccctcag cctcggtgag ttcaatccca 1200
gccatttggg gcaggcgaga gtgggtgaac gaggaaaagt gctgcagggt cttcagccgc 1260
ccccagaggg ctgtcagaag tctccaactc ttgagttccg gcgtgcccca acctctgttt 1320
ccaaattttt ccagcggacg cgcgctcttt tctgggaacc ctgcgtccgc tcagcgcgcg 1380
ctcatcccag tgtctaaggc gctcccgggt ggtcttggga gttgcaagta gggaggaacg 1440
gccgggtaac cacctctttt ccctttatcc aagcagagcc tcggcgtgcc cccaggaccg 1500
gtaaagttcc tctcgccagc cgcatccatg cttctggcgc ggatgaaccc gcaggtgcag 1560
cccgagaaca acggggcgga cacgggtcca gagcagcccc ttcgggcgcg caaaactgcg 1620
gagctgctgg tggtgaagga gcgcaacggc gtccagtgcc tgctggcgcc ccgcgacggc 1680
gacgcgcagc cccgggagac ctggggcaag aagatcgact tcctgctgtc cgtagtcggc 1740
ttcgcagtgg acctggccaa cgtgtggcgc ttcccctacc tctgctacaa gaacggcggc 1800
ggtgagcgtg gggtcggcct gggaatttga atctgggagg tccactgtct gcagcggtgg 1860
ctgggacagg agctggaata cacacggaag ggaggcgagg agacaggggc aaatctgggg 1920
cgcagaaaga actggacagg gctaacggga aaaaaaaaag attggagtcc tctggaaggt 1980
cattttccca ggctctttgc agagtacctc gagctcattc cagcggaagt gtcaggattg 2040
ggcaccctgg aagcaaaaca gcagaagagt gaaatcgagt catgacccta aagtcatggt 2100
aggggtatgg atggaaagga cagaatctgg ggtgccaggt tgggtggggg agcctgacct 2160
tttgatggtc tgctggaagg gaggtggaga ttccaagagc 2200

<210> 17
<211> 18
<212> DNA
<213> Artificial Sequence

<220>
<223> Designed oligonucleotide

<400> 17
ctcagcaccc aggcggcc 18

<210> 18
<211> 20
<212> DNA
<213> Artificial Sequence

<220>
<223> Designed oligonucleotide

<400> 18

ctggccaaac tggagatcgc 20

<210> 19
<211> 386
<212> DNA
<213> Artificial Sequence

<220>
<223> Designed oligonucleotide

<400> 19
ctcagcaccc aggcggccgc gatcatgagg cgcgagcggc gcgcgggctg ttgcagagtc 60
ttgagcgggt ggcacaccgc gatgtagcgg tcggctgtca tgactaccag catgtaggcc 120
gacgcaaaca tgccgaacac ctgcaggtgc ttcaccacgc ggcacagcca gtcggggccg 180
cggaagcggt aggtgatgtc ccagcacatt tgcggcagca cctggaagaa tgccacggcc 240
aggtcggcca ggctgaggtg tcggatgaag aggtgcatgc gggacgtctt gcgcggcgtc 300
cggtgcagag ccagcagtac gctgctgttg cccagcacgg ccaccgcgaa agtcaccgcc 360
agcacggcga tctccagttt ggccag 386

<210> 20
<211> 386
<212> DNA
<213> Artificial Sequence

<220>
<223> Designed oligonucleotide

<400> 20
ctcagcaccc aggcggccgc gatcatgagg cgcgagcggc gcgcgggctg ttgcagagtc 60
ttgagcgggt ggcacaccgc gatgtagcgg tcggctgtca tgactaccag catgtaggcc 120
gacgcaaaca tgccgaacac ctgcaggtgc ttcaccacgc ggcacagcca gtcggggccg 180
cggaagcggt aggtgatgtc ccagcacatt tgcggcagca cctggaagaa tgccacggcc 240
aggtcggcca ggctgaggtg tcggatgaag aggtgcatgc gggacgtctt gcgcggcgtc 300
cggtgcagag ccagcagtac gctgctgttg cccagcacgg ccaccgcgaa agtcaccgcc 360
agcacggcga tctccagttt ggccag 386

<210> 21
<211> 17
<212> DNA
<213> Artificial Sequence

<220>
<223> Designed oligonucleotide

<400> 21
ctggccaaac tggagat 17

<210> 22
<211> 19
<212> DNA
<213> Artificial Sequence

<220>
<223> Designed oligonucleotide

<400> 22
tgagctccgt agggcgtcc 19

<210> 23
<211> 17
<212> DNA
<213> Artificial Sequence

<220>
<223> Designed oligonucleotide

<400> 23
gcgccgggtc cgggccc 17

<210> 24
<211> 121
<212> DNA
<213> Artificial Sequence

<220>
<223> Designed oligonucleotide

<400> 24
gcgccgggtc cgggcccgat gcgttggcgg gccagggctc cgagaacgag gcgttgtcca 60
tctcaacgag ggcagaggag ccggcgacct ggcgtccccc aaggacgccc tacggagctc 120
a 121

<210> 25
<211> 121
<212> DNA
<213> Artificial Sequence

<220>
<223> Designed oligonucleotide

<400> 25
gcgccgggtc cgggcccgat gcgttggcgg gccagggctc cgagaacgag gcgttgtcca 60
tctcaacgag ggcagaggag ccggcgacct ggcgtccccc aaggacgccc tacggagctc 120
a 121

<210> 26
<211> 19
<212> DNA
<213> Artificial Sequence

<220>
<223> Designed oligonucleotide

<400> 26
gacaacgcct cgttctcgg 19

<210> 27
<211> 21
<212> DNA
<213> Artificial Sequence

<220>
<223> Designed oligonucleotide

<400> 27
aggtgagcta cgtgtgtttg g 21

<210> 28
<211> 21
<212> DNA
<213> Artificial Sequence

<220>
<223> Designed oligonucleotide

<400> 28
agacatgtgc tcacgtacgg t 21

<210> 29
<211> 391
<212> DNA
<213> Artificial Sequence

<220>
<223> Designed oligonucleotide

<400> 29
aggtgagcta cgtgtgtttg ggcgtcgtgc actggctcac ttgtacgcgc agaaatggca 60
gcttgtacga ttggtgaccc gccttttcga cactggaccg ctatggacgt ggcggcggtg 120

tggcggcggc tcaatgacct gtggcgcccg tttgtggcgt gcgatagtcg agccgcctgt 180
cacgtgcgcg gccgccctgc tccgtttgac gcgatgcata gcatgcgacc acccagtaat 240
catactgctg acgctattgg tcacgtggtt atggcagctg ctgttgactg cggtggcgtc 360
ccgtttccac accgtacgtg agcacatgtc t 391

<210> 30
<211> 391
<212> DNA
<213> Artificial Sequence

<220>
<223> Designed oligonucleotide

<400> 30
aggtgagcta cgtgtgtttg ggcgtcgtgc actggctcac ttgtacgcgc agaaatggca 60
gcttgtacga ttggtgaccc gccttttcga cactggaccg ctatggacgt ggcggcggtg 120
tggcggcggc tcaatgacct gtggcgcccg tttgtggcgt gcgatagtcg agccgcctgt 180
cacgtgcgcg gccgccctgc tccgtttgac gcgatgcata gcatgcgacc acccagtaat 240
catactgctg acgctattgg tcacgtggtt atggcagctg ctgttgactg cggtggcgtc 360
ccgtttccac accgtacgtg agcacatgtc t 391

<210> 31
<211> 17
<212> DNA
<213> Artificial Sequence

<220>
<223> Designed oligonucleotide

<400> 31
ctggccaaac tggagat 17

<210> 32
<211> 21
<212> DNA
<213> Artificial Sequence

<220>
<223> Designed oligonucleotide

<400> 32
ggacctgtgt ttgacgggta t 21

<210> 33
<211> 21
<212> DNA
<213> Artificial Sequence

<220>
<223> Designed oligonucleotide

<400> 33
agtacagatc tggcgttctc g 21

<210> 34
<211> 117
<212> DNA
<213> Artificial Sequence

<220>
<223> Designed oligonucleotide

<400> 34
ggacctgtgt ttgacgggta taacactaag ttgcgcaatt tgctgtattg cgaaatccgc 60
ccggacgata tcactcttga gcgcatgtgc cgtttccgag aacgccagat ctgtact 117

<210> 35
<211> 117
<212> DNA
<213> Artificial Sequence

<220>
<223> Designed oligonucleotide

<400> 35
ggacctgtgt ttgacgggta taacactaag ttgcgcaatt tgctgtattg cgaaatccgc 60
ccggacgata tcactcttga gcgcatgtgc cgtttccgag aacgccagat ctgtact 117

<210> 36
<211> 21
<212> DNA
<213> Artificial Sequence

<220>
<223> Designed oligonucleotide

<400> 36
agtacagatc tggcgttctc g 21

<210> 37
<211> 271
<212> DNA
<213> Artificial Sequence

<220>
<223> Designed oligonucleotide

<400> 37
tagggagtgc cagacagtgg gcgcaggcca gtgtgtgtgc gcaccgtgcg cgagccgaag 60
cagggcgagg cattgcctca cctgggaagc gcaaggggtc agggagttcc ctttctgagt 120
caaagaaagg ggtgacggtc gcacctggaa aatcgggtca ctcccacccg aatattgcgc 180
ttttcagacc ggcttaagaa acggcgcacc acgagactat atcccacacc tggctcggag 240
ggtcctacgc ccacggaatc tcgctgattg c 271

<210> 38
<211> 17
<212> DNA
<213> Artificial Sequence

<220>
<223> Designed oligonucleotide

<400> 38
ctggccaaac tggagat 17

<210> 39
<211> 85
<212> DNA
<213> Artificial Sequence

<220>
<223> Designed oligonucleotide

<400> 39
cgggcgcggt ggctcacgcc tgtaatccca gcactttggg aggccgaggt gggcggatca 60
cgaggtcagg agatcgagac catcc 85

<210> 40
<211> 21
<212> DNA
<213> Artificial Sequence

<220>
<223> Designed oligonucleotide

<400> 40
ggatggtctc gatctcctga c 21

<210> 41
<211> 21

<212> DNA
<213> Artificial Sequence

<220>
<223> Designed oligonucleotide

<400> 41
aggtgagcta cgtgtgtttg g                                                21

<210> 42
<211> 30
<212> DNA
<213> Artificial Sequence

<220>
<223> Designed oligonucleotide

<400> 42
gcgtcgtgca ctggctcact tgtacgcgca                                       30

<210> 43
<211> 30
<212> DNA
<213> Artificial Sequence

<220>
<223> Designed oligonucleotide

<400> 43
cttgtacgat tggtgacccg ccttttcgac                                       30

<210> 44
<211> 30
<212> DNA
<213> Artificial Sequence

<220>
<223> Designed oligonucleotide

<400> 44
actggaccgc tatggacgtg gcggcggtgt                                       30

<210> 45
<211> 30
<212> DNA
<213> Artificial Sequence

<220>
<223> Designed oligonucleotide

<400> 45
ggcggcggct caatgacctg tggcgcccgt                                       30

<210> 46
<211> 30
<212> DNA
<213> Artificial Sequence

<220>
<223> Designed oligonucleotide

<400> 46
ttgtggcgtg cgatagtcga gccgcctgtc                                       30

<210> 47
<211> 25
<212> DNA
<213> Artificial Sequence

<220>

<223> Designed oligonucleotide

<400> 47
acgtgcgcgg ccgccctgct ccgtt                                          25

<210> 48
<211> 30
<212> DNA
<213> Artificial Sequence

<220>
<223> Designed oligonucleotide

<400> 48
tgacgcgatg catagcatgc gaccacccag                                     30

<210> 49
<211> 30
<212> DNA
<213> Artificial Sequence

<220>
<223> Designed oligonucleotide

<400> 49
actgctgacg ctattggtca cgtggttatg                                     30

<210> 50
<211> 30
<212> DNA
<213> Artificial Sequence

<220>
<223> Designed oligonucleotide

<400> 50
ctgctgttga ctgcggtggc gtcccgtttc                                     30

<210> 51
<211> 21
<212> DNA
<213> Artificial Sequence

<220>
<223> Designed oligonucleotide

<400> 51
ggacctgtgt ttgacgggta t                                              21

<210> 52
<211> 25
<212> DNA
<213> Artificial Sequence

<220>
<223> Designed oligonucleotide

<400> 52
aacactaagt tgcgcaattt gctgt                                          25

<210> 53
<211> 25
<212> DNA
<213> Artificial Sequence

<220>
<223> Designed oligonucleotide

<400> 53
attgcgaaat ccgcccggac gatat                                          25

<210> 54
<211> 25
<212> DNA
<213> Artificial Sequence

<220>
<223> Designed oligonucleotide

<400> 54
cactcttgag cgcatgtgcc gtttc                                          25

<210> 55
<211> 30
<212> DNA
<213> Artificial Sequence

<220>
<223> Designed oligonucleotide

<400> 55
cagtgtgtgt gcgcaccgtg cgcgagccga                                     30

<210> 56
<211> 30
<212> DNA
<213> Artificial Sequence

<220>
<223> Designed oligonucleotide

<400> 56
ggcgaggcat tgcctcacct gggaagcgca                                     30

<210> 57
<211> 30
<212> DNA
<213> Artificial Sequence

<220>
<223> Designed oligonucleotide

<400> 57
ggtgacggtc gcacctggaa aatcgggtca                                     30

<210> 58
<211> 30
<212> DNA
<213> Artificial Sequence

<220>
<223> Designed oligonucleotide

<400> 58
acccgaatat tgcgcttttc agaccggctt                                     30

<210> 59
<211> 30
<212> DNA
<213> Artificial Sequence

<220>
<223> Designed oligonucleotide

<400> 59
tcggagggtc ctacgcccac ggaatctcgc                                     30

<210> 60
<211> 30
<212> DNA

<213> Artificial Sequence

<220>
<223> Designed oligonucleotide

<400> 60
cgggcgcggt ggctcacgcc tgtaatccca                                        30

<210> 61
<211> 30
<212> DNA
<213> Artificial Sequence

<220>
<223> Designed oligonucleotide

<400> 61
tttgggaggc cgaggtgggc ggatcacgag                                        30


<210> 62
<211> 20
<212> DNA
<213> Artificial Sequence

<220>
<223> Designed oligonucleotide

<400> 62
ggtggctcac gcctgtaatc                                                   20

<210> 63
<211> 21
<212> DNA
<213> Artificial Sequence

<220>
<223> Designed oligonucleotide

<400> 63
ggatggtctc gatctcctga c                                                 21

## Description

### TECHNICAL FIELD

The present invention relates to a method for quantifying or detecting DNA having a target DNA region, and so on.

### BACKGROUND ART

Known as a method for quantifying or detecting DNA having a target DNA region contained in a specimen are, for example, a method of detecting DNA having a target DNA region amplified by a chain reaction of DNA synthesis by DNA polymerase (Polymerase Chain Reaction; hereinafter, sometimes referred to as PCR) after extraction of DNA from a specimen, a method of detecting DNA by hybridization of a fluorescent-labeled oligonucleotide with a target DNA region possessed by DNA in a specimen, and so on (see, for example, J. Cataract. Refract. Surg., 2007;33(4):635-641, Environ. Mol. Mutagen., 1991;18(4):259-262).

### DISCLOSURE OF THE INVENTION

It is an object of the present invention to provide a method for quantifying or detecting DNA having a target DNA region in a simple and convenient manner.

Specifically, the present invention provides:

### [Invention 1]

A method for quantifying or detecting DNA comprising a target DNA region contained in a specimen comprising:
(1) First step of preparing from the specimen DNA for which the target DNA region is to be detected;
(2) Second step of treating the DNA prepared in First step with a DNA methylation enzyme;
(3) Third step of preparing single-stranded methylated DNA from the DNA treated in Second step, and making a detection oligonucleotide bind with the single-stranded methylated DNA to obtain a test DNA complex;
(4) Forth step of making an immobilized methylated-DNA antibody bind with the test DNA complex obtained in Third step to obtain a detection complex; and
(5) Fifth step of quantifying or detecting DNA comprising a target DNA region in the single-stranded DNA by quantifying or detecting the detection oligonucleotide contained in the detection complex obtained in Fourth step by its identification function (hereinafter, sometimes referred to as the present method);

### [Invention 2]

The method according to Invention 1, wherein a counter oligonucleotide is added in obtaining a test DNA complex in Third step;

### [Invention 3]

The method according to Invention 1 or 2, wherein the immobilized methylated-DNA antibody is a methylcytosine antibody;

### [Invention 4]

The method according to any one of Inventions 1 to 3, wherein the DNA methylation enzyme is a cytosine methylation enzyme or SssI methylase;

### [Invention 5]

The method according to any one of Inventions 1 to 4, wherein the DNA comprising a target DNA region contained in the specimen is DNA comprising a target DNA region in DNA generated from RNA by a reverse transcriptase;

### [Invention 6]

The method according to any one of Inventions 1 to 5, wherein the specimen is any of the following biological specimen:
(a) mammalian blood, body fluid, excreta, body secretion, cell lysate, or tissue lysate,
(b) DNA extracted from one selected from the group consisting of mammalian blood, body fluid, excreta, body secretion, cell lysate, and tissue lysate,
(c) DNA prepared by using as a template RNA extracted from one selected from the group consisting of mammalian tissue, cell, tissue lysate and cell lysate,
(e) DNA extracted from bacterium, fungus or virus, or
(f) DNA prepared by using as a template RNA extracted from cell, fungus or virus;

### [Invention 7]

The method according to any one of Inventions 1 to 6, wherein the DNA comprising a target DNA region obtained in First step is DNA digested in advance with a restriction enzyme recognition cleavage site for which is not present in the target DNA region, a synthesized oligonucleotide, or DNA purified in advance;

### [Invention 8]

The method according to any one of Inventions 1 to 7, wherein the identification function of the detection oligonucleotide is any of the following identification function:
(a) fluorescence detection of FITC, or
(b) detection by FITC antibody;

### [Invention 9]

The method according to any one of Inventions 1 to 8, wherein the detection oligonucleotide comprises a repetitive sequence or a nucleotide sequence of an overlapping gene or a pseudo gene in human genome or a nucleotide sequence capable of complementarily binding with a part thereof;

### [Invention 10]

The method according to Invention 9, wherein the repetitive sequence in human genome is LINE or SINE;

### [Invention 11]

The method according to any one of Inventions 1 to 10, wherein the detection oligonucleotide comprises a nucleotide sequence capable of complementarily binding with any one of the following nucleotide sequences:
(1) the nucleotide sequence of SEQ ID N0: 37 or a nucleotide sequence having 80% or more sequence identity to the same,
(2) a complementary sequence of the nucleotide sequence of SEQ ID NO: 37 or a nucleotide sequence having 80% or more sequence identity to the same,
(3) the nucleotide sequence of SEQ ID NO: 39 or a nucleotide sequence having 80% or more sequence identity to the same, or
(4) a complementary sequence of the nucleotide sequence of SEQ ID NO: 39 or a nucleotide sequence having 80% or more sequence identity to the same;

### [Invention 12]

The method according to any one of Inventions 1 to 10, wherein the detection oligonucleotide comprises any one of the following nucleotide sequences:
(1) the nucleotide sequence of SEQ ID NO: 38 or a nucleotide sequence having 80% or more sequence identity to the same,
(2) a complementary sequence of the nucleotide sequence of SEQ ID NO: 38 or a nucleotide sequence having 80% or more sequence identity to the same,
(3) the nucleotide sequence of SEQ ID NO: 40 or a nucleotide sequence having 80% or more sequence identity to the same, or
(4) a complementary sequence of the nucleotide sequence of SEQ ID NO: 40 or a nucleotide sequence having 80% or more sequence identity to the same;

### [Invention 13]

The method according to any one of Inventions 1 to 12, wherein concentration of a sodium salt in a solution used in a DNA extracting operation for preparing DNA from a specimen in First step is 100 mM or more and 1000 mM or less;

### [Invention 14]

The method according to any one of Inventions 1 to 12, wherein concentration of a sodium salt in a solution used in a DNA extracting operation for preparing DNA from a specimen in First step is 100 mM or more and 200 mM or less;

### [Invention 15]

A method for selecting a specimen from a cancer patient comprising the step of evaluating that a specimen from a test subject is a specimen from a cancer patient when there is significant difference between a quantification result or a detection result of DNA quantified or detected by using the specimen from the test subject according to the method of any one of Inventions 1 to 14 and a quantification result or a detection result of DNA quantified or detected by using a specimen from a healthy subject according to the same method, and identifying a specimen from a cancer patient based on a result of the evaluation;

### [Invention 16]

The method according to Invention 15, wherein the specimen is mammalian serum; and

### [Invention 17]

The method according to Invention 15 or 16, wherein DNA comprising a target DNA region is free DNA comprising the target DNA region in a mammalian serum; and so on.

### BRIEF DESCRIPTION OF THE DRAWINGS

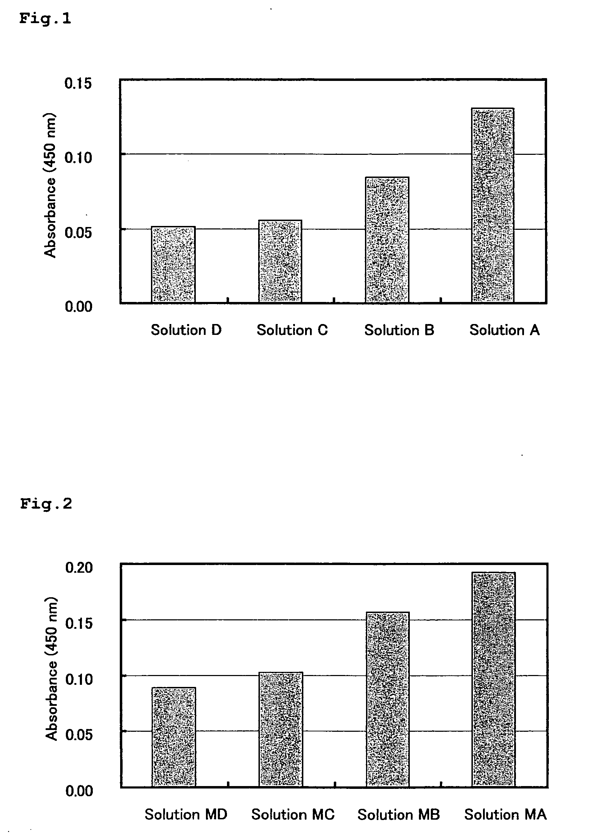

Fig. 1 is a drawing showing results obtained by using 0.5 µg/mL of a biotin-labeled methylcytosine antibody and 5'-end FITC-labeled oligonucleotide F1 in Example 1. DNA amounts measured by absorbance (450nm) are shown for Solution A (10 ng/10 µL TE buffer solution), Solution B (1 ng/10 µL TE buffer solution), Solution C (0.1 ng/10 µL TE buffer solution), and Solution D (0 ng/10 µL TE buffer solution (negative control solution)), respectively in this order from the right.
Fig. 2 is a drawing showing results obtained by using 0.5 µg/mL of a biotin-labeled methylcytosine antibody and 5'-end FITC-labeled oligonucleotide F1 in Example 2. DNA amounts measured by absorbance (450nm) are shown for Solution MA (10 ng each/20 µL TE buffer solution), Solution MB (1 ng each/20 µL TE buffer solution), Solution MC (0.1 ng each/20 µL TE buffer solution), and Solution MD (0 ng each/20 µL TE buffer solution (negative control solution)), respectively in this order from the right.

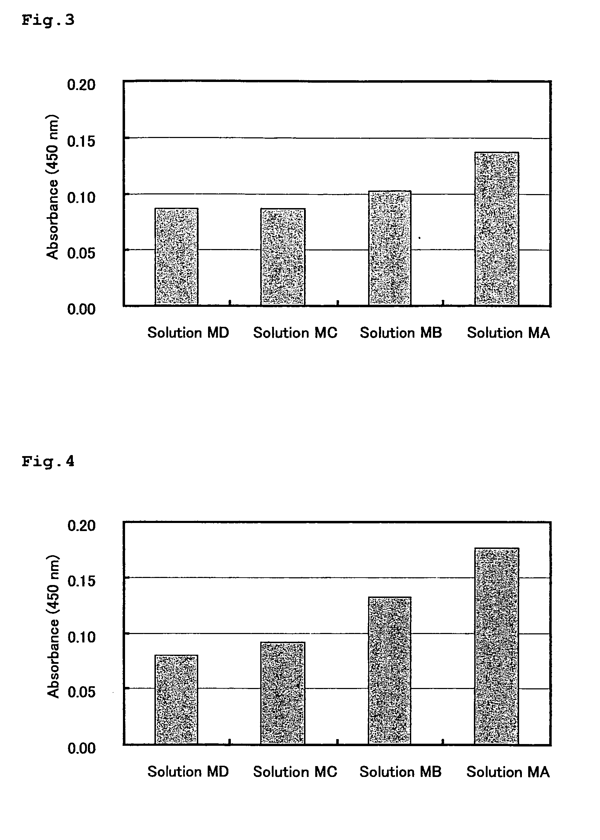

Fig. 3 is a drawing showing results obtained by using 0.5 µg/mL of a biotin-labeled methylcytosine antibody and 5'-end FITC-labeled oligonucleotide F2 in Example 3. DNA amounts measured by absorbance (450nm) are shown for Solution MA (10 ng each/20 µL TE buffer solution), Solution MB (1 ng each/20 µL TE buffer solution), Solution MC (0.1 ng each/20 µL TE buffer solution), and Solution MD (0 ng each/20 µL TE buffer solution (negative control solution)), respectively in this order from the right.
Fig. 4 is a drawing showing results obtained by using 0.5 µg/mL of a biotin-labeled methylcytosine antibody and 5'-end FITC-labeled oligonucleotides F1 and F2 in Example 4. DNA amounts measured by absorbance (450nm) are shown for Solution MA (10 ng each/20 µL TE buffer solution), Solution MB (1 ng each/20 µL TE buffer solution), Solution MC (0. 1 ng each/20 µL TE buffer solution), and Solution MD (0 ng each/20 µL TE buffer solution (negative control solution)), respectively in this order from the right.

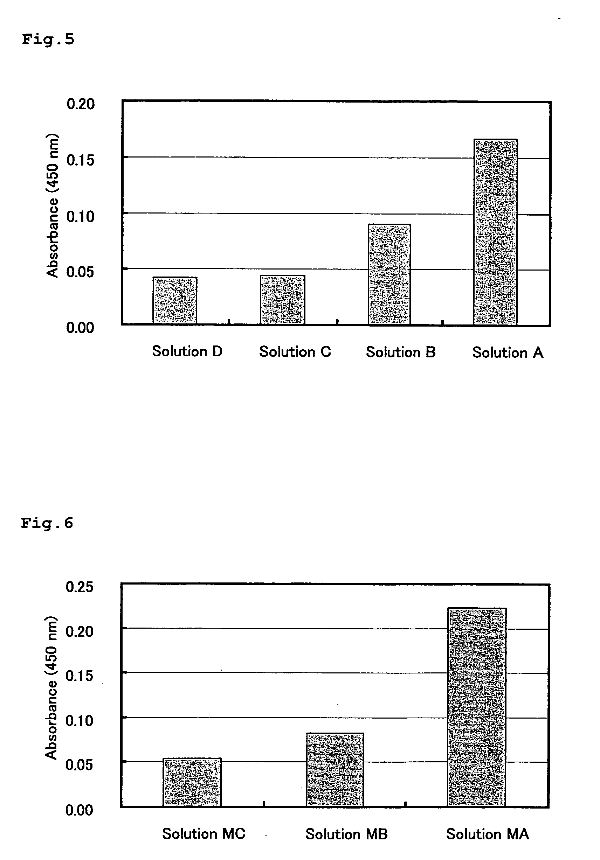

Fig. 5 is a drawing showing results obtained by using 0.5 µg/mL of a biotin-labeled methylcytosine antibody and 5'-end FITC-labeled oligonucleotide F3 in Example 5. DNA amounts measured by absorbance (450nm) are shown for Solution A (10 ng/10 µL TE buffer solution), Solution B (1 ng/10 µL TE buffer solution), Solution C (0.1 ng/10 µL TE buffer solution), and Solution D (0 ng/10 µL TE buffer solution (negative control solution)), respectively in this order from the right.
Fig. 6 is a drawing showing results obtained by using 0.5 µg/mL of a biotin-labeled methylcytosine antibody and 5'-end FITC-labeled oligonucleotide F3 in Example 6. DNA amounts measured by absorbance (450nm) are shown for Solution MA (10 ng each/20 µL TE buffer solution), Solution MB (1 ng each/20 µL TE buffer solution), and Solution MC (0 ng each/20 µL TE buffer solution (negative control solution)), respectively in this order from the right.

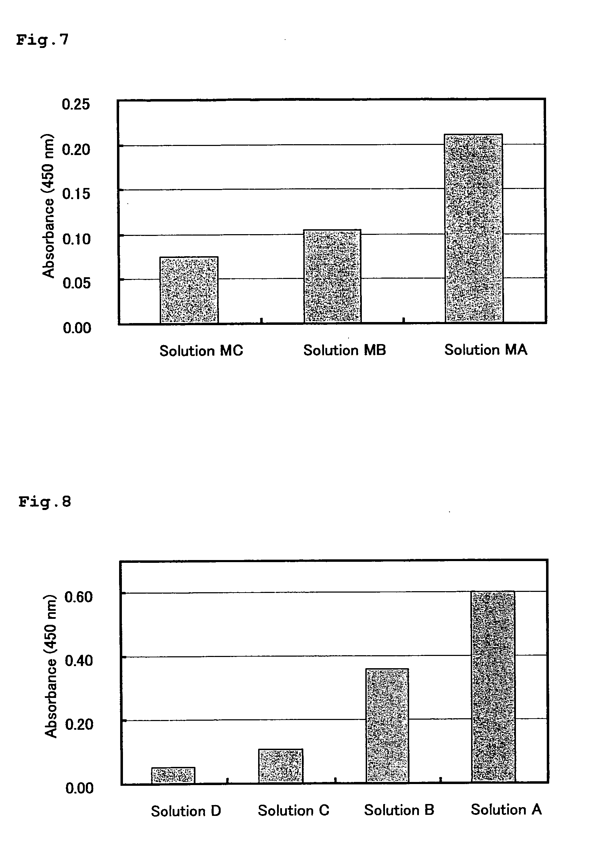

Fig. 7 is a drawing showing results obtained by using 0.5 µg/mL of a biotin-labeled methylcytosine antibody and 5'-end FITC-labeled oligonucleotides F3 and F4 in Example 7. DNA amounts measured by absorbance (450nm) are shown for Solution MA (10 ng each/20 µL TE buffer solution), Solution MB (1 ng each/20 µL TE buffer solution), and Solution MC (0 ng each/20 µL TE buffer solution (negative control solution)), respectively in this order from the right.
Fig. 8 is a drawing showing results obtained by using 0.5 µg/mL of a biotin-labeled methylcytosine antibody and 5'-end FITC-labeled oligonucleotide F5 in Example 8. DNA amounts measured by absorbance (450nm) are shown for Solution A (100 ng/5 µL TE buffer solution), Solution B (10 ng/5 µL TE buffer solution), Solution C (1 ng/5 µL TE buffer solution), and Solution D (0 ng/5 µL TE buffer solution (negative control solution)), respectively in this order from the right.

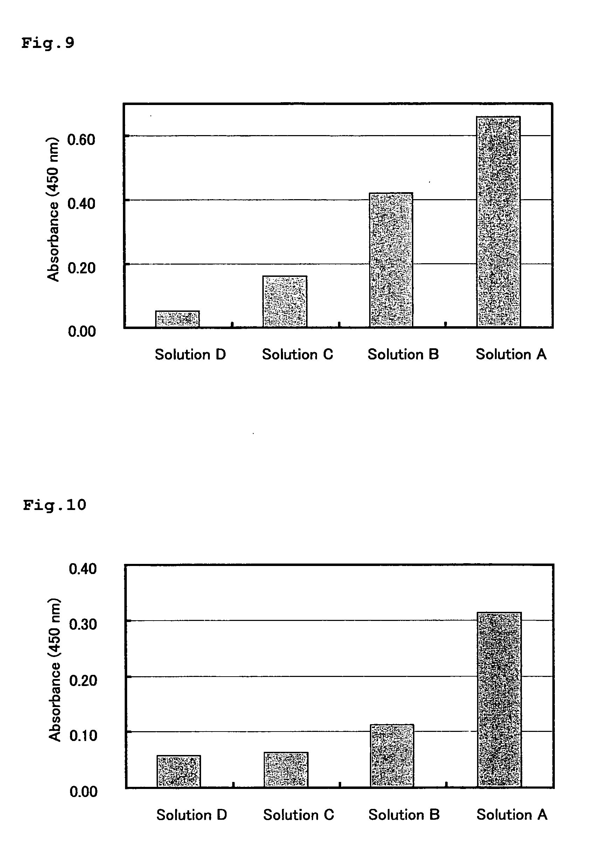

Fig. 9 is a drawing showing results obtained by using 0.5 µg/mL of a biotin-labeled methylcytosine antibody and 5'-end FITC-labeled oligonucleotide F6 in Example 9. DNA amounts measured by absorbance (450nm) are shown for Solution A (100 ng/5 µL TE buffer solution), Solution B (10 ng/5 µL TE buffer solution), Solution C (1 ng/5 µL TE buffer solution), and Solution D (0 ng/5 µL TE buffer solution (negative control solution)), respectively in this order from the right.
Fig. 10 is a drawing showing results obtained by using 0.5 µg/mL of a biotin-labeled methylcytosine antibody and 5'-end FITC-labeled oligonucleotide F3 in Example 10. DNA amounts measured by absorbance (450nm) are shown for Solution A (10 ng/10 µL TE buffer solution), Solution B (1 ng/10 µL TE buffer solution), Solution C (0.1 ng/10 µL TE buffer solution), and Solution D (0 ng/10 µL TE buffer solution (negative control solution)), respectively in this order from the right.

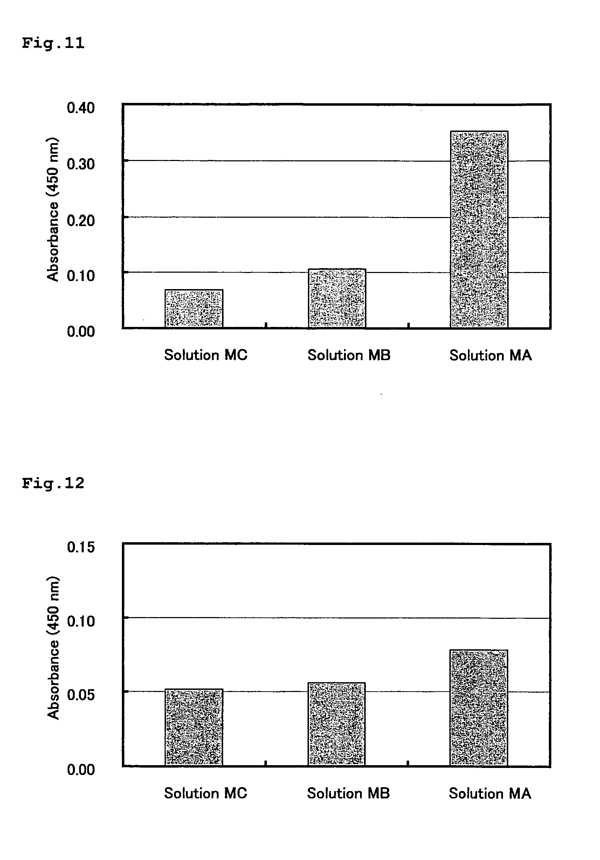

Fig. 11 is a. drawing showing results obtained by using 0.5 µg/mL of a biotin-labeled methylcytosine antibody and 5'-end FITC-labeled oligonucleotide F3 in Example 11. DNA amounts measured by absorbance (450nm) are shown for Solution MA (10 ng each/20 µL TE buffer solution), Solution MB (1 ng each/20 µL TE buffer solution), and Solution MC (0 ng each/20 µL TE buffer solution (negative control solution)), respectively in this order from the right.
Fig. 12 is a drawing showing results obtained by using 0.5 µg/mL of a biotin-labeled methylcytosine antibody and 5'-end FITC-labeled oligonucleotide F4 in Example 12. DNA amounts measured by absorbance (450nm) are shown for Solution MA (10 ng each/20 µL TE buffer solution), Solution MB (1 ng each/20 µL TE buffer solution), Solution MC (0 ng each/20 µL TE buffer solution (negative control solution)), respectively in this order from the right.

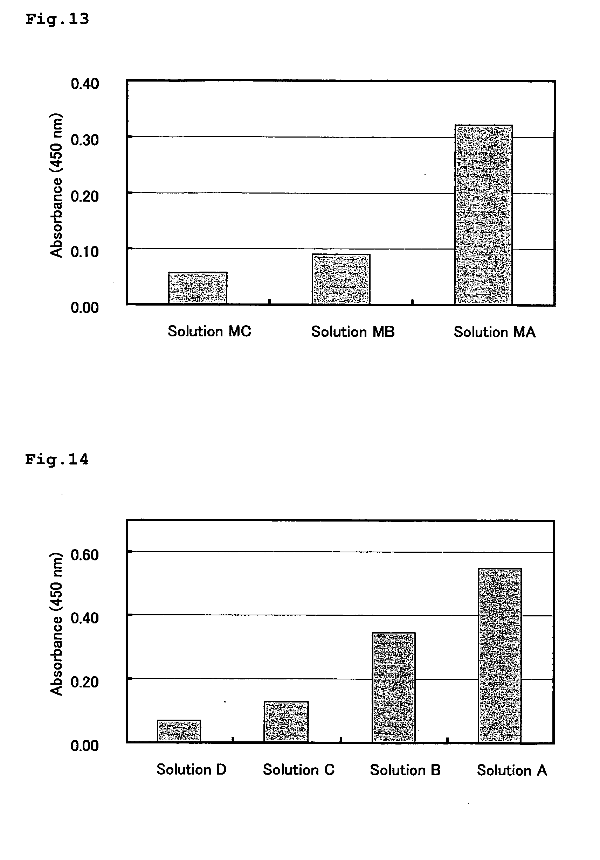

Fig. 13 is a drawing showing results obtained by using 0.5 µg/mL of a biotin-labeled methylcytosine antibody and 5'-end FITC-labeled oligonucleotides F3 and F4 in Example 13. DNA amounts measured by absorbance (450nm) are shown for Solution MA (10 ng each/20 µL TE buffer solution), Solution MB (1 ng each/20 µL TE buffer solution), and Solution MC (0 ng each/20 µL TE buffer solution (negative control solution)), respectively in this order from the right.
Fig. 14 is a drawing showing results obtained by using 0.5 µg/mL of a biotin-labeled methylcytosine antibody and 5'-end FITC-labeled oligonucleotide F5 in Example 14. DNA amounts measured by absorbance (450nm) are shown for Solution A (100 ng/5 µL TE buffer solution), Solution B (10 ng/5 µL TE buffer solution), Solution C (1 ng/5 µL TE buffer solution), and Solution D (0 ng/5 µL TE buffer solution (negative control solution)), respectively in this order from the right.

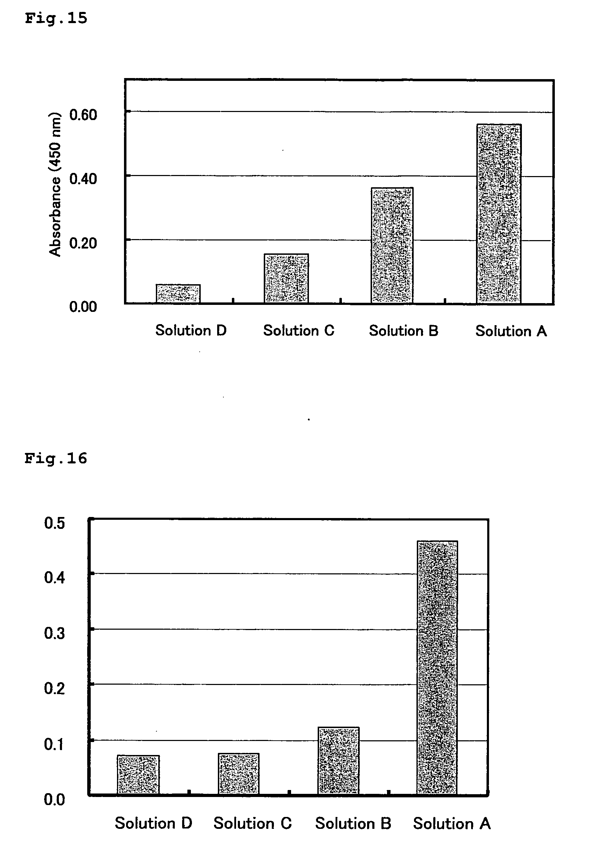

Fig. 15 is a drawing showing results obtained by using 0.5 µg/mL of a biotin-labeled methylcytosine antibody and 5'-end FITC-labeled oligonucleotide F6 in Example 15. DNA amounts measured by absorbance (450nm) are shown for Solution A (100 ng/5 µL TE buffer solution), Solution B (10 ng/5 µL TE buffer solution), Solution C (1 ng/5 µL TE buffer solution), and Solution D (0 ng/5 µL TE buffer solution (negative control solution)), respectively in this order from the right.
Fig. 16 is a drawing showing results obtained by conducting Treatment 1 and detecting DNA using a biotin-labeled methylcytosine antibody and 5'-end FITC-labeled oligonucleotide F6 in Example 16. Measured absorbances (450nm) are shown for Solution A (10 ng/10 µL rat serum solution), Solution B (1 ng/10 µL rat serum solution), Solution C (0.1 ng/10 µL rat serum solution), and Solution D (rat serum (negative control solution)), respectively in this order from the right.

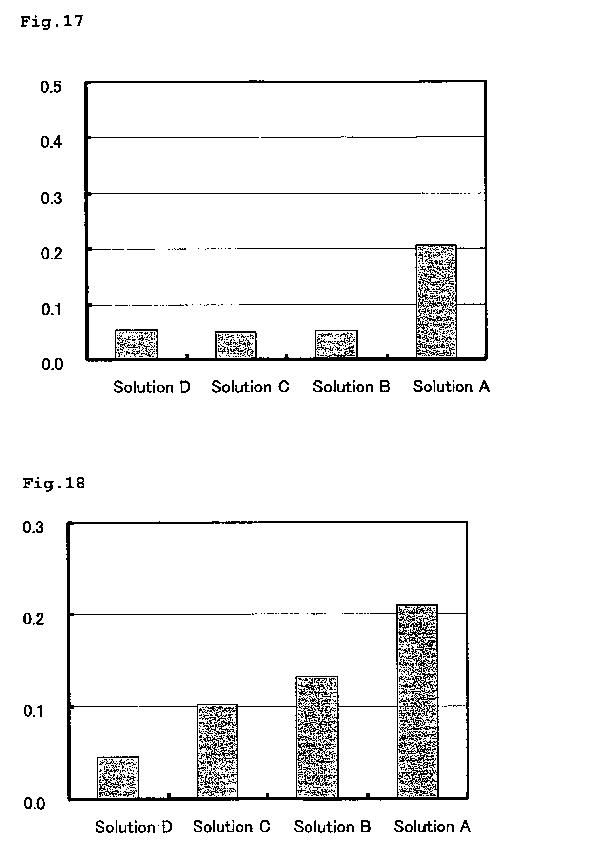

Fig. 17 is a drawing showing results obtained by conducting Treatment 2 and detecting DNA using a biotin-labeled methylcytosine antibody and 5'-end FITC-labeled oligonucleotide F6 in Example 16. Measured absorbances (450nm) are shown for Solution A (10 ng/10 µL rat serum solution), Solution B (1 ng/10 µL rat serum solution), Solution C (0.1 ng/10 µL rat serum solution), and Solution D (rat serum (negative control solution)), respectively in this order from the right.
Fig. 18 is a drawing showing results obtained by detecting DNA using a biotin-labeled methylcytosine antibody and 5'-end FITC-labeled oligonucleotide F6 in Example 17. Measured absorbances (450nm) are shown for Solution A (4 ng/40 µL human serum solution), Solution B (2 ng/40 µL human serum solution), Solution C (1 ng/40 µL human serum solution), and Solution D (human serum (negative control solution)), respectively in this order from the right.

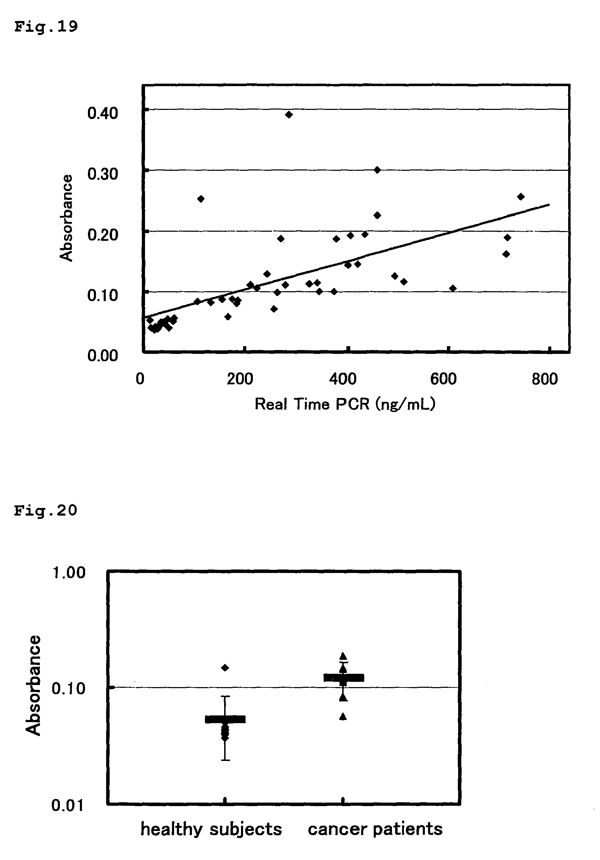

Fig. 19 is a drawing showing a comparison between a result detected by using a biotin-labeled methylcytosine antibody and 5'-end FITC-labeled oligonucleotide F6 and a result quantified by real time PCR in Example 18. In Fig. 19, the detection result is plotted on the vertical axis, and the quantification result by real-time PCR is plotted on the horizontal axis. The straight lines in the graph represent regression line (thick line) and standard error range (thin line).
Fig. 20 shows DNA detection results using a biotin-labeled methylcytosine antibody and 5'-end FITC-labeled oligonucleotide F6 for human serum samples aged 57 or younger in Example 18, which are plotted separately for cancer patients and healthy subjects, together with respective mean values and standard deviations.

### MODE FOR CARRYING OUT THE INVENTION

Examples of the "specimen" in the present method include (a) mammalian blood, body fluid, excreta, body secretion, cell lysate, or tissue lysate, (b) DNA extracted from one selected from the group consisting of mammalian blood, body fluid, excreta, body secretion, cell lysate and tissue lysate, (c) DNA prepared by using as a template RNA extracted from one selected from the group consisting of mammalian tissue, cell, tissue lysate and cell lysate, (e) DNA extracted from cell, fungus or virus, and (f) DNA prepared by using as a template RNA extracted from cell, fungus or virus. The term "tissue" means broadly including blood and lymph node.

The term "mammal" means animals classified into animal kingdom, Chordata, Chordate subphylum, and Mammalia, and concrete examples include human being, monkey, marmoset, guinea pig, rat, mouse, cattle, sheep, dog, and cat.

The term "body fluid" means a liquid existing between cells constituting an individual body, and concretely, plasma and interstitial fluid are recited, and it often functions to maintain homeostasis of an individual body. More concrete examples include lymph, tissue fluid (interinstitutional fluid, intercellular fluid, interstitial fluid), celomic fluid, serous cavity fluid, pleural effusion, ascetic fluid, pericardial fluid, cerebral fluid (spinal fluid), joint fluid (spinal fluid), eye aqueous fluid (aqueous fluid), and cerebrospinal fluid.

The term "body secretion" is a secretion from an exocrine gland, and concrete examples include saliva, gastric juice, bile, pancreatic juice, intestinal juice, sweat, tear, runny nose, semen, vaginal lubricant, amniotic fluid, and milk.

When the specimen is blood, body fluid or body secretion of a human being, a sample collected for a clinical test in a regular health check of human may be utilized.

Examples of the "cell lysate" include lysates containing intracellular fluids obtained by grinding cells, such as cell strains, primary cultured cells or blood cells, cultured in a plate for cell culture. As a method of grinding cells, a method based on sonication, a method using a surfactant, a method of using an alkaline solution and the like are recited. For lysing cells, a commercially available kit and the like may be used.

For example, after culturing cells to be confluent in a 10 cm plate, the culture solution is removed, and 0.6 mL of a RIPA buffer 1x TBS, 1% nonidet P-40, 0.5% sodium deoxysholate, 0.1% SDS, 0.004% sodium azide) is added to the plate. After shaking slowly the plate at 4°C for 15 minutes, cells adhered on the plate are removed by using a scraper or the like, and the liquid on the plate is transferred to a microtube. After adding 10 mg/mL PMSF in an amount of 1/10 volume of the liquid, the tube is left still on ice for 30 to 60 minutes, the solution is centrifuged at 4°C for 10 minutes at 10,000xg, to obtain the supernatant as a cell lysate.

As the "tissue lysate", lysates containing intracellular fluids obtained by grinding cells in tissues collected from animals such as mammals can be recited.

Concretely, after measuring the weight of a tissue obtained from an animal, the tissue is cut into small pieces with the use of a razor or the like. When a frozen tissue is used, it is necessary to make a smaller piece. After cutting, an ice-cooled RIPA buffer is added in a rate of 3 mL per 1 g of tissue, and homogenized at 4°C. Here, as the RIPA buffer, a protease inhibitor, a phosphatase inhibitor and the like may be added, and for example, 10 mg/mL PMSF in an amount of 1/10 volume of the RIPA buffer may be added. For homogenization, a sonicator or a pressurized cell grinder is used. In an operation of homogenization, a homogenized liquid is constantly kept at 4°C for preventing heat generation. The homogenized liquid is transferred to a microtube, and centrifuged at 4°C for 10 minutes at 10,000xg, and the supernatant is obtained as a tissue lysate.

Examples of the "specimen" in the present method include samples and surface adhered matters collected from foods, rivers, soils or general commercial products, and microorganisms such as fungi, cells, viruses and nucleic acids thereof can be contained.

Examples of the DNA used as a specimen include genomic DNA obtained by extraction from the biological sample or the microorganism, and DNA fragment or RNA derived from genomic DNA. For obtaining genomic DNA from a sample derived from a mammal, for example, a commercially available DNA extraction kit and the like may be used. For obtaining DNA from RNA, a reverse transcriptase such as a commercially available cDNA preparation kit and the like may be used. As the specimen, artificially synthesized DNA may be used.

The term "target DNA region" (hereinafter, sometimes referred to as a target region) in the present method means a DNA region intended to be detected or quantified by the present method in DNA contained in a specimen. The target DNA region is represented by a nucleotide sequence on DNA when the specimen is DNA. When the specimen is RNA, the target DNA region is represented by a nucleotide sequence on DNA prepared from RNA by a reverse transcriptase, and is a complementary nucleotide sequence of a prescribed nucleotide sequence to be detected on RNA. In the present method, when cytosine is methylated and detected or quantified, a target region desirably contains a region abundantly containing cytosine or CpG as will be described later.

First step is a step of preparing from a specimen DNA for which a target DNA region is to be detected.

Examples of DNA prepared in First step include a DNA sample digested in advance with a restriction enzyme recognition cleavage site for which in not present in the target DNA region possessed by the DNA, a DNA sample purified in advance, free DNA in blood, DNA derived from microbial genome, and DNA prepared from RNA in a specimen by a reverse transcriptase. As DNA prepared in First step, for example, DNA that is designed based on gene information of the specimen and artificially synthesized may be recited.

When blood is used as a specimen, plasma or serum is prepared from blood by a routine method, and the prepared plasma or serum is used as a specimen, and free DNA (containing DNA derived from cancer cells such as gastric cancer cells) contained therein is analyzed, and thus DNA derived from cancer cells such as gastric cancer cells can be analyzed away from DNA derived from hemocytes, and sensitivity of detecting cancer cells such as gastric cancer cells, and tissues containing the same can be improved.

Examples of DNA prepared in First step include DNA derived from microorganisms such as gram-positive bacteria, gram-negative bacteria, fungi, viruses and pathogenic protozoans, and DNA obtained from RNA derived from such microorganisms by a reverse transcriptase. For example, genomic DNA or DNA prepared by a reverse transcriptase from RNA of Mycoplasma genitalium, Mycoplasma pneumoniae, Borrelia burgdorferi B31, Rickettsia prowazekii, Treponema pallidum, Chlamydia pneumoniae, Chlamydia trachomatis, Helicobacter pylori J99, Helicobacter pylori 26695, Haemophilus influenzae Rd, Mycobacterium tuberculosis H37Rv, Pseudomonas aeruginosa, Legionella pneumophila, Serratia marcescens, Escherichia coli, Listeria monocytogenes, Salmonella enterica, Campylobacter jejuni subsp. Jejuni, Staphylococcus aureus, Vibrio parahaemolyticus, Bacillusu cereus, Clostridium botulinum, Clostridium perfringens, Yersinia enterocolitica, Yersinia pseudotuberuculosis, Trichophyton ruburum, Trichophyton mentagrophytes, Candida albicans, Cryptococcus neoformans, Aspergillus fumigatus, Pneumocystis carinii, Coccidioides immitis, Cytomegalovirus, human herpesvirus 5, Epstein-Barr virus, Human Immunodeficiency Virus, Human Papilloma Virus, Enterovirus, Norovirus Influenza Virus, Toxoplasma gondii, Cryptosporidium parvum, or Entamoeba histolytica may be used for detection of a microorganism responsible for an infection in a specimen, or a microorganism responsible for a food poisoning in food.

For preparing genomic DNA, for example, when the specimen is a sample derived from a mammal, a commercially available DNA extraction kit (Genfind v2 Kit (available from BECKMAN COULTER), FastPure DNA Kit (available from TAKARA BIO INC.)) and the like may be used.

When the specimen is a microorganism such as fungus, genomic DNA may be prepared by a general preparation method of yeast genome or the like as described in Methods in Yeast Genetics (Cold Spring Harbor Laboratory Press), and when the specimen is a prokaryote such as Escherichia coli, a general preparation method of microorganism genome or the like as described in Molecular Cloning -A Laboratory Manual- (Cold Spring Harbor Laboratory Press) may be used.

When the specimen is a food sample, DNA may be prepared after separating a microorganism or the like from the food, and genomic DNA of non-microorganism and genome derived from a microorganism contained in the food may be obtained at the same time. When the specimen is a tissue derived from a mammal, and the target DNA region is DNA derived from a virus, RNA may be extracted from the tissue using such as a commercially available RNA extraction kit (ISOGEN(311-02501)(available from NIPPON GENE CO., LTD.), or FastRNA Pro Green Kit (available from Funakoshi Corporation), FastRNA Pro Blue Kit (available from Funakoshi Corporation), FastRNA Pro Red Kit (available from Funakoshi Corporation), and the like), and DNA may be obtained by a reverse transcriptase. When the specimen is a specimen derived from a mammal, viral DNA may be extracted after extracting virus particles, or after extracting virus particles, viral RNA may be extracted using a commercially available kit (QuickGene RNA tissue kit SII, available FUJIFILM Corporation) or the like, and DNA derived from the virus may be obtained by a reverse transcriptase. RNA may be extracted from a tissue infected by a virus, and DNA derived from the virus may be obtained by a reverse transcriptase, or DNA may be obtained from a tissue infected by a virus, and DNA derived from the virus may be obtained. When DNA is obtained from RNA by a reverse transcriptase, a commercially available kit (Transcripter high fidelity cDNA synthesis kit, available from Roche Diagnostics K.K.) and the like may be used.

In "methylated DNA", any of four kinds of bases constituting gene (genomic DNA) is methylated. For example, in a mammal is known a phenomenon that only cytosine in a nucleotide sequence represented by 5'-CG-3' (C represents cytosine, and G represents guanine. Hereinafter, the nucleotide sequence is occasionally denoted by "CpG") is methylated. A methylation site of cytosine is position 5. In DNA duplication antecedent to cell division, only cytosine in "CpG" in a template chain derived from a parent cell is methylated in nascent double-stranded DNA, and cytosine in "CpG" in a nascent DNA chain is also methylated rapidly by the action of a methyltransferase. In this cytosine methylation is methylated cytosine in CpG in a nascent DNA chain that complementarily binds to CpG containing methylated cytosine in a DNA chain derived from a parent cell. Therefore, the methylation condition of DNA of the parent cell is taken over as it is to new two sets of DNA after DNA duplication. The term "CpG pair" means a double-stranded DNA in which a nucleotide sequence represented by CpG binds to CpG complementary to the sequence.

The term "single-stranded methylated DNA" means single-stranded DNA in which is methylated cytosine at a position 5 in a nucleotide sequence represented by 5'-CG-3' in a nucleotide sequence of the single-stranded DNA.

Examples of the "target DNA region" include promoter regions, untranslated regions or translated regions (coding regions) of useful protein genes such as Lysyl oxidase, HRAS-like suppressor, bA305P22.2.1, Gamma filamin, HAND1, Homologue of RIKEN 2210016F16, FLJ32130, PPARG angiopoietin-related protein, Thrombomodulin, p53-responsive gene 2, Fibrillin 2, Neurofilament 3, disintegrin and metalloproteinase domain 23, G protein-coupled receptor 7, G-protein coupled somatostatin and angiotensin-like peptide receptor, and Solute carrier family 6 neurotransmitter transporter noradrenalin member 2, and preferably include DNA regions containing one or more CpG present in these nucleotide sequences. In the present method, methylated DNA of "target DNA region" may be detected or quantified individually, and, for example, when more methylated DNA of "target DNA region" is detected in one detection system, the quantification accuracy and detection sensitivity are improved correspondingly.

To be more specific, when the useful protein gene is a Lysyl oxidase gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Lysyl oxidase gene derived from human, and a promoter region located 5' upstream of the same can be recited, and more concretely, the nucleotide sequence of SEQ ID NO: 1 (corresponding to a nucleotide sequence represented by base No. 16001 to 18661 in the nucleotide sequence described in Genbank Accession No. AF270645) can be recited. In the nucleotide sequence of SEQ ID NO: 1, ATG codon encoding methionine at amino terminal of Lysyl oxidase protein derived from human is represented in base No. 2031 to 2033, and a nucleotide sequence of the above exon 1 is represented in base No. 1957 to 2661.

To be more specific, when the useful protein gene is a HRAS-like suppressor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HRAS-like suppressor gene derived from human, and a promoter region located 5' upstream of the same can be recited, and more concretely, the nucleotide sequence of SEQ ID NO: 2 (corresponding to a nucleotide sequence represented by base No. 172001 to 173953 in the nucleotide sequence described in Genbank Accession No. AC068162) can be recited. In the nucleotide sequence of SEQ ID NO: 2, the nucleotide sequence of exon 1 of a HRAS-like suppressor gene derived from human is represented in base No. 1743 to 1953.

To be more specific, when the useful protein gene is a bA305P22.2.1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a bA305P22.2.1 gene derived from human, and a promoter region located 5' upstream of the same can be recited, and more concretely, the nucleotide sequence of SEQ ID NO: 3 (corresponding to a nucleotide sequence represented by base No. 13001 to 13889 in the nucleotide sequence described in Genbank Accession No. AL121673) can be recited. In the nucleotide sequence of SEQ ID NO: 3, ATG codon encoding methionine at amino terminal of bA305P22.2.1 protein derived from human is represented in base No. 849 to 851, and a nucleotide sequence of the above exon 1 is represented in base No. 663 to 889.

To be more specific, when the useful protein gene is a Gamma filamin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Gamma filamin gene derived from human, and a promoter region located 5' upstream of the same can be recited, and more concretely, the nucleotide sequence of SEQ ID NO: 4 (corresponding to a complementary sequence to a nucleotide sequence represented by base No. 63528 to 64390 in the nucleotide sequence described in Genbank Accession No. AC074373) can be recited. In the nucleotide sequence of SEQ ID NO: 4, ATG codon encoding methionine at amino terminal of Gamma filamin protein derived from human is represented in base No. 572 to 574, and a nucleotide sequence of the above exon 1 is represented in base No. 463 to 863.

To be more specific, when the useful protein gene is a HAND1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HAND1 gene derived from human, and a promoter region located 5' upstream of the same can be recited, and more concretely, the nucleotide sequence of SEQ ID NO: 5 (corresponding to a complementary sequence to a nucleotide sequence represented by base No. 24303 to 26500 in the nucleotide sequence described in Genbank Accession No. AC026688) can be recited. In the nucleotide sequence of SEQ ID NO: 5, ATG codon encoding methionine at amino terminal of HAND1 protein derived from human is represented in base No. 1656 to 1658, and a nucleotide sequence of the above exon 1 is represented in base No. 1400 to 2198.

To be more specific, when the useful protein gene is a Homologue of RIKEN 2210016F16 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Homologue of RIKEN 2210016F16 gene derived from human, and a promoter region located 5' upstream of the same can be recited, and more concretely, the nucleotide sequence of SEQ ID NO: 6 (corresponding to a complementary nucleotide sequence to a nucleotide sequence represented by base No. 157056 to 159000 in the nucleotide sequence described in Genbank Accession No. AL354733) can be recited. In the nucleotide sequence of SEQ ID NO: 6, a nucleotide sequence of exon 1 of a Homologue of a RIKEN 2210016F16 gene derived from human is represented in base No. 1392 to 1945.

To be more specific, when the useful protein gene is a FLJ32130 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a FLJ32130 gene derived from human, and a promoter region located 5' upstream of the same can be recited, and more concretely, the nucleotide sequence of SEQ ID NO: 7 (corresponding to a complementary nucleotide sequence to a nucleotide sequence represented by base No. 1 to 2379 in the nucleotide sequence described in Genbank Accession No. AC002310) can be recited. In the nucleotide sequence of SEQ ID NO: 7, ATG codon encoding methionine at amino terminal of FLJ32130 protein derived from human is represented in base No. 2136 to 2138, and a nucleotide sequence assumed to be the above exon 1 is represented in base No. 2136 to 2379.

To be more specific, when the useful protein gene is a PPARG angiopoietin-related protein gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a PPARG angiopoietin-related protein gene derived from human, and a promoter region located 5' upstream of the same can be recited, and more concretely, the nucleotide sequence of SEQ ID NO: 8 can be recited. In the nucleotide sequence of SEQ ID NO: 8, ATG codon encoding methionine at amino terminal of PPARG angiopoietin-related protein derived from human is represented in base No. 717 to 719, and a nucleotide sequence of the 5' side part of the above exon 1 is represented in base No. 1957 to 2661.

To be more specific, when the useful protein gene is a Thrombomodulin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Thrombomodulin gene derived from human, and a promoter region located 5' upstream of the same can be recited, and more concretely, the nucleotide sequence of SEQ ID NO: 9 (corresponding to a nucleotide sequence represented by base No. 1 to 6096 in the nucleotide sequence described in Genbank Accession No. AF495471) can be recited. In the nucleotide sequence of SEQ ID NO: 9, ATG codon encoding methionine at amino terminal of Thrombomodulin protein derived from human is represented in base No. 2590 to 2592, and a nucleotide sequence of the above exon 1 is represented in base No. 2048 to 6096.

To be more specific, when the useful protein gene is a p53-responsive gene 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a p53-responsive gene 2 gene derived from human, and a promoter region located 5' upstream of the same can be recited, and more concretely, the nucleotide sequence of SEQ ID NO: 10 (corresponding to a complementary sequence to a nucleotide sequence represented by base No. 113501 to 116000 in the nucleotide sequence described in Genbank Accession No. AC009471) can be recited. In the nucleotide sequence of SEQ ID NO: 10, a nucleotide sequence of exon 1 of a p53-responsive gene 2 gene derived from human is represented in base No. 1558 to 1808.

To be more specific, when the useful protein gene is a Fibrillin2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Fibrillin2 gene derived from human, and a promoter region located 5' upstream of the same can be recited, and more concretely, the nucleotide sequence of SEQ ID NO: 11 (corresponding to a complementary sequence to a nucleotide sequence represented by base No. 118801 to 121000 in the nucleotide sequence described in Genbank Accession No. AC113387) can be recited. In the nucleotide sequence of SEQ ID NO: 11, a nucleotide sequence of exon 1 of a Fibrillin2 gene derived from human is represented in base No. 1091 to 1345.

To be more specific, when the useful protein gene is a Neurofilament3 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Neurofilament3 gene derived from human, and a promoter region located 5' upstream of the same can be recited, and more concretely, the nucleotide sequence of SEQ ID NO: 12 (corresponding to a complementary sequence to a nucleotide sequence represented by base No. 28001 to 30000 in the nucleotide sequence described in Genbank Accession No. AF106564) can be recited. In the nucleotide sequence of SEQ ID NO: 12, a nucleotide sequence of exon 1 of a Neurofilament3 gene derived from human is represented in base No. 614 to 1694.

To be more specific, when the useful protein gene is a disintegrin and metalloproteinase domain 23 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a disintegrin and metalloproteinase domain 23 gene derived from human, and a promoter region located 5' upstream of the same can be recited, and more concretely, the nucleotide sequence of SEQ ID NO: 13 (corresponding to a nucleotide sequence represented by base No. 21001 to 23300 in the nucleotide sequence described in Genbank Accession No. AC009225) can be recited. In the nucleotide sequence of SEQ ID NO: 13, a nucleotide sequence of exon 1 of a disintegrin and metalloproteinase domain 23 gene derived from human is represented in base No. 1194 to 1630.

To be more specific, when the useful protein gene is a G protein-coupled receptor 7 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G protein-coupled receptor 7 gene derived from human, and a promoter region located 5' upstream of the same can be recited, and more concretely, the nucleotide sequence of SEQ ID NO: 14 (corresponding to a nucleotide sequence represented by base No. 75001 to 78000 in the nucleotide sequence described in Genbank Accession No. AC009800) can be recited. In the nucleotide sequence of SEQ ID NO: 14, a nucleotide sequence of exon 1 of a G protein-coupled receptor 7 gene derived from human is represented in base No. 1666 to 2652.

To be more specific, when the useful protein gene is a G-protein coupled somatostatin and angiotensin-like peptide receptor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G-protein coupled somatostatin and angiotensin-like peptide receptor gene derived from human, and a promoter region located 5' upstream of the same can be recited, and more concretely, the nucleotide sequence of SEQ ID NO: 15 (corresponding to a complementary sequence to a nucleotide sequence represented by base No. 57001 to 60000 in the nucleotide sequence described in Genbank Accession No. AC008971) can be recited. In the nucleotide sequence of SEQ ID NO: 15, a nucleotide sequence of exon 1 of a G-protein coupled somatostatin and angiotensin-like peptide receptor gene derived from human is represented in base No. 776 to 2632.

To be more specific, when the useful protein gene is a Solute carrier family 6 neurotransmitter transporter noradrenalin member 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Solute carrier family 6 neurotransmitter transporter noradrenalin member 2 gene derived from human, and a promoter region located 5' upstream of the same can be recited, and more concretely, the nucleotide sequence of SEQ ID NO: 16 (corresponding to a complementary sequence to a nucleotide sequence represented by base No. 78801 to 81000 in the nucleotide sequence described in Genbank Accession No. AC026802) can be recited. In the nucleotide sequence of SEQ ID NO: 16, a nucleotide sequence of exon 1 of a Solute carrier family 6 neurotransmitter transporter noradrenalin member 2 gene derived from human is represented in base No. 1479 to 1804.

Second step is a step of treating the DNA prepared in First step with a DNA methylation enzyme.

The "DNA methylation enzyme" means an enzyme that methylates a base in DNA, and various kinds DNA methylation enzymes are isolated from mammalian cells, bacteria and the like. DNA methylation enzymes are classified into several kinds such as adenine methylation enzymes, and cytosine methylation enzymes according to the kind of the base of a substrate. A cytosine methylation enzyme is an enzyme that recognizes a specific sequence in a DNA nucleotide sequence, and methylates cytosine near the sequence, and different cytosine methylation enzymes are known according to the recognized nucleotide sequences.

A number of methylation reactions of DNA catalyzed by a DNA methylation enzyme are found from a primitive immune system called a restriction-modification system. The restriction-modification system is a function that digests foreign DNA (in particular, bacteriophage) with a restriction enzyme after regularly methylating the entire genome functioning in bacteria to protect it from being digested by a restriction enzyme (restriction endonuclease) that recognizes a specific sequence, and is a system for protecting a microbial genome from bacteriophage infection. Enzymes functioning in methylation of genome are known to methylate cytosine or adenine, and often known to methylate nitrogen at position 6 (N6) or carbon at position 5 (C5) of a purine residue. Among these enzymes, known as a cytosine methylation enzyme that methylates C5 of cytosine are SssI (M.SssI) methylase, AluI methylase, HhaI methylase, HpaII methylase, MspI methylase, HaeIII methylase, and so on. These enzymes that methylate position C5 of cytosine recognize different nucleotide sequences, and a cytosine methylation enzyme that recognizes CpG is only SssI.

As a methylation reaction of DNA in human genome, methylation at position 5 (C5) of cytosine in CpG is known as epigenetics (the mechanism generating diversity of gene expression independent of gene sequence), and as such a cytosine methylation enzyme, DNA methyltransferase is known. As a DNA methyltransferase, DnmtI methyltransferase is known.

In human cells, since position C5 of cytosine in a CpG sequence is methylated, for methylating genome artificially, the same position of the same cytosine in the same sequence (CpG) with methylation in a human cell can be methylated by using SssI.

For methylating DNA by a cytosine methylation enzyme, concretely, for example, a DNA sample is added with 5 µL of an optimum 10x buffer (NEBuffer2 (available from NEB Inc.)), 0.5 µL of S-adenosyl methionine (3.2 mM, available from NEB Inc.) and 0.5 µL of cytosine methylation enzyme SssI(available from NEB Inc.) and then the resultant mixture is added with sterilized ultrapure water to make the liquid amount 50 µL, and then incubated at 37°C for 30 minutes. Since methylation in Second step is executed for making the target DNA region bind with a support by making it bind with the methylated DNA antibody, Second step is not necessarily executed insofar as the target DNA region of the extracted DNA sample is methylated.

In Second step, when a methylated base constituting the methylated DNA modified with the DNA methylation enzyme differs from the methylated base possessed by the later-described detection oligonucleotide, the methylated base on the detection oligonucleotide can be used as an identification function. For example, when the detection oligonucleotide is 6-methyladenine, and cytosine can be modified into 5-methylcytosine in Second step, a 6-methyladenine antibody may be used as the identification function of the detection oligonucleotide, and immobilization to a support can be achieved using a methylcytosine antibody. Concretely, when modification is achieved by SssI methylase in Second step, immobilization to a support may be achieved by using a methylcytosine antibody. That is, when the detection oligonucleotide has methyladenine, and the detection oligonucleotide is detected by a methyladenine antibody, it is possible to detect only methyladenine of the detection oligonucleotide without detecting methylcytosine of DNA having a target DNA region.

Third step is a step of preparing single-stranded methylated DNA from the DNA treated with a DNA methylation enzyme obtained in Second step, and making a detection oligonucleotide bind with the single-stranded methylated DNA having a target DNA region to obtain a test DNA complex.

The "detection oligonucleotide" in the present method is such an oligonucleotide that bases of nucleotides constituting the oligonucleotide has an identification function for detecting or quantifying the detection oligonucleotide, and an adhesion sequence for detection which is a nucleotide sequence for binding by complementation with the DNA having a target DNA region.

The "detection oligonucleotide" in the present invention may comprise a repetitive sequence in human genome as will be described later, a nucleotide sequence of an overlapping gene or a pseudo gene or a nucleotide sequence capable of complementarily binding with a part of the same. Concretely, for example, the nucleotide sequences of SEQ ID NOs: 38 and 40, nucleotide sequences having a complementary sequence therewith and the like are recited.

The adhesion sequence for detection is a nucleotide sequence required for forming a conjugate (double strand) with the single-stranded DNA containing a target DNA region, namely, a nucleotide sequence containing a sequence capable of binding with a part of the nucleotide sequence of the target DNA region by complementary base pairing, or a nucleotide sequence containing a nucleotide sequence that is complementary to a part of a nucleotide sequence in a further 5'-end side DNA region than 5'-end of the target DNA region or a nucleotide sequence containing a nucleotide sequence that is complementary to a part of a nucleotide sequence in a further 3'-end side DNA region than 3' -end of the target DNA region, and desirably not inhibiting binding of the methylated DNA antibody and the methylated DNA in the target DNA region.

The expression "not inhibiting binding of the methylated DNA antibody and the methylated DNA in the target DNA region" means that complementary binding between the present oligonucleotide and the single-stranded DNA does not occur in the occupied space required for the methylated DNA antibody to bind with the methylated single-stranded DNA. That is, it is supposed that the methylated DNA antibody occupies not only the methylated base (cytosine) to which the methylated DNA antibody directly binds, but also the peripheral space where the methylated base (cytosine) exists for binding with the methylated base (cytosine). Therefore, it suffices that the adhesion sequence for detection fails to complementary bind with the single-stranded DNA in the occupied space required for the methylated DNA antibody to bind with the methylated DNA. The adhesion sequence for detection to be bound with the single-stranded DNA is not necessarily one kind, and two or more kinds may be used unless binding of the methylated DNA antibody is inhibited. By using a plurality of the present oligonucleotides, it is possible to improve the quantity accuracy and detection sensitivity.

The expression "obtaining single-stranded methylated DNA having a target DNA region, and making a detection oligonucleotide bind with the single-stranded methylated DNA to obtain a test DNA complex" in Third step means forming a test DNA complex made up of methylated DNA having a target DNA region (hereinafter, also sometimes referred to as single-stranded methylated DNA) and the detection oligonucleotide that are bound complementarily. Concretely, the single-stranded methylated DNA methylated by a DNA methylation enzyme in Second step is prepared into a 1 ng/ µL solution in Tris-HCl buffer (10 mM), and a detection oligonucleotide capable of binding with the single-stranded methylated DNA is prepared into a 0. 02 µM solution in Tris-HCl buffer (10 mM), and 10 µL of respective oligonucleotide solutions and a buffer (330 mM Tris-Acetate pH 7.9, 660 mM KOAc, 100 mM MgOAc₂, 5 mM Dithiothreitol), 10 µL of a 100 mM MgC1₂ solution, and 10 µL of a 1 mg/mL BSA solution are mixed, and the resultant mixture is further added with sterilized ultra pure water to make the liquid amount 100 µL. Then the mixture is heated at 95°C for 10 minutes, rapidly cooled to 70°C and retained at this temperature for 10 minutes, then cooled to 50°C and retained at this temperature for 10 minutes, retained at 37°C for 10 minutes, and then returned to room temperature, to promote formation of the test DNA complex of the single-stranded methylated DNA and the detection oligonucleotide.

The term "complementarily bind" means that double-stranded DNA is formed by base-pairing through a hydrogen bond between bases. For example, it means that bases that constitute respective single-stranded DNAs of a double strand forming DNA form a double strand by base-pairing between purine and pyrimidine, and more concretely, double-stranded DNA is formed by base-pairing through plural sequential hydrogen bonds between thymine and adenine, and guanine and cytosine. Binding based on complementation may be sometimes referred to as "complementarily binding". The term "complementarily binding" may be sometimes expressed by "capable of complementary binding", "capable of complementary base-pairing", "bind by complementation" or "bind (complementary bind (by base-pairing)) by complementation". Nucleotide sequences that are capable of complementarily binding may be sometimes expressed by "having complementation" or "complementary" to each other. It also means binding of inosine contained in an artificially prepared oligonucleotide with cytosine, adenine or thymine through hydrogen bonding.

In the present method, when the single-stranded methylated DNA and the detection oligonucleotide "bind by complementation", it is also included the case where a part of the nucleotide sequence constituting the adhesion sequence for detection of the detection oligonucleotide fails to base-pair with single-stranded methylated DNA. For example, the case where at least 75%, preferably 80% or more bases of the bases constituting the adhesion sequence for detection base-pair with the single-stranded methylated DNA, and the adhesion sequence for detection is able to bind with an oligonucleotide having at least 75% or more, preferably 80% or more homology with the test oligonucleotide is included.

In Third step of the present method, as a preferred aspect in forming the test DNA complex of the single-stranded methylated DNA and the detection oligonucleotide, for example, addition of a counter oligonucleotide is recited.

The "counter oligonucleotide" is obtained by dividing a polynucleotide having the same nucleotide sequence as the target DNA region into "short" oligonucleotides. Here, "short" means length of 10 to 100 bases, and more preferably 20 to 50 bases. The counter oligonucleotide is not designed on the nucleotide sequence where the detection oligonucleotide and the single-stranded methylated DNA bind. The counter oligonucleotide is added excessively compared to the target DNA region. When the target DNA region is a plus strand, it is added for preventing a complementary strand (minus strand) of the target DNA region and the target DNA region (plus strand) from rebinding by complementation when the target DNA region (plus strand) is made into a single-strand and caused to bind with the later-described immobilized methylated DNA antibody. This is because in making the methylated DNA antibody bind with the methylated target DNA region, and measuring an amount of target DNA or an index value correlated therewith, the methylated target region in a single strand state is more likely to bind with the methylated DNA antibody. The counter oligonucleotide is preferably added in an amount of at least 10 times, preferably 100 times or more compared to the target DNA region.

When the target DNA region in the DNA sample prepared in First step is single-stranded DNA, single-stranded methylated DNA can be obtained as the DNA sample without conducting any special operation for "preparing single-stranded methylated DNA" in Third step. Concretely, as the DNA sample, single-stranded DNA synthesized from RNA by a reverse transcriptase treatment, and single-stranded DNA obtained from a virus whose genome is single-stranded DNA, among the DNAs extracted from the virus are recited. When the target DNA region in the DNA sample prepared in First step is double-stranded DNA, an operation for making double-stranded DNA into single strands may be conducted for "preparing single-stranded methylated DNA" in Third step. In this case, concretely, the operation may include heating at 95°C for several minutes, followed by rapid cooling to 4°C or less.

The term "adhesion sequence for detection" in the detection oligonucleotide is an oligonucleotide comprising a nucleotide sequence complementary to a nucleotide sequence possessed by the target DNA region, and means a sequence having a homology of 75% or more, more preferably 90% or more with the nucleotide sequence of the target DNA region capable of pairing with the adhesion sequence for detection. It suffices that the adhesion sequence for detection does not inhibit binding between the later-described immobilized methylated DNA antibody and the test DNA complex. Further, "adhesion sequence for detection" is designed to have a nucleotide sequence that binds with the target DNA region or the vicinity of the target DNA region, and to be able to form a detection complex in Fourth step as will be described later. Only one or two or more adhesion sequences for detection may be designed in the same repetitive sequence (in the target DNA region) as will be described later. When two or more adhesion sequences are designed, it suffices that the plural adhesion sequences for detection and a specific adhesion sequence as will be described later do not mutually inhibit binding with the single-stranded methylated DNA.

Fourth step is a step of making an immobilized methylated DNA antibody bind with the test DNA complex obtained in Third step to obtain a detection complex.

The "immobilized methylated DNA antibody" is a methylated DNA antibody binding with a methylated base in DNA as an antigen, which is immobilized to a "support". As the antibody, preferably, an antibody having the property of recognizing and binding with the cytosine that is methylated at position 5 in single-stranded DNA, and more preferably a methylcytosine antibody may be used. Also a commercially available methylated DNA antibody capable of specifically recognizing DNA in a methylated state described in the specification and specifically binding with the same may be used.

As the "support", the material and form thereof are not particularly limited as far as methylated DNA antibody can bind thereto. For example, any form suited for use purpose may be employed, including the forms of tube, test plate, filter, disc, bead and the like. As the material, those used as supports for a usual immune measuring method, for example, synthetic resins such as polystyrene, polypropylene, polyacrylamide, polymethylmethacrylate, polysulfone, polyacrylonitrile and nylon, or those obtained by incorporating a reactive functional group such as sulfonic group, amino group or the like to the synthetic resins can be recited. Also, glass, polysaccharides or derivatives thereof (cellulose, nitrocellulose and the like), silica gel, porous ceramics, metal oxides and the like may be used.

The "support" may be a microparticle, and a microparticle as same as the support may be bound to the detection oligonucleotide As the microparticles, latex beads, gold colloids (gold nanoparticles) and the like are recited.

When the same kinds of microparticles are bound to the support and the detection oligonucleotide, the microparticle serving as the support, and the microparticle bound to the detection oligonucleotide are able to concurrently bind and aggregate on the methylated DNA having a target DNA region. This means that as a result of formation of a detection complex by binding of the immobilized methylated DNA antibody, the detection oligonucleotide, and the methylated single-stranded DNA, the microparticle serving as the support and the microparticle bound to the detection oligonucleotide form an aggregate. In this case, when the microparticle is a latex bead, the aggregate can be detected by change in turbidity. When the microparticle is a gold colloid (gold nanoparticle), the aggregate can be detected by change in color tone (pink to purple).

When a plurality of methylated DNA antibodies immobilized to supports are bound concurrently on DNA having one target DNA region, aggregation of microparticles can be detected by binding of the methylated DNA antibodies immobilized on microparticles, with plural methylated DNAs on the DNA having a target DNA region. For example, when the support is a latex bead, an aggregate of microparticles can be detected by change in turbidity. When the support is a gold colloid (gold nanoparticle), an aggregate of microparticles can be detected by color tone change (from pink to purple). In this case, an equivalent result to that obtained by adding the detection oligonucleotide can be obtained even when the detection oligonucleotide is not added.

An amount detected by aggregation of microparticles is correlated with a sum of DNA methylated by Second step. When DNA obtained in First step is DNA contained in a cell of a tissue, the degree of methylation of DNA contained in the cell of the tissue differs depending on the tissue, so that the degree of methylation of DNA obtained in Second step includes both the degree of methylation in the cell of the tissue, and the degree of methylation in Second step. That is, an amount detected by an aggregate of microparticles when a DNA methylation enzyme treatment is not executed in Second step is a value correlated with the degree of methylation in the cell of the tissue.

"Methylated DNA antibody" is an antibody that binds with a methylated base in DNA as an antigen. Concretely, methylcytosine antibody can be recited, and an antibody having the property of recognizing cytosine whose position 5 is methylated in single-stranded DNA and binding thereto can be recited. Commercially available methylated DNA antibodies may also be used as far as they specifically recognize DNA in a methylation state described in the present specification, and are capable of specifically binding thereto. Such a methylated DNA antibody can be prepared in a conventional method using a methylated base, methylated DNA or the like as an antigen. For concretely preparing a methylcytosine antibody, selection is made according to specific binding to methylcytosine in DNA as an indicator from antibodies prepared using DNA containing 5-methylcytidine, 5-methylcytosine, or 5-methylcytosine as an antigen. Considering the property of such immobilized methylated-DNA antibody (the fact that one antibody binds to one methylated base (cytosine)), improvements in quantification accuracy and detection sensitivity are expected by selecting the region where a number of methylated bases (cytosine), namely CpG, are present, as the target DNA region.

Known as antibodies that can be obtained by immunizing an animal with an antigen are an antibody of IgG fraction (polyclonal antibody), an antibody producing a single clone (monoclonal antibody) and the like that can be obtained by immunizing an animal with an antigen. In the present invention, since an antibody capable of specifically recognizing methylated DNA or methylcytosine is preferred, use of a monoclonal antibody is advisable.

As a method of preparing a monoclonal antibody, a procedure based on a cell fusion method can be recited. For example, in the cell fusion method, a hybridoma is prepared by allowing cell fusion between a spleen cell (B cell) derived from an immunized mouse and a myeloma cell, and an antibody produced by the hybridoma is selected for preparation of a methyl cytosine antibody (monoclonal antibody). When a monoclonal antibody is prepared by a cell fusion method, it is not necessary to purify an antigen, and for example, a mixture of 5-methyl cytidine, 5-methyl cytosine or DNA or the like containing 5-methyl cytosine may be administered as an antigen to an animal used for immunization. As an administration method, 5-methyl cytidine, 5-methyl cytosine or DNA or the like containing 5-methyl cytosine is directly administered to a mouse for production of an antibody. When an antibody is difficult to be produced, an antigen bound to a support may be used for immunization. Also, by thoroughly mixing an adjuvant solution (prepared, for example, by mixing liquid paraffin and Aracel A, and mixing killed tubercle bacilli as an adjuvant) and an antigen and administering the same, or immunizing via liposome incorporating the same, immunity of an antigen can be improved. Also a method involving adding equivalent amounts of a solution containing an antigen and an adjuvant solution, fully emulsifying them, and subcutaneously or intraperitoneally injecting the resultant mixture to a mouse, and a method of adding killed Bordetella pertussis as an adjuvant after mixing well with alum water are known. A mouse may be boosted intraperitoneally or intravenously after an appropriate term from initial immunization. When the amount of an antigen is small, a solution in which the antigen is suspended may be directly injected into a mouse spleen to effect immunization.

After exenterating a spleen and peeling an adipose tissue off after several days from the final immunization, a spleen cell suspension is prepared. The spleen cell is fused, for example, with an HGPRT-deficient myeloma cell to prepare a hybridoma. As a cell fusion agent, any means capable of efficiently fusing a spleen cell (B cell) and a myeloma cell is applicable, and for example, a method of using a hemagglutinating virus of Japan (HVJ), polyethyleneglycol (PEG) and the like are recited. Cell fusion may be conducted by a method using a high voltage pulse.

After the cell fusion operation, cells are cultured in an HAT medium, a clone of a hybridoma in which a spleen cell and a myeloma cell are fused is selected, and the cell is allowed to grow until screening becomes possible. In a method of detecting an antibody for selecting a hybridoma that produces an intended antibody, or a method of measuring a titer of an antibody, an antigen-antibody reaction system may be used. Concretely, as a method of measuring an antibody against a soluble antigen, a radioisotope immune assay (RIA), an enzyme-linked immunosorbent assay (ELISA) and the like can be recited.

Single-stranded DNA is able to bind with an anti-methylation antibody as far as CpG existing therein is methylated at least at one site. Therefore, the term "methylated" in the present method means DNA in which CpG existing therein is methylated at least at one site, and is not limited to DNA in which every CpG existing therein is methylated.

The "detection complex" in Fourth step means a complex made up of the test DNA complex obtained in Third step and the immobilized methylated DNA antibody bound to each other. Since the methylated DNA antibody is used as an entity that can be immobilized to a support, it suffices that the methylated DNA antibody can be eventually immobilized to the support in the condition that the test DNA complex of the single-stranded DNA containing methylated DNA in the target DNA region and the detection oligonucleotide is formed, and
(1) the methylated DNA antibody may be immobilized to the support in the stage prior to binding of the test DNA complex and the methylated DNA antibody, or
(2) the methylated DNA antibody may be immobilized to the support in the stage posterior to binding of the test DNA complex and the methylated DNA antibody.

One exemplary concrete method for immobilizing a methylated DNA antibody to a support involves immobilizing a biotinylated methylated DNA antibody obtained by biotinylating a methylated DNA antibody to a streptavidin-coated support (for example, a PCR tube coated with streptavidin, magnetic beads coated with streptavidin, a chromatostrip partially coated with streptavidin and so on).

Also there is a method of letting a molecule having an active functional group such as an amino group, a thiol group, or an aldehyde group covalently bind to a methylated DNA antibody, and letting the resultant conjugate covalently bind to a support made of glass, a polysaccharide derivative, silica gel or the synthetic resin or thermostable plastic whose surface is activated by a silane coupling agent or the like. The above described covalent bonding may be achieved, for example, by covalently coupling the molecule having an active functional group to a methylated DNA antibody using a spacer formed by serially connecting five triglycerides, a cross linker or the like.

A methylated DNA antibody may be directly immobilized to a support, or an antibody against a methylated DNA antibody (secondary antibody) may be immobilized to a support, and a methylated antibody may be bound to the secondary antibody to achieve immobilization to a support.

The expression "making the immobilized methylated DNA antibody bind with a test DNA complex to obtain a detection complex" which is Fourth step means making the single-stranded methylated DNA contained in the test DNA complex obtained in Third step bind with the immobilized methylated DNA antibody to immobilize it to the support. For example, when "the methylated DNA antibody is immobilized to the support in the stage prior to binding of the test DNA complex and the methylated DNA antibody", concretely, for example, as the methylated DNA antibody that can be immobilized to the support, a "biotin-labeled biotinylated methylated DNA antibody" is used, and when the DNA sample derived from a specimen is a DNA sample derived from genome contained in a specimen derived from a biological specimen, the practice may be executed in the following manner.
(a) An appropriate amount (for example, 4 µg/mL solution, 100 µL/well) of a biotinylated methylated DNA antibody is added to an avidin-coated plate, and then left still at room temperature, for example, for about 2 hours to promote immobilization of the biotinylated methylated DNA antibody and streptavidin. Thereafter, the remaining solution is removed and washed. A washing buffer (for example, 0.05% Tween20-containing phosphate buffer (1 mM KH₂ PO_{4,} 3 mM Na₂HPO 7H₂O, 154 mM NaCl pH7.4)) is added in an amount of, for example, 300 µL/well, and the solution is removed. This operation is repeated several times and the biotinylated methylcytosine antibody that is immobilized to the support is left on the well.
(b) A DNA sample derived from genome contained in a specimen derived from a biological specimen is added with 5 µL of NEBuffer2 (available from NEB Inc.), 0.5 µL of S-adenosyl methionine (3.2 mM, available from NEB Inc.), and 0.5 µL of cytosine methylation enzyme SssI (available from NEB Inc.), and then the mixture is added with sterilized ultra pure water to make the liquid amount 50 µL, and incubated at 37°C for 30 minutes. Then methylated single-stranded DNA obtained by separating double-stranded DNA, a detection oligonucleotide, and an annealing buffer (for example, 33 mM Tris-Acetate pH 7.9, 66 mM KOAc, 10 mM MgOAc₂, 0.5 mM Dithothreitol) are mixed, and heated at 95°C, for example, for several minutes. Then for forming a conjugate of single-stranded DNA containing a target DNA region and a detection oligonucleotide, the mixture is rapidly cooled to the temperature lower than Tm value of the detection oligonucleotide by about 10 to 20°C, and retained at this temperature for example, for several minutes, and then returned to room temperature (the conjugate formed in this stage includes a conjugate of single-stranded DNA not containing DNA methylated in a target region and a detection oligonucleotide, as well as a conjugate of methylated single-stranded DNA containing DNA methylated in a target DNA region and a detection oligonucleotide).
(c) The formed conjugate of methylated single-stranded DNA and a detection oligonucleotide is added to an avidin-coated plate to which a biotinylated methylated DNA antibody is immobilized, and then left still at room temperature for about 3 hours to promote formation of a complex of a biotinylated methylated DNA antibody, methylated single-stranded DNA containing DNA methylated in a target DNA region, among the methylated single-stranded DNAs, and the detection oligonucleotide (formation of complex) (in this stage, if the DNA sample containing a target DNA region is not methylated, the conjugate of single-stranded DNA and a detection oligonucleotide will not form a complex with a methylated DNA antibody). Thereafter, the remaining solution is removed and washed. A washing buffer (for example, 0.05% Tween20-containing phosphate buffer (1 mM KH₂ PO₄, 3 mM Na₂HPO 7H₂O, 154 mM NaCl pH7.4)) is added in an amount of, for example, 300 µL/well, and the solution is removed. This washing operation is repeated several times to leave the complex on the well (separation of complex).

As the annealing buffer used in (b), those suited for binding the detection oligonucleotide and methylated single-stranded DNA containing a target DNA region may be used without limited to the aforementioned annealing buffer. Stability of binding increases when an annealing buffer in which a divalent ion, preferably a magnesium ion is dissolved at a concentration of 1 to 600 mM is used.

The washing operation in (a) and (c) is important for removing an unimmobilized methylated DNA antibody suspended in the solution, unmethylated single-stranded DNA that is not bound to a methylated DNA antibody and suspended in the solution, methylated single-stranded DNA in a region other than the target region that does not form a conjugate with the detection oligonucleotide and the like, from the reaction solution. The washing buffers may be those suited for removing the free methylated DNA antibody, methylated single-stranded DNA suspended in the solution and the like, and a DELFIA buffer (available from Perkin Elmer, Tris-HC1 pH 7.8 with Tween 80), a TE buffer and the like may also be used without limited to the aforementioned washing buffer.

In the above (a) to (c), after binding of the methylated single-stranded DNA obtained by methylating the target DNA region, and the detection oligonucleotide, a biotinylated methylated DNA antibody is caused to bind with the resultant conjugate to form a complex, however, the order is not limited thereto. That is, after causing the methylated single-stranded DNA containing a target DNA region to bind with the biotinylated methylated DNA antibody, the detection oligonucleotide may be bound to the resultant conjugate to form a complex. For example, a biotinylated methylated DNA antibody immobilized to a support coated with streptavidin may be added with a DNA sample derived from genome, 5 µL of NEBuffer2 (available from NEB Inc.), 0.5 µL of S-adenosyl methionine (3.2 mM, available from NEB Inc.), and 0. 5 µL of cytosine methylation enzyme SssI (available from NEB Inc.), and then the resultant mixture is added with sterilized ultra pure water to make the liquid amount 50 µL, and incubated at 37°C for 30 minutes, and then added with methylated single-stranded DNA obtained by separation from double-stranded DNA, to thereby form a conjugate of the biotinylated methylated DNA antibody immobilized to the support and the methylated single-stranded DNA (the conjugate formed in this stage includes a conjugate of methylated single-stranded DNA methylated in a region other than the target DNA region and a methylated antibody, as well as a conjugate of methylated single-stranded DNA containing DNA methylated in a target DNA region and the methylated antibody). Thereafter, the detection oligonucleotide may be added thereto to form a complex with the conjugate having methylated single-stranded DNA containing DNA methylated in a target DNA region, among the aforementioned bound bodies, and the complex may be separated (in this stage, the conjugate of the methylated single-stranded DNA containing DNA methylated in a region other than the target DNA region and the methylated antibody will not form a complex).

The operations of the above (a) to (c) may be conducted using a chromatostrip. In this case, the operations are concretely conducted as follows. For example, first, an appropriate amount of a biotinylated methylated DNA antibody is developed by a chromatostrip that is partly coated with streptavidin. By this operation, the biotinylated methylated DNA antibody is immobilized to the part coated with streptavidin. Then the conjugate of the methylated single-stranded DNA and the detection oligonucleotide obtained in (b) (the conjugate formed in this stage includes a conjugate of single-stranded DNA not containing DNA methylated in a target region and a detection oligonucleotide, as well as a conjugate of methylated single-stranded DNA containing DNA methylated in a target DNA region and a detection oligonucleotide) is developed by the chromatostrip. Through these operations, a complex made up of the methylated single-stranded DNA obtained by adding a DNA sample derived from genome DNA, 5 µL of NEBuffer2 (available from NEB Inc.), 0.5 µL of S-adenosyl methionine (3.2 mM, available from NEB Inc.), and 0.5 µL of cytosine methylation enzyme SssI (available from NEB Inc.), and adding the resultant mixture with sterilized ultra pure water to make the liquid amount 50 µL, and incubating at 37°C for 30 minutes, followed by separation from double-stranded DNA, the detection oligonucleotide, and the biotinylated methylated DNA antibody is immobilized to the part coated with streptavidin (formation of complex and immobilization to support)(in this stage, the conjugate of single-stranded DNA not containing DNA methylated in a target DNA region and a detection oligonucleotide fails to form a complex). The order of operations for formation of a complex is not limited to this order of operations. For example, after forming a complex made up of the single-stranded DNA containing DNA methylated in a target DNA region, the detection oligonucleotide, and the biotinylated methylated DNA antibody, this may be developed by a chromatostrip, and the complex may be immobilized to the part coated with streptavidin. In these operations, unnecessary components can be removed and a washing operation can be omitted by developing the solution by the chromatostrip. Between these operations, a washing operation (development of the chromatostrip by a washing buffer (for example, 0.05% Tween20-containing phosphate buffer (1 mM KH₂ PO₄, 3 mM Na₂HPO 7H₂O, 154 mM NaCl pH7.4)) may be executed.

Fifth step is a step of quantifying or detecting DNA having a target DNA region in the single-stranded DNA by quantifying or detecting the detection oligonucleotide contained in the detection complex obtained in Fourth step according to its identification function.

The term "detection" used herein means determining whether the degree of methylation of DNA methylated up to Second step is a certain degree or more according to the identification function of the detection oligonucleotide. That is, it usually means that a significant value is obtained by the identification function as will be described later.

The term "quantification" means quantification of an amount detected by the identification function determined in Fifth step. That is, it means that a value correlated with a total amount of the DNA having a target DNA region obtained in First step, and the methylated DNA methylated by DNA methylation enzyme treatment in Second step is obtained. For example, the quantified value of the amount of the methylated DNA antibody or the present oligonucleotide as the identification function as will be described later is a value correlated with the amount of DNA in the target DNA region in the specimen, and, for example, when the specimen is 1 mL of serum, it means acquiring a value correlated with a total amount of the DNA having a target DNA region obtained in First step and the methylated DNA methylated by DNA methylation enzyme treatment in Second step, among the DNAs of the target region contained in 1 mL of serum.

The term "identification function" in Fifth step is a function capable of detecting or quantifying the detection oligonucleotide. The identification function may be any function possessed by the detection oligonucleotide, and for example, an identification function based on labeling of the detection oligonucleotide, and an identification function imparted to the detection oligonucleotide by a detection molecule binding to the detection oligonucleotide can be recited. Concretely, a fluorescent or a chromogenic property of the detection oligonucleotide labeled with europium, gold colloid, latex bead, radioisotope, fluorescent substance (FITC or the like), horseradish Peroxidase (HRP), alkaline phosphatase, or the like at its 5'-end or 3'-end are recited.

For detection of europium, after adding and mixing Enhancement Solution (available from PerkinElmer), and keeping still at room temperature for about 45 minutes, fluorescence (excitation 340nm/fluorescence 612 nm) may be measured by a fluorescent detector. When the detection oligonucleotide is a methylated oligonucleotide, as a detection molecule, concretely, a methylated DNA antibody, an osmium complex (J. Am. Chem. Soc., 2007;129:5612-5620) and the like can be recited. The term "methylated oligonucleotide" means an oligonucleotide wherein at least one of bases of nucleotides constituting the oligonucleotide is methylated, and more concretely, means oligonucleotides including 5-methylcytosine, 6-methyladenine and the like. Further, when the detection oligonucleotide is labeled with FITC, a FITC antibody can be recited as a detection molecule.

When the detection molecule is a methylated DNA antibody, the function capable of quantifying or detecting by being bound to the antibody can impart an identification function to the antibody. However, a methylated DNA antibody having substrate cross reactivity with an immobilized methylated DNA antibody cannot be used. For example, when an immobilized methylated DNA antibody uses a methylcytosine antibody, a methylated DNA antibody capable of binding with methylcytosine cannot be used. Concretely, labels such as europium label, gold colloid label, latex bead label, radioisotope label, fluorescent substance (FITC or the like) label, horseradish Peroxidase (HRP) label, alkaline phosphatase label, biotin label and the like are fluorescent, chromogenic and the like functions. As a method of imparting an identification function to the antibody as a detection molecule, the identification function may be directly bound to an antibody which is a detection molecule, or a secondary antibody or a tertiary antibody having an identification function may be bound to an antibody which is a detection molecule. Concretely, since an antibody labeled with a fluorescent substance, an antibody labeled with horseradish Peroxidase (HRP), an antibody labeled with alkaline phosphatase, an antibody labeled with biotin, and an antibody labeled with europium are commercially available, they may be used as a secondary antibody or a tertiary antibody. Also an antibody to which a substrate detectable by an enzyme cycle method is bound may be used. As a means for quantifying or detecting these functions, for example, measurement by a radiation detector, a spectrophotometer and the like, or visual observation and the like are recited. For example, as a case of detecting or quantifying a detection oligonucleotide according to its identification function, when a secondary antibody to which europium is added as a concretely detectable or quantifiable function, Enhancement Solution (available from PerkinElmer) is added and mixed after the detection complex is bound to the secondary antibody, and left still at room temperature for about 45 minutes. Thereafter, fluorescence (excitation 340 nm/fluorescence 612 nm) may be measured by a fluorescent detector.

When a methylated DNA antibody (methylated DNA antibody having substrate specificity different from that of an immobilized methylated DNA antibody) is caused to bind with methylated DNA on the detection oligonucleotide and detection or quantification is executed according to its function, concretely, when the own characteristic of the antibody is used as the function, the operation may be executed in the following manner. After causing the methylated DNA antibody to bind with the complex, a secondary antibody against the methylated DNA antibody (for example, Eu-N1-labeled mouse IgG antibody: available from PerkinElmer) is added, and left still at room temperature for about 1 hour, to thereby promote binding of the secondary antibody to the complex. Thereafter, Enhancement Solution (available from PerkinElmer) is added and mixed, and left still at room temperature, for example, for about 45 minutes. Then, by measuring fluorescence (excitation 340 nm/fluorescence 612 nm) by a fluorescent detector, detection or quantification is conducted.

When the methylated DNA antibody binding to methylated DNA on the detection oligonucleotide is labeled with FITC, an antibody to which FITC is bound may also be used as a secondary antibody. In this case, fluorescence of FITC may be measured by a known method to achieve detection or quantification, or detection or quantification may be achieved by using an anti-FITC antibody as a secondary antibody. Further, when FITC is directly bound to the detection oligonucleotide, FITC may be used as an identification function, or labeling function may be imparted by a horseradish Peroxidase (HRP)-labeled FITC antibody, an alkaline phosphatase-labeled FITC antibody, a biotin-labeled FITC antibody, an europium-labeled FITC antibody and the like. Concretely, as the detection oligonucleotide, when a FITC-labeled oligonucleotide is used as the detection oligonucleotide, a detection complex immobilized to a support containing the detection oligonucleotide is added with an antibody labeled with horseradish Peroxidase (HRP) (for example, HRP-labeled FITC antibody (available from Jackson ImmunoResearch Laboratories)), and left still at room temperature for about 1 to 2 hours, to promote binding of the FITC antibody to the detection complex. Then after washing and removing the FITC antibody solution, an appropriate substrate (for example, Substrate Reagent Pack #DY999: available from R&D SYSTEMS) is added and mixed. After leaving still at room temperature for about 5 to 60 minutes, a stop solution (2N H2SO4 aqueous solution) is added to stop the reaction of horseradish Peroxidase (HRP), and absorbance at 450 nm may be measured within 30 minutes after stopping of the reaction.

When biotin is not used for immobilization of the methylated DNA antibody, a biotinylated detection oligonucleotide can be used for detection or quantification. When a biotinylated detection oligonucleotide is detected or quantified, for example, HRP-labeled streptavidin is added and mixed with a immobilized detection complex to form and separate a conjugate of the biotinylated detection oligonucleotide and HRP-labeled streptavidin, and then activity of HRP is measured by a known method to thereby detect or quantify the biotinylated methylated DNA antibody.

The identification function may utilize a substrate used in a high sensitivity detection method such as an enzyme cycle method and the like. Concretely, an antibody to which an enzyme used in an enzyme cycle method may be bound as a detection molecule to the detection complex. The identification function imparted to the detection molecule in the present method is not limited to the methods as described above.

It suffices that the "detection molecule" has a property of detecting or quantifying a detection oligonucleotide. The detection molecule may recognize a detection sequence of a detection oligonucleotide, or may be preliminarily bound to a detection oligonucleotide. That is, the detection molecule has a property of specifically binding to a detection oligonucleotide and has an "identification function" which is the function or characteristic that is utilized for quantification or detection or is a molecule capable of being provided with the identification function. Concretely, it suffices that the detection molecule is able to bind with a methylated oligonucleotide and detect the methylated oligonucleotide, and specifically binds with the methylated oligonucleotide to exhibit the identification function when the detection sequence is the methylated oligonucleotide (a methylated DNA antibody having substrate cross reactivity with the immobilized methylated DNA antibody cannot be used. For example, when the immobilized methylated DNA antibody uses a methylcytosine antibody, a methylcytosine antibody cannot be used as a detection molecule). As others, for example, the detection molecule may be a methylated DNA antibody. However, a methylated DNA antibody having substrate cross reactivity with the immobilized methylated DNA antibody cannot be used. For example, when the immobilized methylated DNA antibody uses a methylcytosine antibody, the methylcytosine antibody cannot be used as a detection molecule. When the detection sequence is a detection molecule itself, it is not necessary to add a new detection molecule for detecting the detection oligonucleotide, and by detecting the detection molecule incorporated into the detection oligonucleotide, it becomes possible to detect the detection oligonucleotide.

As a method of quantifying or detecting minor substances contained in a biological sample such as blood or urine, immunological measuring methods are generally used. Among such methods, what is called immuno chromatography using chromatography is currently widely used in various situations including, for example, clinical examinations in hospitals, assays in laboratories and the like because of its simple operation and short time required for assay. In recent years, what is called hybrid chromatography has been utilized in which labeled DNA (gene) is developed on a chromatostrip, and target DNA (gene) is detected by hybridization using a probe capable of capturing the target DNA (gene). Also this method is now coming to be widely used in situations including, for example, clinical examinations in hospitals, assays in laboratories and the like because of its simple operation and short required time for assay. The present method conceptually enables a combined method of the immuno chromatography and the hybrid chromatography. In the present method, since the order of formation of a complex and selection of a complex is not particularly limited, various methods are possible. Concretely, such methods may be executed in the following manner.

Method 1: A sample directly after end of Second step is added with a detection oligonucleotide having an identification function to form a conjugate of methylated single-stranded DNA containing a target DNA region and the detection oligonucleotide having an identification function (the conjugate formed in this stage includes a conjugate of single-stranded DNA not containing DNA methylated in a target DNA region and a detection oligonucleotide, as well as a conjugate of single-stranded DNA containing DNA methylated in a target DNA region and a detection oligonucleotide), and then added with a biotinylated methylated antibody, to form a complex in which a single-stranded DNA containing DNA methylated in a target DNA region, the detection oligonucleotide having an identification function, and a biotinylated methylated DNA antibody are bound. As the obtained sample is dropped (introduced) into an introduction part of a chromatostrip, the complex migrates in a development part by a capillary phenomenon, and is trapped in the part preliminarily coated with streptavidin. Thereafter, by detecting or quantifying the detection oligonucleotide contained in the obtained complex according to its identification function, DNA having a target DNA region can be detected or quantified.

Method 2: A sample directly after end of Second step is added with a biotinylated methylated DNA antibody, to form a conjugate of methylated single-stranded DNA having methylated cytosine (there exist single-stranded DNA containing a target DNA region and single-stranded DNA other than the target) and a biotinylated methylated DNA antibody (the conjugate formed in this stage includes a conjugate of methylated single-stranded DNA other than the target DNA region and a methylated antibody, as well as a conjugate of single-stranded DNA containing DNA methylated in a target DNA region and a methylated antibody). As the obtained sample is dropped (introduced) into an introduction part of a chromatostrip, the conjugate migrates in a development part by a capillary phenomenon, and is trapped in the part preliminarily coated with streptavidin (also in this stage, the conjugate includes the conjugate of methylated single-stranded DNA other than the target DNA region and a methylated antibody, as well as the conjugate of single-stranded DNA containing DNA methylated in a target DNA region and a methylated antibody). Thereafter, as the detection oligonucleotide having an identification function is dropped (introduced) into the introduction part, it migrates in the development part, and binds only to methylated single-stranded DNA containing a target DNA region, among the aforementioned bound bodies, to form a complex in which single-stranded DNA containing DNA methylated in a target DNA region, a detection oligonucleotide having an identification function, and a biotinylated methylated DNA antibody are bound. By detecting or quantifying the detection oligonucleotide contained in the obtained complex according to its identification function, it is possible to detect or quantify DNA having a target DNA region.

Method 3: As a biotinylated methylated DNA antibody is dropped (introduced) into an introduction part of a chromatostrip, the methylated DNA antibody migrates in a development part by a capillary phenomenon, and is trapped in the part preliminarily coated with streptavidin. Then as a sample directly after end of Second step is dropped (introduced) into the introduction part, it migrates in the development part, and single-stranded DNA having methylated cytosine is trapped by the already trapped methylated DNA antibody as the conjugate (the conjugate formed in this stage includes a conjugate of methylated single-stranded DNA other than the target DNA region and a methylated antibody, as well as a conjugate of single-stranded DNA containing DNA methylated in a target DNA region and a methylated antibody). Thereafter, as the detection oligonucleotide having an identification function is dropped (introduced) into the introduction part, it migrates in the development part, and binds only to methylated single-stranded DNA containing a target DNA region, among the aforementioned bound bodies, to form a detection complex in which single-stranded DNA containing DNA methylated in a target DNA region, a detection oligonucleotide having an identification function, and a biotinylated methylated DNA antibody are bound. By detecting or quantifying the detection oligonucleotide contained in the obtained complex according to its identification function, it is possible to detect or quantify DNA having a target DNA region.

Method 4: As a biotinylated methylated DNA antibody is dropped (introduced) into an introduction part of a chromatostrip, the methylated DNA antibody migrates in a development part by a capillary phenomenon, and is trapped in the part preliminarily coated with streptavidin. A sample directly after end of Second step is added with a detection oligonucleotide having an identification function, to form a test DNA complex which is a conjugate of methylated single-stranded DNA containing a target DNA region and a detection oligonucleotide having an identification function. Then as the obtained conjugate is dropped (introduced) into the introduction part, it migrates in the development part, and only single-stranded DNA containing DNA methylated in a target DNA region, among the aforementioned bound bodies, binds to the methylated DNA antibody that is already trapped, to form a detection complex in which single-stranded DNA containing DNA methylated in a target DNA region, a detection oligonucleotide having an identification function, and a biotinylated methylated DNA antibody are bound. By detecting or quantifying the detection oligonucleotide contained in the obtained complex according to its identification function, it is possible to detect or quantify DNA having a target DNA region.

Also a plurality of detection sites may be provided in the target DNA region (using detection oligonucleotides respectively capable of complementarily binding to different target DNA regions), and each target DNA region may be sequentially detected or quantified, and also by using a detection oligonucleotide capable of complementarily binding to a plurality of target DNA regions such that a repetitive sequence in genome or an overlapping gene or a plurality of different genes are concurrently detected for enabling formation of complexes with a plurality of target DNA regions, the detection sensitivity can be dramatically improved. Also by designing a number of detection oligonucleotides in a single target region, and using them on the support side or on the detection side, the detection sensitivity can be dramatically improved.

As a method of executing the process of forming a complex of the detection oligonucleotide, the biotinylated methylated antibody, and the single-stranded DNA methylated in a target DNA region and binding it to the support, a method of using an immunological antibody method may be applied without limited to Methods 1 to 4 as described above. For example, the ELISA method allows execution of the process of forming a complex and binding it to a support in the order described in Methods 1 to 4 because it uses the principle similar to that of the chromatostrip method.

The DNA methylation enzyme, the detection oligonucleotide, or the methylated DNA antibody that can be used in the present method are useful as reagents for a detection kit. The present method also provides a detection kit containing such a DNA methylation enzyme, a specific oligonucleotide, or a methylated DNA antibody as a reagent, and a detection chip comprising such a present oligonucleotide, or a methylated DNA antibody immobilized on a support, and the scope of the present method includes use in the form of a detection kit or a detection chip as described above using the substantial principle of the present invention.

Generally, for examining presence or absence of a pathogenic microorganism contained in a biopsy sample or food, presence or absence of such a pathogenic microorganism is examined, or such a pathogenic microorganism is identified by a test based on immunization method for each microbial antigen. However, preparation of an antibody used for such a immunization method is not easy, and for detecting a plurality of pathogenic microorganisms, it is necessary to prepare antibodies against respective antigens of the pathogenic microorganisms. By using the present method, it is possible to realize a simple test-four pathogenic microorganisms without conducting such complicated antibody preparation. Further, according to the present method, since nucleotide sequences of different pathogenic microorganisms can be tested at the same time, it is possible to detect several kinds of pathogenic microorganisms contained in one specimen at the same time. Concretely, food poisoning bacteria such as Listeria monocytogenes, Salmonella enterica, Campylobacter jejuni subsp. Jejuni, Staphylococcus aureus, Vibrio parahaemolyticus, Bacillusu cereus, Clostridium botulinum, Yersinia enterocolitica, Yersinia pseudotuberuculosis and Clostridium perfringens are known, however, a technique of detecting several kinds of these food poisoning bacteria at the same time is not known. However, by using the present method, it is possible to detect nucleotide sequences of several kinds of food poisoning bacteria at the same time. Further, when a nucleotide sequence found plurally in genome such as CRISPR (Clustered regularly interspaced short palindromic repeats) region is selected as a nucleotide sequence to be detected by a specific oligonucleotide, detection at higher sensitivity is realized compared to the case of detecting one gene in one genome. Such a technique is useful also for diagnosis of infection and rapid detection of food poisoning bacteria. Further, the present method may be used for identification of an industrially useful bacterium, or for a simple test of a microbial community in soil, river or lake sediments and the like by detecting genomes of microorganisms in such environments. Of the microorganisms in such environments, inhabitation of, for example, Methanococcus jannaschii, Methanobacterium thermoautotrophicum deltaH, Aquifex aeolicus, Pyrococcus horikoshii OT3, Archaeoglobus fulgidus, Thermotoga maritima MSB8, Aeropyrum pernix K1, and Haloferax mediterranei can be verified. It is also possible to detect and identify industrially available bacteria such as Geobacter sulfurreducens and microorganisms used for fermentation such as Streptococcus thermophilus.

When the target DNA region in the present method is a nucleotide sequence derived from a microorganism, it means genomic DNA or a DNA fragment extracted from a specimen, or a nucleotide sequence of DNA obtained from RNA extracted from a specimen by a reverse transcriptase. Therefore, as a nucleotide sequence capable of complementarily binding with a detection oligonucleotide, a region specific to the microorganism may be selected. For example, when the target DNA region in the present invention is a microbial nucleotide sequence, for selectively extracting the target region from the specimen, a nucleotide sequence that is peculiar to the microorganism in the vicinity of the target region may be selected as a nucleotide sequence that specifically binds with the specific oligonucleotide, from microbial genomic DNA, or nucleotide sequences of DNA that are prepared from RNA extracted from the specimen by a reverse transcriptase.

For example, applicable as a region where the target region for detecting genome in a microorganism and the specific oligonucleotide complementarily bind is, concretely, a nucleotide sequence not encoding a gene such as a region corresponding to the nucleotide number 271743-272083 of yeast chromosome VII (SEQ ID NO: 29) shown, for example, in Genbank Accession No. NC_001139, or the nucleotide number 384569-384685 of yeast chromosome VII (SEQ ID NO: 34) shown, for example, in Genbank Accession No. NC_001139. It is also useful to detect a nucleotide sequence conserved among pathogenic microorganisms in a characteristic gene that is common in various pathogenic microorganisms because a method of detecting a plurality of pathogenic microorganisms at the same time can be provided. Concretely, since mce-family gene (Micobacterium tuberculosis), tRNA-Tyrnucleotide sequence on 13th chromosome (Cryptococcus neoformans), and chitin synthase activator (Chs3) have a nucleotide sequence peculiar to Aspergillus fumigatus and genus Neosartorya, they can be used for assay of infection by a microorganism, by assaying whether DNA derived from these microorganisms is contained in DNA extracted from a biopsy sample of human expectoration or lung. Further, since actA (Listeria monocytogenes), pyrG (NC 002163, Campylobacter jejuni subsp. jejuni) and the like are common genes peculiar to food poisoning bacteria, these genes may be used for a microbial assay in food poisoning. ThrA has a sequence that is conserved among Salmonella enterica, Yersinia enterocolitica, and Escherichia coli, so that a plurality of microorganisms can be detected by one gene.

Of nucleotide sequences published on a database, a nucleotide sequence peculiar to a microorganism may be retrieved, and a nucleotide sequence peculiar to a microorganism may be searched. For example, a nucleotide sequence on a published database such as PubMed may be obtained through regular procedure, and the obtained nucleotide sequence can be examined whether it is a peculiar nucleotide sequence by Blast search through regular procedure. The peculiar nucleotide sequence means that a nucleotide sequence in a detection object does not include a nucleotide sequence having homology with a nucleotide sequence derived from an organism other than the genomic nucleotide sequence of the microorganism to be detected.

In particular, when the specimen is a human biopsy sample, it is important to design a specific oligonucleotide that would not complementarily bind with human genes. Similarly, when the specimen is food, it is important to design a specific oligonucleotide that would not complementarily bind with a nucleotide sequence derived from an organism other than the detection object contained in the food.

For detecting free DNA in blood, any region correlated with an amount of free DNA may be used, and when it is intended to quantify or detect free DNA, what is called repetitive sequence where the same sequence in genome appears repetitively, several or more times, is preferred, and a simple repetitive sequence (called tandem repetitive sequence, or tandem repeat), and an interspersed repeat sequence are more preferred.

The simple repetitive sequence is **characterized in that** the same sequences neighbor in the same orientation, and a series of nucleotide sequences such as satellite DNA, minisatellite, microsatellite, centromere, telomere, kinetochore, and ribosome group genes are known.

The interspersed repetitive sequence is **characterized in that** the same sequences are interspersed without neighboring each other, and is believed to be DNA derived from retrotransposon. Interspersed repetitive sequences are classified into SINE (Short Interspersed Repetitive Element: short chain interspersed repetitive sequence) and LINE (Long Interspersed Elements: long chain interspersed repetitive sequence) depending on the length of the nucleotide sequence, and as a human nucleotide sequence, Alu sequence and LINE-1 sequence are respectively known as representative repetitive sequences. Also an inactive processed pseudogene that is reverse transcribed from RNA or protein, and a gene sequence amplified by gene duplication are also known.

The term dupulicated gene indicates the case where a plurality of genes having high homology exist on one genome, and is, in many cases, a nucleotide sequence that exists in tandem near one gene. It is often the case where a pseudogene is one of duplicated genes.

As concrete examples of the repetitive sequence, such sequences as (A)n, (T)n, (GA)n, (CA)n, (TAA)n, (GGA)n, (CAGC) n, (CATA)n, (GAAA)n, (TATG)n, (TTTG)n, (TTTA)n, (TTTC)n, (TAAA)n, (TTCA)n, (TATAA)n, (TCTCC)n, (TTTCC)n, (TTTAA)n, (TTTTC)n, (TTTTA)n, (TTTTG)n, (CAAAA)n, (CACCC)n, (TATATG)n, (CATATA)n, (TCTCTG)n, (AGGGGG)n, (CCCCCA)n, and (TGGGGG)n (n means a number of repetition) are known as repetition comprising a relatively short nucleotide sequence, and as a sequence derived from a transcription factor, MER1-Charlie, and Zaphod of hAT group, and MER2-Tigger, Tc-1, and Mariner of Tc-1 group can be recited. As others, concretely, Tigger1, Tigger2a, Tigger5, Charlie4a, Charlie7 and the like are known. These sequences are generally short and simple nucleotide sequences, and are difficult to set the specific adhesion sequence and a detection adhesion sequence as will be described later, however, these sequences can be used in the present method as far as they have a sequence that can be set into setting objects of the specific adhesion sequence and a detection adhesion sequence as will be described later. Therefore, it is not necessarily excluded as an object of the present method. Further, satellite DNA, minisatellite, microsatellite and the like are repetitive sequences classified into simple repetitive sequences.

Further, as a sequence having multi-copies in gene, ALR6 as a sequence existing in centromere, U2 and U6 as snRNA, as well as the genes such as tRNA and rRNA that are generally known to have multi-copies in genome, and the genes that have plural copies in genome as a result of gene duplication are recited.

It is also known that a retrovirus, a retrotransposon having LTR (Long terminal repeat) in its terminal, an endogenous sequence such as MaLRs (Mammalian apparent LTR-Retrotransposons) considered to be derived from viruses, and LTR derived from a retrovirus exist in multicopy in one genome.

For example, as the LTR derived from a retrovirus, concretely, subfamilies such as LTR1, LTR1B, LTR5, LTR7, LTR8, LTR16A1, LTR16A1, LTR16C, LTR26, LTR26E, MER48, and MLT2CB are known. The LTRs derived from a retrotransposon are classified into classes of ERV, ERVK and ERVL, and concrete examples include subfamilies such as LTR8A, LTR28, MER21B, MER83, MER31B, MER49, MER66B, HERVH, ERVL, LTR16A1, LTR33A, LTR50, LTR52, MLT2A1, MLT2E, MER11C, and MER11C. Further, MaLRs indicate DNA factors including LTRs in both ends likewise a typical retrotransposon, wherein an internal sequence sandwiched between LTRs is not derived from a retrovirus. For example, subfamilies such as MLT1A1, MLT1A2, MLT1B, MLT1C, MLT1D, MLT1F, MLT1G, MLT1H, MLT1J, MLT1K, MLT1I, MLT2CB, MSTA, MSTA-int, MSTB, THE1A, THE1B, THE1B-internal, and THE1 can be recited.

The interspersed repetitive sequences are **characterized in that** the same sequences are interspersed without neighboring each other, and are considered to be derived from a retrotransposon. Further, the interspersed repetitive sequences are classified into SINE (Short Interspersed Repetitive Element: short chain interspersed repetitive sequences) and LINE (Long Interspersed Elements: long-chain interspersed repetitive sequences) according to the length. Most of SINEs are sequences belonging to the Alu family. A common feature is that it has a sequence of 3'-side or a sequence of 5'-side of 7SL RNA, and that it has an AT-Rich region sandwiched between a Left-monomer and a Right-monomer. As subfamilies of the Alu family, Alu, AluJb, AluJo, AluSc, AluSg, AluSp, AluSq, AluSx, AluY, and FAM (Fossil Alu Monomer), FLAM (Free Left Alu Monomer) having a sequence of FAM, and FRAM (Free Right Alu Monomer) can be recited. As SINEs other than the Alu family, MIR, and Ther/MIR3 are known, and MIR and MIR3 are known as respective subfamilies. As subfamilies of the Alu family including other biological species, B1, B2, B4, PB1, PB1D and so on are known. As LINEs, subfamilies of LINE1 to Line23 are reported, and it is known that subfamilies such as LINE-1, LINE2, and LINE3 broadly exist in a genome. As for LINE-1, for example, L1M1, L1M2, L1M3, L1M3d, L1M4, L1M4c, L1MA2, L1MA7, L1MA8, L1MA9, L1MB1, L1MB1, L1MB3, L1MB4, L1MB5, L1MB6, L1MB7, L1MCa, L1MCb, L1MC2, L1MC3, L1MC4, L1MC4a, L1MC5, L1MDa, L1ME, L1MEc, L1MEd, L1MEg, L1ME1, L1ME2, L1ME3, L1ME3A, L1ME3B, L1ME4a, L1PB3, L1P4, L1PA2, L1PA3, L1PA4, L1PA5, L1PA6, L1PA7, L1PA10, L1PA12, L1PA13, L1PA14, L1PA16, L1PB1, L1PB3, L1PB4, L1PREC2, and HAL1 are known, and as LINE-2, subfamilies such as L2 and L2c are known. For example, if the later-described specific adhesion sequence and the detection adhesion sequence can be set, for a sequence common to the Alu family or subfamilies of Alu, or the LINE-1 family or subfamilies of LINE-1, a plurality of detection objects can be set in one genome, so that sensitivity of genome detection can be improved.

As a target DNA region, concretely, for example, a partial sequence of LINE-1 (the nucleotide sequence of SEQ ID NO: 37), a partial sequence of Alu (the nucleotide sequence of SEQ ID NO: 39) or nucleotide sequences having homology to these sequences can be recited.

For example, when a repetitive sequence in a certain region needs to be examined, databases such as Repbase (http://www.girinst.org/repbase/) and RepeatMasker
(http://www.repeatmasker.org/) may be used because it is difficult to retrieve a general sequence retrieving database such as PuMed. If a specific adhesion sequence of the present method can be set, the detection sensitivity can be improved. Measuring these repetitive sequences can be treated, for example, as a surrogate marker of a free DNA amount in blood, and can be utilized for identification of an organism species when an organism species-specific repetitive sequence is noted.

In the present method, by measuring a repetitive sequence, a nucleotide sequence existing plurally in one genome can be measured concurrently. For example, a nucleotide sequence having a sequence homology of 80% or higher with the nucleotide sequence of SEQ ID NO: 37 has about 280 copies in a human genome, and a nucleotide sequence having a sequence identity of 80% or higher with the nucleotide sequence of SEQ ID NO: 39 has about 820 copies in a human genome. Therefore, if a specific adhesion sequence can be set in each nucleotide sequence, the detection sensitivity of one genome can be improved to 280 to 820 folds theoretically, compared to the case where a specific adhesion sequence is set for a sequence having just one kind in genome.

A duplicated gene means a gene or a gene fragment that is generated by doubling of a specific gene or gene fragment in genome due to gene duplication. Gene duplication is a phenomenon that a certain region of DNA including a gene is overlapped. As a cause of gene duplication, abnormality of gene recombination, translocation of retrotransposon, duplication of the entire chromosome and the like are recited. For example, it means that one gene is copied and inserted into genomic DNA, and the copy is inserted to a different chromosome site in some cases, and inserted near the original gene in the other cases. The site where copied genes are aligned as a result of insertion near the original gene is called a tandem repeat, and a group of genes generated by gene duplication is called a gene family.

A pseudogene means a gene having a characteristic nucleotide sequence that is assumable to have encoded a gene product (particularly protein) in a sequence of DNA, but currently loosing the function. It is assumed that it is generated as a result of mutation of the original functioning sequence. For example, there is the case where a stop codon arises by mutation and a peptide chain of a protein is shortened, so that the function as a protein is no longer effective, and there is the case where a function of a regulatory sequence required for normal transcription is impaired due to mutation such as single nucleotide substitution. In many pseudogenes, the original normal genes are remained separately, however, those becoming pseudogenes by themselves are also known.

Pseudogenes are classified into three types according to the characteristic of the gene sequence. There are known the case where DNA prepared from mRNA by a reverse transcriptase of retrotransposon is inserted into genome (processed pseudogene), the case where an original gene sequence is duplicated in genome, and a part of the copies looses the function due to mutation or the like to become a pseudogene (duplicated pseudogene or non-processed pseudogene), and the case where gene in genome (in the condition of single gene with no duplicated gene) looses the function to become a pseudogene.

Currently, among the genes known as pseudogenes, transcribed examples, examples having a gene function (whether it is called a pseudogene is not determined) and the like also have been known, so that the term "pseudogene" in the present method means the "processed pseudogene" or "duplicated pseudogene (non-processed pseudogene)" rather than presence or absence of gene function or whether it is transcribed or not.

When DNA having a target DNA region is a repetitive sequence in genome, the repetitive sequence is a group of nucleotide sequences having homology, so that there is the possibility that in complementary base pairing between DNA having a target DNA region and an adhesion sequence for detection, every nucleotide sequence fails to base-pair with DNA having a target DNA region. Concretely, as the LINE sequence, there are about 280 copies of nucleotide sequences having a homology of 80% or more with respect to SEQ ID NO: 37 in genome, and as the SINE (Alu) sequence, there are found about 820 copies of nucleotide sequences having a homology of 80% or more with respect to SEQ ID NO: 39 in genome. However, these nucleotide sequences having homology found in genome include those having one or several different bases with respect to SEQ ID NO: 37 and SEQ ID NO: 39.

In First step of the present method, it is preferred to extract DNA from a specimen by a system containing a sodium salt at high concentration. Concretely, as a concentration of sodium salt in a solution (for example, buffer) used in a DNA extraction operation for obtaining DNA from a specimen in First step of the present method, at least 50 mM or more, and preferably 100 mM or more can be recited. More concretely, 50 mM or more and 1000 mM or less, preferably 100 mM or more and 1000 mM or less, more preferably 100 mM or more and 200 mM or less can be recited. Any salts including NaCl, NaCO₃, Na₂SO₄ and the like are applied as far as it is a salt containing a sodium ion, and preferably means NaCl.

The present invention is a method of selecting a specimen derived from a cancer patient, and includes the steps of evaluating a specimen derived from a test subject as a specimen derived from a cancer patient when there is a significant difference between a DNA quantification result or detection result quantified or detected using a specimen derived from a test subject by the method according to any one of Inventions 1 to 13, and a DNA quantification result or detection result quantified or detected using a specimen derived from a healthy subject by the method, and identifying the specimen derived from a cancer patient based on the evaluation result. As a preferred aspect of the present invention, the invention in which the specimen is a serum derived from a mammal, and the invention in which the DNA comprising a target DNA region is free DNA comprising a target DNA region in serum derived from a mammal can be recited. Use of these inventions will make it possible to identify a cancer patient in a simple and convenient manner by a blood test.

Here, the "cancer patient" is a test subject developing a cancer, and as the cancer, solid cancers developing in organs of human and mammals, and non-solid cancers developing in blood of human and mammals such as lung cancer (non-small-cell lung cancer, small-cell lung cancer), esophageal cancer, gastric cancer, duodenal cancer, colon cancer, rectal cancer, hepatic cancer (hepatocarcinoma, cholangiocellular carcinoma), gallbladder cancer, bile duct cancer, pancreatic cancer, colon cancer, anal cancer, breast cancer, cervical cancer, uterine cancer, ovarian cancer, vulvar cancer, vaginal cancer, prostate cancer, kidney cancer, ureter cancer, bladder cancer, prostate cancer, penile cancer, testicular (testis) cancer, maxillary cancer, tongue cancer, (naso-, oro-, hypo-) pharyngeal cancer, acute myeloid leukemia, chronic myelogenous leukemia, acute lymphoblastic leukemia, chronic lymphoblastic leukemia, malignant lymphoma, myelodysplastic syndrome, thyroid cancer, brain tumor, osteosarcoma and skin cancer (basal cell cancer, squamous cell cancer) are included.

### EXAMPLES

In the following, the present invention will be described in detail by way of examples, however, the present invention is not limited to these examples.

### Example 1

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂ PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂ PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

For genomic DNA derived from human blood purchased from Clontech, a DNA fragment (X, SEQ ID NO: 19, the region corresponding to the nucleotide numbers 25687390-25687775 shown in Genbank Accession No. NT_029419) used as a test sample was amplified by conducting PCR using the following oligonucleotide primers (PF1 and PR1) designed for PCR of SEQ ID NO: 17 and SEQ ID NO: 18 and the following reaction condition.

### <Oligonucleotide primer designed for PCR>

PF1:5'- CTCAGCACCCAGGCGGCC -3' (SEQ ID NO: 17)
PR1:5'- CTGGCCAAACTGGAGATCGC -3' (SEQ ID NO: 18)

### <DNA fragment>

X:5'-
CTCAGCACCCAGGCGGCCGCGATCATGAGGCGCGAGCGGCGCGGGCTGTTGCAGAGTCTT
GAGCGGGTGGCACACCGCGATGTAGCGGTCGGCTGTCATGACTACCAGCATGTAGGCCGACG
CAAACATGCCGAACACCTGCAGGTGCTTCACCACGCGGCACAGCCAGTCGGGGCCGCGGAAG
CGGTAGGTGATGTCCCAGCACATTTGCGGCAGCACCTGGAAGAATGCCACGGCCAGGTCGGC
CAGGCTGAGGTGTCGGATGAAGAGGTGCATGCGGGACGTCTTGCGCGGCGTCCGGTGCAGAG
CCAGCAGTACGCTGCTGTTGCCCAGCACGGCCACCGCGAAAGTCACCGCCAGCACGGCGATC
TCCAGTTTGGCCAG -3'(SEQ ID NO: 19)

As a reaction liquid of PCR, 10 ng of genomic DNA as a template, each 3 µL of 5 µM of the above primer solutions, 5 µL of each 2 mM dNTP, and 5 µL of 10xbuffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 30 seconds at 95°C, 30 seconds at 61°C and 45 seconds at 72°C.

After conducting PCR, amplification was checked by 2% agarose gel electrophoresis, and the DNA fragment X was purified by Wizard SV Gel/PCR Kit (PROMEGA Corporation).

For the DNA fragment X, the following solutions were prepared respectively in duplicate.
Solution A: DNA fragment X 10ng/10 µL TE buffer solution
Solution B: DNA fragment X 1ng/10 µL TE buffer solution
Solution C: DNA fragment X 0.1ng/10 µL TE buffer solution
Solution D: TE buffer solution (negative control solution)

Ten (10) µL of each obtained solution, 0. 5 µL of SssI methylase (available from NEB Inc.), 5 µL of 10xNEBuffer2 (available from NEB Inc.), and 0.5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F1 having the nucleotide sequence of SEQ ID NO: 21 capable of binding by complementation with oligonucleotide X' comprising a target DNA region of SEQ ID NO: 20, and a 0.02 µM solution in Tris-HCl buffer (10 mM) was prepared.

### <Oligonucleotide comprising target DNA region>

X′:5′-

CTCAGCACCCAGGCGGCCGCGATCATGAGGCGCGAGCGGCGCGGGCTGTTGCAGAGTCTT

GAGCGGGTGGCACACCGCGATGTAGCGGTCGGCTGTCATGACTACCAGCATGTAGGCCGACG

CAAACATGCCGAACACCTGCAGGTGCTTCACCACGCGGCACAGCCAGTCGGGGCCGCGGAAG

CGGTAGGTGATGTCCCAGCACATTTGCGGCAGCACCTGGAAGAATGCCACGGCCAGGTCGGC

CAGGCTGAGGTGTCGGATGAAGAGGTGCATGCGGGACGTCTTGCGCGGCGTCCGGTGCAGAG

CCAGCAGTACGCTGCTGTTGCCCAGCACGGCCACCGCGAAAGTCACCGCCAGCACGGCGATC

TCCAGTTTGGCCAG -3′ (SEQ ID NO: 20)

### <5'-end FITC-labeled oligonucleotide>

F1:5'- CTGGCCAAACTGGAGAT -3' (SEQ ID NO: 21)

Each obtained reaction liquid was subjected to the following treatments.

In a PCR tube, 40 µL of the reaction liquid prepared in the above, 10 µL of the 5'-end FITC-labeled oligonucleotide solution, 10 µL of a buffer (330 mM Tris-Acetate pH 7.9, 660 mM KoAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4) ] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂ PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 15 minutes, 50 µL of a stop solution (2N H₂ SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 1. In Solution A, Solution B, and Solution C, increase in absorbance was observed compared to Solution D. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 2

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂ PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

For genomic DNA derived from human blood purchased from Clontech, a DNA fragment (X, SEQ ID NO: 19, the region corresponding to the nucleotide numbers 25687390-25687775 shown in Genbank Accession No. NT_029419) used as a test sample was amplified by conducting PCR using the following oligonucleotide primers (PF1 and PR1) designed for PCR of SEQ ID NO: 17 and SEQ ID NO: 18 and the following reaction condition.

### <Oligonucleotide primer designed for PCR>

PF1:5'- CTCAGCACCCAGGCGGCC -3' (SEQ ID NO: 17)
PR1:5'- CTGGCCAAACTGGAGATCGC -3' (SEQ ID NO: 18)

### <DNA fragment>

X:5'-
CTCAGCACCCAGGCGGCCGCGATCATGAGGCGAGCGGCGCGGGCTGTTGCAGAGTCTT
GAGCGGGTGGCACACCGCGATGTAGCGGTCGGCTGTCATGACTACCAGCATGTAGGCCGACG
CAAACATGCCGAACACCTGCAGGTGCTTCACCACGCGGCACAGCCAGTCGGGGCCGCGGAAG
CGGTAGGTGATGTCCCAGCACATTTGCGGCAGCACCTGGAAGAATGCCACGGCCAGGTCGGC
CAGGCTGAGGTGTCGGATGAAGAGGTGCATGCGGGACGTCTTGCGCGGCGTCCGGTGCAGAG
CCAGCAGTACGCTGCTGTTGCCCAGCACGGCCACCGCGAAAGTCACCGCCAGCACGGCGATC
TCCAGTTTGGCCAG -3'(SEQ ID NO: 19)

As a reaction liquid of PCR, 10 ng of genomic DNA as a template, each 3 µL of 5 µM of the above primer solutions, 5 µL of each 2 mM dNTP, and 5 µL of 10xbuf fer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 30 seconds at 95°C, 30 seconds at 61°C and 45 seconds at 72°C.

After conducting PCR, amplification was checked by 2% agarose gel electrophoresis, and the DNA fragment X was purified by Wizard SV Gel/PCR Kit (PROMEGA Corporation).

For the DNA fragment X, the following solutions were prepared respectively in duplicate.
Solution A: DNA fragment X 10ng/10 µL TE buffer solution
Solution B: DNA fragment X 1ng/10 µL TE buffer solution
Solution C: DNA fragment X 0.1ng/10 µL TE buffer solution
Solution D: TE buffer solution (negative control solution)

For genomic DNA derived from human blood purchased from Clontech, a DNA fragment (Y, SEQ ID NO: 24, the region corresponding to the nucleotide numbers 76606-76726 shown in Genbank Accession No. AC009800) used as a test sample was amplified by conducting PCR using the following oligonucleotide primers (PF2 and PR2) designed for PCR of SEQ ID NO: 22 and SEQ ID NO: 23 and the following reaction condition.

### <Oligonucleotide primers designed for PCR>

PF2:5'- TGAGCTCCGTAGGGCGTCC -3' (SEQ ID NO: 22)
PR2:5'- GCGCCGGGTCCGGGCCC -3' (SEQ ID NO: 23)

### <DNA fragment>

Y:5′-
GCGCCGGGTCCGGGCCCGATGCGTTGGCGGGCCAGGGCTCCGAGAACGAGGCGTTGTCCATC
TCAACGAGGGCAGAGGAGCCGGCGACCTGGCGTCCCCCAAGGACGCCCTACGGAGCTCA -3′
(SEQ ID NO: 24)

As a reaction liquid of PCR, 10 ng of genomic DNA as a template, each 3 µL of 5 µM of the above primer solutions, 5 µL of each 2 mM dNTP, and 5 µL of 10xbuffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 50 cycles each consisting of 30 seconds at 95°C, 30 seconds at 60°C and 45 seconds at 72°C.

After conducting PCR, amplification was checked by 2% agarose gel electrophoresis, and the DNA fragment Y was purified by Wizard SV Gel/PCR Kit (PROMEGA Corporation).

For the DNA fragment Y, the following solutions were prepared respectively.
Solution A: DNA fragment Y 10ng/10 µL TE buffer solution
Solution: DNA fragment Y 1ng/10 µL TE buffer solution
Solution C: DNA fragment Y 0.1ng/10 µL TE buffer solution
Solution D: TE buffer solution (negative control solution)

Solution A of DNA fragment X and Solution A of DNA fragment Y, Solution B of DNA fragment X and Solution B of DNA fragment Y, Solution C of DNA fragment X and Solution C of DNA fragment Y, and Solution D of DNA fragment X and Solution D of DNA fragment Y prepared in the above were respectively mixed, to prepare the following DNA fragment-mixed Solutions MA to MD respectively in duplicate.
Solution MA: 10 ng/20 µL TE buffer solution
Solution MB: 1 ng/20 µL TE buffer solution
Solution MC: 0.1 ng/20 µL TE buffer solution
Solution MD: TE buffer solution (negative control solution)

Twenty (20) µL of each obtained solution, 0.5 µL of SssI methylase (available from NEB Inc.), 5 µL of 10xNEBuffer2 (available from NEB Inc.), and 0. 5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F1 having the nucleotide sequence of SEQ ID NO: 21 capable of binding by complementation with oligonucleotide X' comprising a target DNA region of SEQ ID NO: 20, and a 0.02 µM solution in Tris-HCl buffer (10 mM) was prepared.

### <Oligonucleotide comprising target DNA region>

X′:5′-
CTCAGCACCCAGGCGGCCGCGATCATGAGGCGAGCGGCGCGGGCTGTTGCAGAGTCTT
GAGCGGGTGGCACACCGCGATGTAGCGGTCGGCTGTCATGACTACCAGCATGTAGGCCGACG
CAAACATGCCGAACACCTGCAGGTGCTTCACCACGCGGCACAGCCAGTCGGGGCCGCGGAAG
CGGTAGGTGATGTCCCAGCACATTTGCGGCAGCACCTGGAAGAATGCCACGGCCAGGTCGGC
CAGGCTGAGGTGTCGGATGAAGAGGTGCATGCGGACGTCTTGCGCGGCGTCCGGTGCAGAG
CCAGCAGTACGCTGCTGTTGCCCAGCACGGCCACCGCGAAAGTCACCGCCAGCACGGCGATC
TCCAGTTTGGCCAG -3′ (SEQ ID NO: 20)

### <5'-end FITC-labeled oligonucleotide>

F1:5'- CTGGCCAAACTGGAGAT -3' (SEQ ID NO: 21)

Each obtained reaction liquid was subjected to the following treatments.

In a PCR tube, 40 µL of the reaction liquid prepared in the above, 10 µL of the 5'-end FITC-labeled oligonucleotide solution, 10 µL of a buffer (330 mM Tris-Acetate pH 7.9, 660 mM KOAc, 100 mMMgOAc2_{,} 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂ PO₄, 3 mM Na₂HPO 7H₂O 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0. 1% BSA-containing phosphate buffer (1 mM KH₂ PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 30 minutes, 50 µL of a stop solution (2N H₂ SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 2. In Solution MA, Solution MB, and Solution MC, increase in absorbance was observed compared to Solution MD. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 3

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂ PO₄, 3 mM Na₂HPO 7H₂O 154 mM NaCl pH7.4).

A 0.5 µg/mL solution of the synthetically obtained biotin-labeled methylcytosine antibody in 0.1% BSA-containing phosphate buffer (1 mM KH₂ PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

For genomic DNA derived from human blood purchased from Clontech, a DNA fragment (X, SEQ ID NO: 19, the region corresponding to the nucleotide numbers 25687390-25687775 shown in Genbank Accession No. NT_029419) used as a test sample was amplified by conducting PCR using the following oligonucleotide primers (PF1 and PR1) designed for PCR of SEQ ID NO: 17 and SEQ ID NO: 18 and the following reaction condition.

### <Oligonucleotide primer designed for PCR>

PF1:5'- CTCAGCACCCAGGCGGCC -3' (SEQ ID NO: 17)
PR1:5'- CTGGCCAAACTGGAGATCGC -3' (SEQ ID NO: 18)

### <DNA fragment>

X:5'-
CTCAGCACCCAGGCGGCCGCGATCATGAGGCGCGAGCGGCGCGGGCTGTTGCAGAGTCTT
GAGCGGGTGGCACACCGCGATGTAGCGGTCGGCTGTCATGACTACCAGCATGTAGGCCGACG
CAAACATGCCGAACACCTGCAGGTGCTTCACCACGCGGCACAGCCAGTCGGGGCCGCGGAAG
CGGTAGGTGATGTCCCAGCACATTTGCGGCAGCACCTGGAAGAATGCCACGGCCAGGTCGGC
CAGGCTGAGGTGTCGGATGAAGAGGTGCATGCGGGACGTCTTGCGCGGCGTCCGGTGCAGAG
CCAGCAGTACGCTGCTGTTGCCCAGCACGGCCACCGCGAAAGTCACCGCCAGCACGGCGATC
TCCAGTTTGGCCAG -3'(SEQ ID NO: 19)

As a reaction liquid of PCR, 10 ng of genomic DNA as a template, each 3 µL of 5 µM of the above primer solutions, 5 µL of each 2 mM dNTP, and 5 µL of 10xbuffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 30 seconds at 95°C, 30 seconds at 61°C and 45 seconds at 72°C.

After conducting PCR, amplification was checked by 2% agarose gel electrophoresis, and the DNA fragment X was purified by Wizard SV Gel/PCR Kit (PROMEGA Corporation).

For the DNA fragment X, the following solutions were prepared respectively in duplicate.
Solution A: DNA fragment X 10ng/10 µL TE buffer solution
Solution B: DNA fragment X 1ng/10 µL TE buffer solution
Solution C: DNA fragment X 0.1ng/10 µL TE buffer solution
Solution D: TE buffer solution (negative control solution)

For genomic DNA derived from human blood purchased from Clontech, a DNA fragment (Y, SEQ ID NO: 24, the region corresponding to the nucleotide numbers 76606-76726 shown in Genbank Accession No. AC009800) used as a test sample was amplified by conducting PCR using the following oligonucleotide primers (PF2 and PR2) designed for PCR of SEQ ID NO: 22 and SEQ ID NO: 23 and the following reaction condition.

### <Oligonucleotide primers designed for PCR>

PF2:5'- TGAGCTCCGTAGGGCGTCC -3' (SEQ ID NO: 22)
PR2:5'- GCGCCGGGTCCGGGCCC -3' (SEQ ID NO: 23)

### <DNA fragment>

Y:5′-
GCGCCGGGTCCGGGCCCGATGCGTTGGCGGGCCAGGGCTCCGAGAACGAGGCGTTGTCCATC
TCAACGAGGGCAGAGGAGCCGGCGACCTGGCGTCCCCCAAGGACGCCCTACGGAGCTCA -3′
(SEQ ID NO: 24)

As a reaction liquid of PCR, 10 ng of genomic DNA as a template, each 3 µL of 5 µM of the above primer solutions, 5 µL of each 2 mM dNTP, and 5 µL of 10xbuffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 50 cycles each consisting of 30 seconds at 95°C, 30 seconds at 60°C and 45 seconds at 72°C.

After conducting PCR, amplification was checked by 2% agarose gel electrophoresis, and the DNA fragment Y was purified by Wizard SV Gel/PCR Kit (PROMEGA Corporation).

For the DNA fragment Y, the following solutions were prepared respectively.
Solution A: DNA fragment Y 10ng/10 µL TE buffer solution
Solution B: DNA fragment Y 1ng/10 µL TE buffer solution
Solution C: DNA fragment Y 0.1ng/10 µL TE buffer solution
Solution D: TE buffer solution (negative control solution)

Solution A of DNA fragment X and Solution A of DNA fragment Y, Solution B of DNA fragment X and Solution B of DNA fragment Y, Solution C of DNA fragment X and Solution C of DNA fragment Y, and Solution D of DNA fragment X and Solution D of DNA fragment Y prepared in the above were respectively mixed, to prepare the following DNA fragment-mixed Solutions MA to MD respectively in duplicate.
Solution MA: 10 ng/20 µL TE buffer solution
Solution MB: 1 ng/20 µL TE buffer solution
SolutionMC: 0.1 ng/20 µL TE buffer solution
Solution MD: TE buffer solution (negative control solution)

Twenty (20) µL of each obtained solution, 0.5 µL of SssI methylase (available from NEB Inc.), 5 µL of 10xNEBuffer2 (available from NEB Inc.), and 0. 5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F2 having the nucleotide sequence of SEQ ID NO: 26 capable of binding by complementation with oligonucleotide X' comprising a target DNA region of SEQ ID NO: 25, and a 0.02 µM solution in Tris-HCl buffer (10 mM) was prepared.

### <Oligonucleotide comprising target DNA region>

Y′:5′-
GCGCCGGGTCCGGGCCCGATGCGTTGGCGGGCCAGGGCTCCGAGAACGAGGCGTTGTCCATC
TCAACGAGGGCAGAGGAGCCGGCGACCTGGCGTCCCCCAAGGACGCCCTACGGAGCTCA -3′
(SEQ ID NO: 25)

### <5'-end FITC-labeled oligonucleotide>

F2:5'- GACAACGCCTCGTTCTCGG -3' (SEQ ID NO: 26)

Each obtained reaction liquid was subjected to the following treatments.

In a PCR tube, 40 µL of the reaction liquid prepared in the above, 10 µL of the 5'-end FITC-labeled oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0. 05% Tween20-containing phosphate buffer (1 mM KH₂ PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂ PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 30 minutes, 50 µL of a stop solution (2N H₂ SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 3. In Solution MA and Solution MB, increase in absorbance was observed compared to Solution MD. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 4

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂ PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂PO₄, 3 mM Na₂HPO 7H₂O, 154 mM NaCl pH 7. 4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

For genomic DNA derived from human blood purchased from Clontech, a DNA fragment (X, SEQ ID NO: 19, the region corresponding to the nucleotide numbers 25687390-25687775 shown in Genbank Accession No. NT_029419) used as a test sample was amplified by conducting PCR using the following oligonucleotide primers (PF1 and PR1) designed for PCR of SEQ ID NO: 17 and SEQ ID NO: 18 and the following reaction condition.

### <Oligonucleotide primer designed for PCR>

PF1:5'- CTCAGCACCCAGGCGGCC -3' (SEQ ID NO: 17)
PR1:5'- CTGGCCAAACTGGAGATCGC -3' (SEQ ID NO: 18)

### <DNA fragment>

X:5′-
CTCAGCACCCAGGCGGCCGCGATCATGAGGCGCGAGCGGCGCGGGCTGTTGCAGAGTCTT
GAGCGGGTGGCACACCGCGATGTAGCGGTCGGCTGTCATGACTACCAGCATGTAGGCCGACG
CAAACATGCCGAACACCTGCAGGTGCTTCACCACGCGGCACAGCCAGTCGGGGCCGCGGAAG
CGGTAGGTGATGTCCCAGCACATTTGCGGCAGCACCTGGAAGAATGCCACGGCCAGGTCGGC
CAGGCTGAGGTGTCGGATGAAGAGGTGCATGCGGGACGTCTTGCGCGGCGTCCGGTGCAGAG
CCAGCAGTACGCTGCTGTTGCCCAGCACGGCCACCGCGAAAGTCACCGCCAGCACGGCGATC
TCCAGTTTGGCCAG -3′(SEQ ID NO: 19)

As a reaction liquid of PCR, 10 ng of genomic DNA as a template, each 3 µL of 5 µM of the above primer solutions, 5 µL of each 2 mM dNTP, and 5 µL of 10xbuffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 30 seconds at 95°C, 30 seconds at 61°C and 45 seconds at 72°C.

After conducting PCR, amplification was checked by 2% agarose gel electrophoresis, and the DNA fragment X was purified by Wizard SV Gel/PCR Kit (PROMEGA Corporation).

For the DNA fragment X, the following solutions were prepared respectively in duplicate.
Solution A: DNA fragment X 10ng/10 µL TE buffer solution
Solution B: DNA fragment X 1ng/10 µL TE buffer solution
Solution C: DNA fragment X 0.1ng/10 µL TE buffer solution
Solution D: TE buffer solution (negative control solution)

For genomic DNA derived from human blood purchased from Clontech, a DNA fragment (Y, SEQ ID NO: 24, the region corresponding to the nucleotide numbers 76606-76726 shown in Genbank Accession No. AC009800) used as a test sample was amplified by conducting PCR using the following oligonucleotide primers (PF2 and PR2) designed for PCR of SEQ ID NO: 22 and SEQ ID NO: 23 and the following reaction condition.

### <Oligonucleotide primers designed for PCR>

PF2:5'- TGAGCTCCGTAGGGCGTCC -3' (SEQ ID NO: 22)
PR2:5'- GCGCCGGGTCCGGGCCC -3' (SEQ ID NO: 23)

### <DNA fragment>

Y:5′-
GCGCCGGGTCCGGCCCGATGCGTTGGCGGCCAGGGCTCCGAGAACGAGGCGTTGTCCATC
TCAACGAGGGCAGAGGAGCCGGCGACCTGGCGTCCCCCAAGGACGCCCTACGGAGCTCA -3′
(SEQ ID NO: 24)

As a reaction liquid of PCR, 10 ng of genomic DNA as a template, each 3 µL of 5 µM of the above primer solutions, 5 µL of each 2 mM dNTP, and 5 µL of 10xbuffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 50 cycles each consisting of 30 seconds at 95°C, 30 seconds at 60°C and 45 seconds at 72°C.

After conducting PCR, amplification was checked by 2% agarose gel electrophoresis, and the DNA fragment Y was purified by Wizard SV Gel/PCR Kit (PROMEGA Corporation).

For the DNA fragment Y, the following solutions were prepared respectively.
Solution A: DNA fragment Y 10ng/10 µL TE buffer solution
Solution B: DNA fragment Y 1ng/10 µL TE buffer solution
Solution C: DNA fragment Y 0.1ng/10 µL TE buffer solution
Solution D: TE buffer solution (negative control solution)

Solution A of DNA fragment X and Solution A of DNA fragment Y, Solution B of DNA fragment X and Solution B of DNA fragment Y, Solution C of DNA fragment X and Solution C of DNA fragment Y, and Solution D of DNA fragment X and Solution D of DNA fragment Y prepared in the above were respectively mixed, to prepare the following DNA fragment-mixed Solutions MA to MD respectively in duplicate.
Solution MA: 10 ng/20 µL TE buffer solution
Solution MB: 1 ng/20 µL TE buffer solution
Solution MC: 0.1 ng/20 µL TE buffer solution
Solution MD: TE buffer solution (negative control solution)

Ten (10) µL of each obtained solution, 0. 5 µL of SssI methylase (available from NEB Inc.), 5 µL of 10xNEBuffer2 (available from NEB Inc.), and 0.5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized were 5'-end FITC-labeled oligonucleotide F1 having the nucleotide sequence of SEQ ID NO: 21 capable of binding by complementation with oligonucleotide X' comprising a target DNA region of SEQ ID NO: 20 and 5'-end FITC-labeled oligonucleotide F2 having the nucleotide sequence of SEQ ID NO: 26 capable of binding by complementation with oligonucleotide X' comprising a target DNA region of SEQ ID NO: 25, and respective 0.02 µM solutions in Tris-HCl buffer (10 mM) were prepared.

### <Oligonucleotide comprising target DNA region>

X′:5′-
CTCAGCACCCAGGCGGCCGCGATCATGAGGCGCGAGCGGCGCGGGCTGTTGCAGAGTCTT
GAGCGGGTGGCACACCGCGATGTAGCGGTCGGCTGTCATGACTACCAGCATGTAGGCCGACG
CAAACATGCCGAACACCTGCAGGTGCTTCACCACGCGGCACAGCCAGTCGGGGCCGCGGAAG
CGGTAGGTGATGTCCCAGCACATTTGCGGCAGCACCTGGAAGAATGCCACGGCCAGGTCGGC
CAGGCTGAGGTGTCGGATGAAGAGGTGCATGCGGGACGTCTTGCGCGGCGTCCGGTGCAGAG
CCAGCAGTACGCTGCTGTTGCCCAGCACGGCCACCGCGAAAGTCACCGCCAGCACGGCGATC
TCCAGTTTGGCCAG -3′ (SEQ ID NO: 20)

Y′:5′-
GCGCCGGGTCCGGGCCCGATGCGTTGGCGGGCCAGGGCTCCGAGAACGAGGCGTTGTCCATC
TCAACGAGGGCAGAGGAGCCGGCGACCTGGCGTCCCCCAAGGACGCCCTACGGAGCTCA -3′
(SEQ ID NO: 25)

### <5'-end FITC-labeled oligonucleotide>

F1:5'- CTGGCCAAACTGGAGAT -3' (SEQ ID NO: 21)
F2:5'- GACAACGCCTCGTTCTCGG -3' (SEQ ID NO: 26)

Each obtained reaction liquid was subjected to the following treatments.

In a PCR tube, 40 µL of the reaction liquid prepared in the above, 10 µL of the 5'-end FITC-labeled oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂ PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 30 minutes, 50 µL of a stop solution (2N H₂ SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 4. In Solution MA, Solution MB, and Solution MC, increase in absorbance was observed compared to Solution MD. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 5

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂ PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂ PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mMEDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment to be used as a test sample (S, SEQ ID NO: 29, the region corresponding to the nucleotide numbers 271743-272083 of yeast chromosome VII shown in Genbank Accession No. NC_001139) was amplified by conducting PCR using oligonucleotide primers (PF3 and PR3) designed for PCR of SEQ ID NO: 27 and SEQ ID NO: 28 and the following reaction condition.

### <Oligonucleotide primers designed for PCR>

PF3: 5'-AGGTGAGCTACGTGTGTTTGG-3' (SEQ ID NO: 27)
PR3: 5'-AGACATGTGCTCACGTACGGT-3' (SEQ ID NO: 28)

### <DNA fragment>

S:

5′-AGGTGAGCTACGTGTGTTTGGGCGTCGTGCACTGGCTCACTTGTACGCGCAGAAATGGC

AGCTTGTACGATTGGTGACCCGCCTTTTCGACACTGGACCGCTATGGACGTGGCGGCGGTGT

GGCGGCGGCTCAATGACCTGTGGCGCCCGTTTGTGGCGTGCGATAGTCGAGCCGCCTGTCAC

GTGCGCGGCCGCCCTGCTCCGTTTGACGCGATGCATAGCATGCGACCACCCAGTAATCATAC

TGCTGACGCTATTGGTCACGTGGTTATGGCAGCTGCTGTTGACTGCGGTGGCGTCCCGTTTC
CACACCGTACGTGAGCACATGTCT-3′ (SEQ ID NO: 29)

As a reaction liquid of PCR, 10 ng of genomic DNA as a template, each 3 µL of 5 µM of the above primer solutions, 5 µL of each 2 mM dNTP, and 5 µL of 10xbuffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment S, the following solutions were prepared respectively in duplicate.
Solution A: DNA fragment S 10ng/20 µL TE buffer solution
Solution B: DNA fragment S 1ng/20 µL TE buffer solution
Solution C: DNA fragment S 0.1ng/20 µL TE buffer solution
Solution D: TE buffer solution (negative control solution)

Twenty (20) µL of each obtained solution, 0.5 µL of SssI methylase (available from NEB Inc.), 5 µL of 10xNEBuffer2 (available from NEB Inc.), and 0.5 µL of 3. 2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F3 having the nucleotide sequence of SEQ ID NO: 31 capable of binding by complementation with oligonucleotide S' comprising a target DNA region of SEQ ID NO: 30, and a 0.02 µM solution in Tris-HCl buffer (10 mM) was prepared.

### <Oligonucleotide comprising target DNA region>

S':5'-

AGGTGAGCTACGTGTGTTTGGGCGTCGTGCACTGGCTCACTTGTACGCGCAGAAATGGCAGC

TTGTACGATTGGTGACCCGCCTTTTCGACACTGGACCGCTATGGACGTGGCGGCGGTGTGGC

GGCGGCTCAATGACCTGTGGCGCCCGTTTGTGGCGTGCGATAGTCGAGCCGCCTGTCACGTG

CGCGGCCGCCCTGCTCCGTTTGACGCGATGCATAGCATGCGACCACCCAGTAATCATACTGC

TGACGCTATTGGTCACGTGGTTATGGCAGCTGCTGTTGACTGCGGTGGCGTCCCGTTTCCAC

ACCGTACGTGAGCACATGTCT -3' (SEQ ID NO: 30)

### <5'-end FITC-labeled oligonucleotide>

F3:5'- CTGGCCAAACTGGAGAT -3' (SEQ ID NO: 31)

Each obtained reaction liquid was subjected to the following treatments.

In a PCR tube, 40 µL of the reaction liquid prepared in the above, 10 µL of the 5'-end FITC-labeled oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0. 05% Tween20-containing phosphate buffer (1 mM KH₂ PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂ PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 30 minutes, 50 µL of a stop solution (2N H₂SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 5. In Solution A, Solution B, and Solution C, increase in absorbance was observed compared to Solution D. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 6

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment to be used as a test sample (S, SEQ ID NO: 29, the region corresponding to the nucleotide numbers 271743-272083 of yeast chromosome VII shown in Genbank Accession No. NC_001139) was amplified by conducting PCR using oligonucleotide primers (PF3 and PR3) designed for PCR of SEQ ID NO: 27 and SEQ ID NO: 28 and the following reaction condition.

### <Oligonucleotide primers designed for PCR>

PF3: 5'-AGGTGAGCTACGTGTGTTTGG-3' (SEQ ID NO: 27)
PR3: 5'-AGACATGTGCTCACGTACGGT-3' (SEQ ID NO: 28)

### <DNA fragment>

S:

5′-AGGTGAGCTACGTGTGTTTGGGCGTCGTGCACTGGCTCACTTGTACGCGCAGAAATGGC

AGCTTGTACGATTGGTGACCCGCCTTTTCGACACTGGACCGCTATGGACGTGGCGGCGGTGT
GGCGGCGGCTCAATGACCTGTGGCGCCCGTTTGTGGCGTGCGATAGTCGAGCCGCCTGTCAC
GTGCGCGGCCGCCCTGCTCCGTTTGACGCGATGCATAGCATGCGACCACCCAGTAATCATAC
TGCTGACGCTATTGGTCACGTGGTTATGGCAGCTGCTGTTGACTGCGGTGGCGTCCCGTTTC
CACACCGTACGTGAGCACATGTCT-3′ (SEQ ID NO: 29)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment S, the following solutions were prepared respectively in duplicate.
Solution A: DNA fragment S 10ng/10 µL TE buffer solution
Solution B: DNA fragment S 1ng/10 µL TE buffer solution
Solution C: TE buffer solution (negative control solution)

From the obtained genomic DNA, a DNA fragment to be used as a test sample (T, SEQ ID NO: 34, the region corresponding to the nucleotide numbers 384569-384685 of yeast chromosome VII shown in Genbank Accession No. NC_001139) was amplified by conducting PCR using oligonucleotide primers (PF4 and PR4) designed for PCR of SEQ ID NO: 32 and SEQ ID NO: 33 and the following reaction condition.

### <Oligonucleotide primers designed for PCR>

PF4: 5'-GGACCTGTGTTTGACGGGTAT-3' (SEQ ID NO: 32)
PR4: 5'-AGTACAGATCTGGCGTTCTCG-3' (SEQ ID NO: 33)

### <DNA fragment>

T:

5′-GGACCTGTGTTTGACGGGTATAACACTAAGTTGCGCAATTTGCTGTATTGCGAAATCCG

CCCGGACGATATCACTCTTGAGCGCATGTGCCGTTTCCGAGAACGCCAGATCTGTACT-3′

(SEQ ID NO: 34)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment T, the following solutions were prepared respectively in duplicate.
Solution A: DNA fragment T 10ng/10 µL TE buffer solution
Solution B: DNA fragment T 1ng/10 µL TE buffer solution
Solution C: TE buffer solution (negative control solution)

Solution A of DNA fragment S and Solution A of DNA fragment T, Solution B of DNA fragment S and Solution B of DNA fragment T, and Solution C of DNA fragment S and Solution C of DNA fragment T prepared in the above were respectively mixed, to prepare the following DNA fragment-mixed Solutions MA to MC respectively in duplicate.
Solution MA: 10 ng/20 µL TE buffer solution
Solution MB: 1 ng/20 µL TE buffer solution
Solution MC: TE buffer solution (negative control solution)

Twenty (20) µL of each obtained solution, 0.5 µL of SssI methylase (available from NEB Inc.), 5 µL of 10×NEBuffer2 (available from NEB Inc.), and 0. 5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F3 having the nucleotide sequence of SEQ ID NO: 31 capable of binding by complementation with oligonucleotide S' comprising a target DNA region of SEQ ID NO: 30, and a 0.02 µM solution in Tris-HCl buffer (10 mM) was prepared.

### <Oligonucleotide comprising target DNA region>

S′:5′-

AGGTGAGCTACGTGTGTTTGGGCGTCGTGCACTGGCTCACTTGTACGCGCAGAAATGGCAGC
TTGTACGATTGGTGACCCGCCTTTTCGACACTGGACCGCTATGGACGTGGCGGCGGTGTGGC
GGCGGCTCAATGACCTGTGGCGCCCGTTTGTGGCGTGCGATAGTCGAGCCGCCTGTCACGTG
CGCGGCCGCCCTGCTCCGTTTGACGCGATGCATAGCATGCGACCACCCAGTAATCATACTGC
TGACGCTATTGGTCACGTGGTTATGGCAGCTGCTGTTGACTGCGGTGGCGTCCCGTTTCCAC
ACCGTACGTGAGCACATGTCT -3′ (SEQ ID NO: 30)

### <5'-end FITC-labeled oligonucleotide>

F3:5'- AGACATGTGCTCACGTACGGT -3' (SEQ ID NO: 31)

Each obtained reaction liquid was subjected to the following treatments.

In a PCR tube, 40 µL of the reaction liquid prepared in the above, 10 µL of the 5'-end FITC-labeled oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 60 minutes, 50 µL of a stop solution (2N H₂SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 6. In Solution MA and Solution MB, increase in absorbance was observed compared to Solution MC. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 7

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂PO₄, 3 mM Na₂HPO 7H₂O, 154 mM NaCl pH7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment to be used as a test sample (S, SEQ ID NO: 29, the region corresponding to the nucleotide numbers 271743-272083 of yeast chromosome VII shown in Genbank Accession No. NC_001139) was amplified by conducting PCR using oligonucleotide primers (PF3 and PR3) designed for PCR of SEQ ID NO: 27 and SEQ ID NO: 28 and the following reaction condition.

### <Oligonucleotide primers designed for PCR>

PF3: 5'-AGGTGAGCTACGTGTGTTTGG-3' (SEQ ID NO: 27)
PR3: 5'-AGACATGTGCTCACGTACGGT-3' (SEQ ID NO: 28)

### <DNA fragment>

S:

5′-AGGTGAGCTACGTGTGTTTGGGCGTCGTGCACTGGCTCACTTGTACGCGCAGAAATGGC

AGCTTGTACGATTGGTGACCCGCCTTTTCGACACTGGACCGCTATGGACGTGGCGGCGGTGT

GGCGGCGGCTCAATGACCTGTGGCGCCCGTTTGTGGCGTGCGATAGTCGAGCCGCCTGTCAC

GTGCGCGGCCGCCCTGCTCCGTTTGACGCGATGCATAGCATGCGACCACCCAGTAATCATAC

TGCTGACGCTATTGGTCACGTGGTTATGGCAGCTGCTGTTGACTGCGGTGGCGTCCCGTTTC

CACACCGTACGTGAGCACATGTCT-3′ (SEQ ID NO: 29)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment S, the following solutions were prepared respectively in duplicate.
Solution A: DNA fragment S 10ng/10 µL TE buffer solution
Solution B: DNA fragment S 1ng/10 µL TE buffer solution
Solution C: TE buffer solution (negative control solution)

From the obtained genomic DNA, a DNA fragment to be used as a test sample (T, SEQ ID NO: 34, the region corresponding to the nucleotide numbers 384569-384685 of yeast chromosome VII shown in Genbank Accession No. NC_001139) was amplified by conducting PCR using oligonucleotide primers (PF4 and PR4) designed for PCR of SEQ ID NO: 32 and SEQ ID NO: 33 and the following reaction condition.

### <Oligonucleotide primers designed for PCR>

PF4: 5'-GGACCTGTGTTTGACGGGTAT-3' (SEQ ID NO: 32)
PR4: 5'-AGTACAGATCTGGCGTTCTCG-3' (SEQ ID NO: 33)

### <DNA fragment>

T:

5′-GGACCTGTGTTTGACGGGTATAACACTAAGTTGCGCAATTTGCTGTATTGCGAAATCCG

CCCGGACGATATCACTCTTGAGCGCATGTGCCGTTTCCGAGAACGCCAGATCTGTACT-3′

(SEQ ID NO: 34)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment T, the following solutions were prepared respectively in duplicate.
Solution A: DNA fragment T 10ng/10 µL TE buffer solution
Solution B: DNA fragment T 1ng/10 µL TE buffer solution
Solution C: TE buffer solution (negative control solution)

Solution A of DNA fragment S and Solution A of DNA fragment T, Solution B of DNA fragment S and Solution B of DNA fragment T, and Solution C of DNA fragment S and Solution C of DNA fragment T prepared in the above were respectively mixed, to prepare the following DNA fragment-mixed Solutions MA to MC respectively in duplicate.
Solution MA: 10 ng/20 µL TE buffer solution
Solution MB: 1 ng/20 µL TE buffer solution
Solution MC: TE buffer solution (negative control solution)

Twenty (20) µL of each obtained solution, 0.5 µL of SssI methylase (available from NEB Inc.), 5 µL of 10×NEBuffer2 (available from NEB Inc.), and 0.5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized were 5'-end FITC-labeled oligonucleotide F3 having the nucleotide sequence of SEQ ID NO: 31 capable of binding by complementation with oligonucleotide S' comprising a target DNA region of SEQ ID NO: 30 and 5'-end FITC-labeled oligonucleotide F4 having the nucleotide sequence of SEQ ID NO: 36 capable of binding by complementation with oligonucleotide T' comprising a target DNA region of SEQ ID NO: 35, and respective 0.02 µM solutions in Tris-HCl buffer (10 mM) were prepared.

### <Oligonucleotide comprising target DNA region>

S':5'-
AGGTGAGCTACGTGTGTTTGGGCGTCGTGCACTGGCTCACTTGTACGCGCAGAAATGGCAGC
TTGTACGATTGGTGACCCGCCTTTTCGACACTGGACCGCTATGGACGTGGCGGCGGTGTGGC
GGCGGCTCAATGACCTGTGGCGCCCGTTTGTGGCGTGCGATAGTCGAGCCGCCTGTCACGTG
CGCGGCCGCCCTGCTCCGTTTGACGCGATGCATAGCATGCGACCACCCAGTAATCATACTGC
TGACGCTATTGGTCACGTGGTTATGGCAGCTGCTGTTGACTGCGGTGGCGTCCCGTTTCCAC
ACCGTACGTGAGCACATGTCT -3' (SEQ ID NO: 30)

T':5'-

GGACCTGTGTTTGACGGGTATAACACTAAGTTGCGCAATTTGCTGTATTGCGAAATCCGCCC

GGACGATATCACTCTTGAGCGCATGTGCCGTTTCCGAGAACGCCAGATCTGTACT -3′
(SEQ ID NO: 35)

### <5'-end FITC-labeled oligonucleotide>

F3:5'- AGACATGTGCTCACGTACGGT -3' (SEQ ID NO: 31)
F4:5'- AGTACAGATCTGGCGTTCTCG -3' (SEQ ID NO: 36)

Each obtained reaction liquid was subjected to the following treatments.

In a PCR tube, 40 µL of the reaction liquid prepared in the above, 10 µL of the 5'-end FITC-labeled oligonucleotide solution, 10 µL of a buffer (330 mM Tris-Acetate pH 7.9, 660 mM KOAc, 100 mMMgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 60 minutes, 50 µL of a stop solution (2N H₂SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 7. In Solution MA and Solution MB, increase in absorbance was observed compared to Solution MC. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 8

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

Using genomic DNA derived from human blood purchased from Clontech, the following solutions were prepared respectively in duplicate.

Solution A: Genomic DNA derived from human blood 100 ng/5 µL TE buffer solution
Solution B: Genomic DNA derived from human blood 10 ng/5 µL TE buffer solution
Solution C: Genomic DNA derived from human blood 1 ng/5 µL TE buffer solution
Solution D: TE buffer solution (negative control solution)

Five (5) µL of each obtained solution, 10 U of restriction enzyme XspI, and 2 µL of 10x buffer optimum for XspI (200 mM Tris-HCl pH8.5, 100 mM MgCl₂, 10 mM Dithiothreitol, 1000 mM KCl) were mixed, and added with sterilized ultrapure water to prepare a reaction liquid having a liquid amount of 20 µL. The reaction liquid was incubated at 37°C for 1 hour.

Twenty (20) µL of each obtained solution, 0.5 µL of SssI methylase (available from NEB Inc.), 5 µL of 10xNEBuffer2 (available from NEB Inc.), and 0. 5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F5 having the nucleotide sequence of SEQ ID NO: 38 capable of binding by complementation with oligonucleotide Z (region corresponding to the nucleotide numbers 115-386 shown in Genbank Accession No. M80340) comprising a target DNA region of SEQ ID NO: 37 designed in LINE1 region known as human transposon, and a 0.02 µM solution in Tris-HCl buffer (10 mM) was prepared.

### <Oligonucleotide comprising target DNA region>

Z:5′-

TAGGGAGTGCCAGACAGTGGGCGCAGGCCAGTGTGTGTGCGCACCGTGCGCGAGCCGAAGCA

GGGCGAGGCATTGCCTCACCTGGGAAGCGCAAGGGGTCAGGGAGTTCCCTTTCTGAGTCAAA

GAAAGGGGTGACGGTCGCACCTGGAAAATCGGGTCACTCCCACCCGAATATTGCGCTTTTCA

GACCGGCTTAAGAAACGGCGCACCACGAGACTATATCCCACACCTGGCTCGGAGGGTCCTAC

GCCCACGGAATCTCGCTGATTGC -3′ (SEQ ID NO: 37)

### <5'-end FITC-labeled oligonucleotide>

F5:5'- CTGGCCAAACTGGAGAT -3' (SEQ ID NO: 38)

Each obtained reaction liquid was subjected to the following treatments.

In a PCR tube, 40 µL of the reaction liquid prepared in the above, 10 µL of the 5'-end FITC-labeled oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0. 05% Tween20-containing phosphate buffer (1 mM KH₂PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 10 minutes, 50 µL of a stop solution (2N H₂SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 8. In Solution A, Solution B, and Solution C, increase in absorbance was observed compared to Solution D. The intensity increased depending on the concentration of genomic DNA. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 9

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂PO₄, 3 mM Na₂HPO 7H₂O, 154 mM NaCl pH7.4).

A 0.5 µg/mL solution.of the synthetically obtained biotin-labeled methylcytosine antibody in 0.1% BSA-containing phosphate buffer (1 mM KH₂ PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

Using genomic DNA derived from human blood purchased from Clontech, the following solutions were prepared respectively in duplicate.
Solution A: Genomic DNA derived from human blood 100 ng/5 µL TE buffer solution
Solution B: Genomic DNA derived from human blood 10 ng/5 µL TE buffer solution
Solution C: Genomic DNA derived from human blood 1 ng/5 µL TE buffer solution
Solution D: TE buffer solution (negative control solution)

Five (5) µL of each obtained solution, 4 U of restriction enzyme MspI, and 2 µL of 10x buffer optimum for MspI (100 mM Tris-HCl pH 8.5, 100 mM MgCl₂, 10 mM Dithiothreitol, 500 mM NaCl) were mixed, and added with sterilized ultrapure water to prepare a reaction liquid having a liquid amount of 20 µL. The reaction liquid was incubated at 37°C for 1 hour.

Twenty (20) µL of each obtained solution, 0.5 µL of SssI methylase (available from NEB Inc.), 5 µL of 10xNEBuffer2 (available from NEB Inc.), and 0. 5 µL of 3. 2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F6 having the nucleotide sequence of SEQ ID NO: 40 capable of binding by complementation with oligonucleotide W (region corresponding to the nucleotide numbers 178-262 shown in Genbank Accession No. AF458110) comprising a target DNA region of SEQ ID NO: 39 designed in Alu region known as human transposon, and a 0.02 µM solution in Tris-HCl buffer (10 mM) was prepared.

### <Oligonucleotide comprising target DNA region>

W:5'-

CGGGCGCGGTGGCTCACGCCTGTAATCCCAGCACTTTGGGAGGCCGAGGTGGGCGGATCACG

AGGTCAGGAGATCGAGACCATCC -3' (SEQ ID NO: 39)

### <5'-end FITC-labeled oligonucleotide>

F6:5'- GGATGGTCTCGATCTCCTGAC -3' (SEQ ID NO: 40)

Each obtained reaction liquid was subjected to the following treatments.

In a PCR tube, 40 µL of the reaction liquid prepared in the above, 10 µL of the 5'-end FITC-labeled oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0. 05% Tween20-containing phosphate buffer (1 mM KH₂PO4, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 8 minutes, 50 µL of a stop solution (2N H₂SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 9. In Solution A, Solution B, and Solution C, increase in absorbance was observed compared to Solution D. The intensity increased depending on the concentration of genomic DNA. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 10

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0. 1% BSA-containing phosphate buffer (1 mM KH₂PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment to be used as a test sample (S, SEQ ID NO: 29, the region corresponding to the nucleotide numbers 271743-272083 of yeast chromosome VII shown in Genbank Accession No. NC_001139) was amplified by conducting PCR using oligonucleotide primers (PF3 and PR3) designed for PCR of SEQ ID NO: 27 and SEQ ID NO: 28 and the following reaction condition.

### <Oligonucleotide primers designed for PCR>

PF3: 5'-AGGTGAGCTACGTGTGTTTGG-3' (SEQ ID NO: 27)
PR3: 5'-AGACATGTGCTCACGTACGGT-3' (SEQ ID NO: 28)

### <DNA fragment>

S:

5′-AGGTGAGCTACGTGTGTTTGGGCGTCGTGCACTGGCTCACTTGTACGCGCAGAAATGGC

AGCTTGTACGATTGGTGACCCGCCTTTTCGACACTGGACCGCTATGGACGTGGCGGCGGTGT

GGCGGCGGCTCAATGACCTGTGGCGCCCGTTTGTGGCGTGCGATAGTCGAGCCGCCTGTCAC

GTGCGCGGCCGCCCTGCTCCGTTTGACGCGATGCATAGCATGCGACCACCCAGTAATCATAC

TGCTGACGCTATTGGTCACGTGGTTATGGCAGCTGCTGTTGACTGCGGTGGCGTCCCGTTTC

CACACCGTACGTGAGCACATGTCT-3′ (SEQ ID NO: 29)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment S, the following solutions were prepared respectively in duplicate.
Solution A: DNA fragment S 10ng/20 µL TE buffer solution
Solution B: DNA fragment S 1ng/20 µL TE buffer solution
Solution C: DNA fragment S 0.1ng/20 µL TE buffer solution
Solution D: TE buffer solution (negative control solution)

Twenty (20) µL of each obtained solution, 0.5 µL of SssI methylase (available from NEB Inc.), 5 µL of 10xNEBuffer2 (available from NEB Inc.), and 0.5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F3 having the nucleotide sequence of SEQ ID NO: 31 capable of binding by complementation with oligonucleotide S' comprising a target DNA region of SEQ ID NO: 30, and a 0.02 µM solution in Tris-HCl buffer (10 mM) was prepared.

### <Oligonucleotide comprising target DNA region>

S′:5′-
AGGTGAGCTACGTGTGTTTGGGCGTCGTGCACTGGCTCACTTGTACGCGCAGAAATGGCAGC
TTGTACGATTGGTGACCCGCCTTTTCGACACTGGACCGCTATGGACGTGGCGGCGGTGTGGC
GGCGGCTCAATGACCTGTGGCGCCCGTTTGTGGCGTGCGATAGTCGAGCCGCCTGTCACGTG
CGCGGCCGCCCTGCTCCGTTTGACGCGATGCATAGCATGCGACCACCCAGTAATCATACTGC
TGACGCTATTGGTCACGTGGTTATGGCAGCTGCTGTTGACTGCGGTGGCGTCCCGTTTCCAC
ACCGTACGTGAGCACATGTCT -3′ (SEQ ID NO: 30)

### <5'-end FITC-labeled oligonucleotide>

F3:5'- CTGGCCAAACTGGAGAT -3' (SEQ ID NO: 31)

Synthesized were counter oligonucleotides C1, C2, C3, C4, C5, C6, C7, C8, C9 and C10 having the nucleotide sequences of SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48 and SEQ ID NO: 49 and SEQ ID NO: 50, respectively capable of binding by complementation with a minus strand of DNA fragment S' comprising the target DNA region of SEQ ID NO: 30, and respective 0.01 µM TE buffer solutions were prepared.

### <Counter oligonucleotides>

C1:5'- AGGTGAGCTACGTGTGTTTGG -3' (SEQ ID NO: 41)
C2:5'- GCGTCGTGCACTGGCTCACTTGTACGCGCA -3' (SEQ ID NO: 42)
C3:5'- CTTGTACGATTGGTGACCCGCCTTTTCGAC -3' (SEQ ID NO: 43)
C4:5'- ACTGGACCGCTATGGACGTGGCGGCGGTGT -3' (SEQ ID NO: 44)
C5:5'- GGCGGCGGCTCAATGACCTGTGGCGCCCGT -3' (SEQ ID NO: 45)
C6:5'- TTGTGGCGTGCGATAGTCGAGCCGCCTGTC -3' (SEQ ID NO: 46)
C7:5'- ACGTGCGCGGCCGCCCTGCTCCGTT -3' (SEQ ID NO: 47)
C8:5'- TGACGCGATGCATAGCATGCGACCACCCAG -3' (SEQ ID NO: 48)
C9:5'- ACTGCTGACGCTATTGGTCACGTGGTTATG -3' (SEQ ID NO: 49)
C10:5'- CTGCTGTTGACTGCGGTGGCGTCCCGTTTC -3' (SEQ ID NO: 50)

Each obtained reaction liquid was subjected to the following treatments.

In a PCR tube, 40 µL of the reaction liquid prepared in the above, 10 µL of the 5'-end FITC-labeled oligonucleotide solution, 10 µL of the counter oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 30 minutes, 50 µL of a stop solution (2N H₂SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 10. In Solution A, Solution B, and Solution C, increase in absorbance was observed compared to Solution D. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 11

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture.was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment to be used as a test sample (S, SEQ ID NO: 29, the region corresponding to the nucleotide numbers 271743-272083 of yeast chromosome VII shown in Genbank Accession No. NC_001139) was amplified by conducting PCR using oligonucleotide primers (PF3 and PR3) designed for PCR of SEQ ID NO: 27 and SEQ ID NO: 28 and the following reaction condition.

### <Oligonucleotide primers designed for PCR>

PF3: 5'-AGGTGAGCTACGTGTGTTTGG-3' (SEQ ID NO: 27)
PR3: 5'-AGACATGTGCTCACGTACGGT-3' (SEQ ID NO: 28)

### <DNA fragment>

S:

5′-AGGTGAGCTACGTGTGTTTGGGCGTCGTGCACTGGCTCACTTGTACGCGCAGAAATGGC

AGCTTGTACGATTGGTGACCCGCCTTTTCGACACTGGACCGCTATGGACGTGGCGGCGGTGT

GGCGGCGGCTCAATGACCTGTGGCGCCCGTTTGTGGCGTGCGATAGTCGAGCCGCCTGTCAC

GTGCGCGGCCGCCCTGCTCCGTTTGACGCGATGCATAGCATGCGACCACCCAGTAATCATAC

TGCTGACGCTATTGGTCACGTGGTTATGGCAGCTGCTGTTGACTGCGGTGGCGTCCCGTTTC

CACACCGTACGTGAGCACATGTCT-3′ (SEQ ID NO: 29)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment S, the following solutions were prepared respectively in duplicate.
Solution A: DNA fragment S 10ng/10 µL TE buffer solution
Solution B: DNA fragment S 1ng/10 µL TE buffer solution
Solution C: TE buffer solution (negative control solution)

From the obtained genomic DNA, a DNA fragment to be used as a test sample (T, SEQ ID NO: 34, the region corresponding to the nucleotide numbers 384569-384685 of yeast chromosome VII shown in Genbank Accession No. NC_001139) was amplified by conducting PCR using oligonucleotide primers (PF4 and PR4) designed for PCR of SEQ ID NO: 32 and SEQ ID NO: 33 and the following reaction condition.

### <Oligonucleotide primers designed for PCR>

PF4: 5'-GGACCTGTGTTTGACGGGTAT-3' (SEQ ID NO: 32)
PR4: 5'-AGTACAGATCTGGCGTTCTCG-3' (SEQ ID NO: 33)

### <DNA fragment>

T:

5′-GGACCTGTGTTTGACGGGTATAACACTAAGTTGCGCAATTTGCTGTATTGCGAAATCCG

CCCGGACGATATCACTCTTGAGCGCATGTGCCGTTTCCGAGAACGCCAGATCTGTACT-3′

(SEQ ID NO: 34)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment T, the following solutions were prepared respectively in duplicate.
Solution A: DNA fragment T 10ng/10 µL TE buffer solution
Solution B: DNA fragment T 1ng/10 µL TE buffer solution
Solution C: TE buffer solution (negative control solution)

Solution A of DNA fragment S and Solution A of DNA fragment T, Solution B of DNA fragment S and Solution B of DNA fragment T, and Solution C of DNA fragment S and Solution C of DNA fragment T prepared in the above were respectively mixed, to prepare the following DNA fragment-mixed Solutions MA to MC respectively in duplicate.
Solution MA: 10 ng/20 µL TE buffer solution
Solution MB: 1 ng/20 µL TE buffer solution
Solution MC: TE buffer solution (negative control solution)

Twenty (20) µL of each obtained solution, 0.5 µL of SssI methylase (available from NEB Inc.), 5 µL of 10xNEBuffer2 (available from NEB Inc.), and 0. 5 µL of 3. 2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F3 having the nucleotide sequence of SEQ ID NO: 31 capable of binding by complementation with oligonucleotide S' comprising a target DNA region of SEQ ID NO: 30, and a 0.02 µM solution in Tris-HCl buffer (10 mM) was prepared.

### <Oligonucleotide comprising target DNA region>

S′:5′-
AGGTGAGCTACGTGTGTTTGGGCGTCGTGCACTGGCTCACTTGTACGCGCAGAAATGGCAGC
TTGTACGATTGGTGACCCGCCTTTTCGACACTGGACCGCTATGGACGTGGCGGCGGTGTGGC
GGCGGCTCAATGACCTGTGGCGCCCGTTTGTGGCGTGCGATAGTCGAGCCGCCTGTCACGTG
CGCGGCCGCCCTGCTCCGTTTGACGCGATGCATAGCATGCGACCACCCAGTAATCATACTGC
TGACGCTATTGGTCACGTGGTTATGGCAGCTGCTGTTGACTGCGGTGGCGTCCCGTTTCCAC
ACCGTACGTGAGCACATGTCT -3′ (SEQ ID NO: 30)

### <5'-end FITC-labeled oligonucleotide>

F3:5'- AGACATGTGCTCACGTACGGT -3' (SEQ ID NO: 31)

Synthesized were counter oligonucleotides C1, C2, C3, C4, C5, C6, C7, C8, C9 and C10 having the nucleotide sequences of SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48 and SEQ ID NO: 49 and SEQ ID NO: 50, respectively capable of binding by complementation with a minus strand of DNA fragment S' comprising the target DNA region of SEQ ID NO: 30, and respective 0.01 µM TE buffer solutions were prepared.

### <Counter oligonucleotides>

C1:5'- AGGTGAGCTACGTGTGTTTGG -3' (SEQ ID NO: 41)
C2:5'- GCGTCGTGCACTGGCTCACTTGTACGCGCA -3' (SEQ ID NO: 42)
C3:5'- CTTGTACGATTGGTGACCCGCCTTTTCGAC -3' (SEQ ID NO: 43)
C4:5'- ACTGGACCGCTATGGACGTGGCGGCGGTGT -3' (SEQ ID NO: 44)
C5:5'- GGCGGCGGCTCAATGACCTGTGGCGCCCGT -3' (SEQ ID NO: 45)
C6:5'- TTGTGGCGTGCGATAGTCGAGCCGCCTGTC -3' (SEQ ID NO: 46)
C7:5'- ACGTGCGCGGCCGCCCTGCTCCGTT -3' (SEQ ID NO: 47)
C8:5'- TGACGCGATGCATAGCATGCGACCACCCAG -3' (SEQ ID NO: 48)
C9:5'- ACTGCTGACGCTATTGGTCACGTGGTTATG -3' (SEQ ID NO: 49)
C10:5'- CTGCTGTTGACTGCGGTGGCGTCCCGTTTC -3' (SEQ ID NO: 50)

Each obtained reaction liquid was subjected to the following treatments.

In a PCR tube, 40 µL of the reaction liquid prepared in the above, 10 µL of the 5'-end FITC-labeled oligonucleotide solution, 10 µL of the counter oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 60 minutes, 50 µL of a stop solution (2N H₂SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 11. In Solution MA and Solution MB, increase in absorbance was observed compared to Solution MC. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 12

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment to be used as a test sample (S, SEQ ID NO: 29, the region corresponding to the nucleotide numbers 271743-272083 of yeast chromosome VII shown in Genbank Accession No. NC_001139) was amplified by conducting PCR using oligonucleotide primers (PF3 and PR3) designed for PCR of SEQ ID NO: 27 and SEQ ID NO: 28 and the following reaction condition.

### <Oligonucleotide primers designed for PCR>

PF3: 5'-AGGTGAGCTACGTGTGTTTGG-3' (SEQ ID NO: 27)
PR3: 5'-AGACATGTGCTCACGTACGGT-3' (SEQ ID NO: 28)

### <DNA fragment>

S:

5′-AGGTGAGCTACGTGTGTTTGGGCGTCGTGCACTGGCTCACTTGTACGCGCAGAAATGGC

AGCTTGTACGATTGGTGACCCGCCTTTTCGACACTGGACCGCTATGGACGTGGCGGCGGTGT

GGCGGCGGCTCAATGACCTGTGGCGCCCGTTTGTGGCGTGCGATAGTCGAGCCGCCTGTCAC
GTGCGCGGCCGCCCTGCTCCGTTTGACGCGATGCATAGCATGCGACCACCCAGTAATCATAC
TGCTGACGCTATTGGTCACGTGGTTATGGCAGCTGCTGTTGACTGCGGTGGCGTCCCGTTTC
CACACCGTACGTGAGCACATGTCT-3' (SEQ ID NO: 29)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment S, the following solutions were prepared respectively in duplicate.
Solution A: DNA fragment S 10ng/10 µL TE buffer solution
Solution B: DNA fragment S 1ng/10 µL TE buffer solution
Solution C: TE buffer solution (negative control solution)

From the obtained genomic DNA, a DNA fragment to be used as a test sample (T, SEQ ID NO: 34, the region corresponding to the nucleotide numbers 384569-384685 of yeast chromosome VII shown in Genbank Accession No. NC_001139) was amplified by conducting PCR using oligonucleotide primers (PF4 and PR4) designed for PCR of SEQ ID NO: 32 and SEQ ID NO: 33 and the following reaction condition.

### <Oligonucleotide primers designed for PCR>

PF4: 5'-GGACCTGTGTTTGACGGGTAT-3' (SEQ ID NO: 32)
PR4: 5'-AGTACAGATCTGGCGTTCTCG-3' (SEQ ID NO: 33)

### <DNA fragment>

T:

5′-GGACCTGTGTTTGACGGGTATAACACTAAGTTGCGCAATTTGCTGTATTGCGAAATCCG

CCCGGACGATATCACTCTTGAGCGCATGTGCCGTTTCCGAGAACGCCAGATCTGTACT-3′

(SEQ ID NO: 34)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment T, the following solutions were prepared respectively in duplicate.
Solution A: DNA fragment T 10ng/10 µL TE buffer solution
Solution B: DNA fragment T 1ng/10 µL TE buffer solution
Solution C: TE buffer solution (negative control solution)

Solution A of DNA fragment S and Solution A of DNA fragment T, Solution B of DNA fragment S and Solution B of DNA fragment T, and Solution C of DNA fragment S and Solution C of DNA fragment T prepared in the above were respectively mixed, to prepare the following DNA fragment-mixed Solutions MA to MC respectively in duplicate.
Solution MA: 10 ng/20 µL TE buffer solution
Solution MB: 1 ng/20 µL TE buffer solution
Solution MC: TE buffer solution (negative control solution)

Twenty (20) µL of each obtained solution, 0.5 µL of SssI methylase (available from NEB Inc.), 5 µL of 10xNEBuffer2 (available from NEB Inc.), and 0. 5 µL of 3. 2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F4 having the nucleotide sequence of SEQ ID NO: 36 capable of binding by complementation with oligonucleotide T' comprising a target DNA region of SEQ ID NO: 35, and a 0.02 µM solution in Tris-HCl buffer (10 mM) was prepared.

### <Oligonucleotide comprising target DNA region>

T′:5′-

GGACCTGTGTTTGACGGGTATAACACTAAGTTGCGCAATTTGCTGTATTGCGAAATCCGCCC

GGACGATATCACTCTTGAGCGCATGTGCCGTTTCCGAGAACGCCAGATCTGTACT -3'
(SEQ ID NO: 35)

### <5'-end FITC-labeled oligonucleotide>

F4:5'- AGTACAGATCTGGCGTTCTCG -3' (SEQ ID NO: 36)

Synthesized were counter oligonucleotides C11, C12, C13 and C14 having the nucleotide sequences of SEQ ID NO: 51, SEQ ID NO: 52, SEQ ID NO: 53 and SEQ ID NO: 54, respectively capable of binding by complementation with a minus strand of DNA fragment T' comprising the target DNA region of SEQ ID NO: 35, and respective 0.01 µM TE buffer solutions were prepared.

### <Counter oligonucleotides>

C11:5'- GGACCTGTGTTTGACGGGTAT -3' (SEQ ID NO: 51)
C12:5'- AACACTAAGTTGCGCAATTTGCTGT -3' (SEQ ID NO: 52)
C13:5'- ATTGCGAAATCCGCCCGGACGATAT -3' (SEQ ID NO: 53)
C14:5'- CACTCTTGAGCGCATGTGCCGTTTC -3' (SEQ ID NO: 54)

Each obtained reaction liquid was subjected to the following treatments.

In a PCR tube, 40 µL of the reaction liquid prepared in the above, 10 µL of the 5'-end FITC-labeled oligonucleotide solution, 10 µL of the counter oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 60 minutes, 50 µL of a stop solution (2N H₂ SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 12. In Solution MA and Solution MB, increase in absorbance was observed compared to Solution MC. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 13

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂ PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂ PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method) .

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment to be used as a test sample (S, SEQ ID NO: 29, the region corresponding to the nucleotide numbers 271743-272083 of yeast chromosome VII shown in Genbank Accession No. NC_001139) was amplified by conducting PCR using oligonucleotide primers (PF3 and PR3) designed for PCR of SEQ ID NO: 27 and SEQ ID NO: 28 and the following reaction condition.

### <Oligonucleotide primers designed for PCR>

PF3: 5'-AGGTGAGCTACGTGTGTTTGG-3' (SEQ ID NO: 27)
PR3: 5'-AGACATGTGCTCACGTACGGT-3' (SEQ ID NO: 28)

### <DNA fragment>

S:

5′-AGGTGAGCTACGTGTGTTTGGGCGTCGTGCACTGGCTCACTTGTACGCGCAGAAATGGC

AGCTTGTACGATTGGTGACCCGCCTTTTCGACACTGGACCGCTATGGACGTGGCGGCGGTGT

GGCGGCGGCTCAATGACCTGTGGCGCCCGTTTGTGGCGTGCGATAGTCGAGCCGCCTGTCAC
GTGCGCGGCCGCCCTGCTCCGTTTGACGCGATGCATAGCATGCGACCACCCAGTAATCATAC
TGCTGACGCTATTGGTCACGTGGTTATGGCAGCTGCTGTTGACTGCGGTGGCGTCCCGTTTC
CACACCGTACGTGAGCACATGTCT-3′ (SEQ ID NO: 29)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment S, the following solutions were prepared respectively in duplicate.
Solution A: DNA fragment S 10ng/10 µL TE buffer solution
Solution B: DNA fragment S 1ng/10 µL TE buffer solution
Solution C: TE buffer solution (negative control solution)

From the obtained genomic DNA, a DNA fragment to be used as a test sample (T, SEQ ID NO: 34, the region corresponding to the nucleotide numbers 384569-384685 of yeast chromosome VII shown in Genbank Accession No. NC_001139) was amplified by conducting PCR using oligonucleotide primers (PF4 and PR4) designed for PCR of SEQ ID NO: 32 and SEQ ID NO: 33 and the following reaction condition.

### <Oligonucleotide primers designed for PCR>

PF4: 5'-GGACCTGTGTTTGACGGGTAT-3' (SEQ ID NO: 32)
PR4: 5'-AGTACAGATCTGGCGTTCTCG-3' (SEQ ID NO: 33)

### <DNA fragment>

T:
5′-GGACCTGTGTTTGACGGGTATAACACTAAGTTGCGCAATTTGCTGTATTGCGAAATCCG
CCCGGACGATATCACTCTTGAGCGCATGTGCCGTTTCCGAGAACGCCAGATCTGTACT-3′
(SEQ ID NO: 34)

As a reaction liquid of PCR, 10 ng of genomic DNA as a template, each 3 µL of 5 µM of the above primer solutions, 5 µL of each 2 mM dNTP, and 5 µL of 10×buffer (100 mM Tris-HCl pH 8.3, 500 mM KCl, 15 mM MgCl₂, 0.01% Gelatin) were mixed with 0.25 µL of 5U/µL thermostable DNA polymerase (AmpliTaq Gold), and added with sterilized ultrapure water to prepare a reaction liquid having a liquid amount of 50 µL. After retaining the reaction liquid at 95°C for 10 minutes, PCR was conducted by 40 cycles each consisting of 20 seconds at 95°C, 30 seconds at 58°C and 30 seconds at 72°C.

After conducting PCR, amplification was checked by 2% agarose gel electrophoresis, and the DNA fragment S was purified by Wizard SV Gel/PCR Kit (PROMEGA Corporation).

For the DNA fragment T, the following solutions were prepared respectively in duplicate.
Solution A: DNA fragment T 10ng/10 µL TE buffer solution
Solution B: DNA fragment T 1ng/10 µL TE buffer solution
Solution C: TE buffer solution (negative control solution)

Solution A of DNA fragment S and Solution A of DNA fragment T, Solution B of DNA fragment S and Solution B of DNA fragment T, and Solution C of DNA fragment S and Solution C of DNA fragment T prepared in the above were respectively mixed, to prepare the following DNA fragment-mixed Solutions MA to MC respectively in duplicate.
Solution MA: 10 ng/20 µL TE buffer solution
Solution MB: 1 ng/20 µL TE buffer solution
Solution MC: TE buffer solution (negative control solution)

Twenty (20) µL of each obtained solution, 0.5 µL of SssI methylase (available from NEB Inc.), 5 µL of 10xNEBuffer2 (available from NEB Inc.), and 0. 5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized were 5'-end FITC-labeled oligonucleotide F3 having the nucleotide sequence of SEQ ID NO: 31 capable of binding by complementation with oligonucleotide S' comprising a target DNA region of SEQ ID NO: 30 and 5'-end FITC-labeled oligonucleotide F4 having the nucleotide sequence of SEQ ID NO: 36 capable of binding by complementation with oligonucleotide T' comprising a target DNA region of SEQ ID NO: 35, and respective 0. 02 µM solutions in Tris-HCl buffer (10 mM) were prepared.

### <Oligonucleotide comprising target DNA region>

S′:5′-
AGGTGAGCTACGTGTGTTTGGGCGTCGTGCACTGGCTCACTTGTACGCGCAGAAATGGCAGC
TTGTACGATTGGTGACCCGCCTTTTCGACACTGGACCGCTATGGACGTGGCGGCGGTGTGGC
GGCGGCTCAATGACCTGTGGCGCCCGTTTGTGGCGTGCGATAGTCGAGCCGCCTGTCACGTG
CGCGGCCGCCCTGCTCCGTTTGACGCGATGCATAGCATGCGACCACCCAGTAATCATACTGC
TGACGCTATTGGTCACGTGGTTATGGCAGCTGCTGTTGACTGCGGTGGCGTCCCGTTTCCAC
ACCGTACGTGAGCACATGTCT -3′ (SEQ ID NO: 30)

T′:5′-
GGACCTGTGTTTGACGGGTATAACACTAAGTTGCGCAATTTGCTGTATTGCGAAATCCGCCC
GGACGATATCACTCTTGAGCGCATGTGCCGTTTCCGAGAACGCCAGATCTGTACT -3′
(SEQ ID NO: 35)

### <5'-end FITC-labeled oligonucleotide>

F3:5'- AGACATGTGCTCACGTACGGT -3' (SEQ ID NO: 31)
F4:5'- AGTACAGATCTGGCGTTCTCG -3' (SEQ ID NO: 36)

Synthesized were counter oligonucleotides C1, C2, C3, C4, C5, C6, C7, C8, C9 and C10 having the nucleotide sequences of SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48 and SEQ ID NO: 49 and SEQ ID NO: 50, respectively capable of binding by complementation with a minus strand of DNA fragment S' comprising the target DNA region of SEQ ID NO: 30, and respective 0.01 µM TE buffer solutions were prepared.

Synthesized were counter oligonucleotides C11, C12, C13 and C14 having the nucleotide sequences of SEQ ID NO: 51, SEQ ID NO: 52, SEQ ID NO: 53 and SEQ ID NO: 54, respectively capable of binding by complementation with a minus strand of DNA fragment T' comprising the target DNA region of SEQ ID NO: 35, and respective 0.01 µM TE buffer solutions were prepared.

### <Oligonucleotide comprising target DNA region>

S':5'-

AGGTGAGCTACGTGTGTTTGGGCGTCGTGCACTGGCTCACTTGTACGCGCAGAAATGGCAGC

TTGTACGATTGGTGACCCGCCTTTTCGACACTGGACCGCTATGGACGTGGCGGCGGTGTGGC

GGCGGCTCAATGACCTGTGGCGCCCGTTTGTGGCGTGCGATAGTCGAGCCGCCTGTCACGTG

CGCGGCCGCCCTGCTCCGTTTGACGCGATGCATAGCATGCGACCACCCAGTAATCATACTGC

TGACGCTATTGGTCACGTGGTTATGGCAGCTGCTGTTGACTGCGGTGGCGTCCCGTTTCCAC

ACCGTACGTGAGCACATGTCT -3' (SEQ ID NO: 30)

T':5'-

GGACCTGTGTTTGACGGGTATAACACTAAGTTGCGCAATTTGCTGTATTGCGAAATCCGCCC

GGACGATATCACTCTTGAGCGCATGTGCCGTTTCCGAGAACGCCAGATCTGTACT -3'

(SEQ ID NO: 35)

### <Counter oligonucleotides>

C1:5'- AGGTGAGCTACGTGTGTTTGG -3' (SEQ ID NO: 41)
C2:5'- GCGTCGTGCACTGGCTCACTTGTACGCGCA -3' (SEQ ID NO: 42)
C3:5'- CTTGTACGATTGGTGACCCGCCTTTTCGAC -3' (SEQ ID NO: 43)
C4:5'- ACTGGACCGCTATGGACGTGGCGGCGGTGT -3' (SEQ ID NO: 44)
C5:5'- GGCGGCGGCTCAATGACCTGTGGCGCCCGT -3' (SEQ ID NO: 45)
C6:5'- TTGTGGCGTGCGATAGTCGAGCCGCCTGTC -3' (SEQ ID NO: 46)
C7:5'- ACGTGCGCGGCCGCCCTGCTCCGTT -3' (SEQ ID NO: 47)
C8:5'- TGACGCGATGCATAGCATGCGACCACCCAG -3' (SEQ ID NO: 48)
C9:5'- ACTGCTGACGCTATTGGTCACGTGGTTATG -3' (SEQ ID NO: 49)
C10:5'- CTGCTGTTGACTGCGGTGGCGTCCCGTTTC -3' (SEQ ID NO: 50)
C11:5'- GGACCTGTGTTTGACGGGTAT -3' (SEQ ID NO: 51)
C12:5'- AACACTAAGTTGCGCAATTTGCTGT -3' (SEQ ID NO: 52)
C13:5'- ATTGCGAAATCCGCCCGGACGATAT -3' (SEQ ID NO: 53)
C14:5'- CACTCTTGAGCGCATGTGCCGTTTC -3' (SEQ ID NO: 54)

Each obtained reaction liquid was subjected to the following treatments.

In a PCR tube, 40 µL of the reaction liquid prepared in the above, 10 µL of the 5'-end FITC-labeled oligonucleotide solution, 10 µL of the counter oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂ PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 60 minutes, 50 µL of a stop solution (2N H₂ SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 13. In Solution MA and Solution MB, increase in absorbance was observed compared to Solution MC. The intensity increased depending on the concentration of the DNA fragment. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 14

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂ PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂ PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method) .

Using genomic DNA derived from human blood purchased from Clontech, the following solutions were prepared respectively in duplicate.
Solution A: Genomic DNA derived from human blood 100 ng/5 µL TE buffer solution
Solution B: Genomic DNA derived from human blood 10 ng/5 µL TE buffer solution
Solution C: Genomic DNA derived from human blood 1 ng/5 µL TE buffer solution
Solution D: TE buffer solution (negative control solution)

Five (5) µL of each obtained solution, 10 U of restriction enzyme XspI, and 2 µL of 10x buffer optimum for XspI (200 mM Tris-HCl pH 8.5, 100 mM MgCl₂, 10 mM Dithiothreitol, 1000 mM KCl) were mixed, and added with sterilized ultrapure water to prepare a reaction liquid having a liquid amount of 20 µL. The reaction liquid was incubated at 37°C for 1 hour.

Twenty (20) µL of each obtained solution, 0.5 µL of SssI methylase (available from NEB Inc.), 5 µL of 10xNEBuffer2 (available from NEB Inc.), and 0. 5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F5 having the nucleotide sequence of SEQ ID NO: 38 capable of binding by complementation with oligonucleotide Z (region corresponding to the nucleotide numbers 115-386 shown in Genbank Accession No. M80340) comprising a target DNA region of SEQ ID NO: 37 designed in LINE1 region known as human transposon, and a 0.02 µM solution in Tris-HCl buffer (10 mM) was prepared.

### <Oligonucleotide comprising target DNA region>

Z:5'-

TAGGGAGTGCCAGACAGTGGGCGCAGGCCAGTGTGTGTGCGCACCGTGCGCGAGCCGAAGCA
GGGCGAGGCATTGCCTCACCTGGGAAGCGCAAGGGGTCAGGGAGTTCCCTTTCTGAGTCAAA
GAAAGGGGTGACGGTCGCACCTGGAAAATCGGGTCACTCCCACCCGAATATTGCGCTTTTCA
GACCGGCTTAAGAAACGGCGCACCACGAGACTATATCCCACACCTGGCTCGGAGGGTCCTAC
GCCCACGGAATCTCGCTGATTGC -3′ (SEQ ID NO: 37)

### <5'-end FITC-labeled oligonucleotide>

F5:5'- ATAGTCTCGTGGTGCGCCGT -3' (SEQ ID NO: 38)

Synthesized were counter oligonucleotides C15, C16, C17, C18 and C19 having the nucleotide sequences of SEQ ID NO: 55, SEQ ID NO: 56, SEQ ID NO: 57, SEQ ID NO: 58 and SEQ ID NO: 59, respectively capable of binding by complementation with a minus strand of DNA fragment W comprising the target DNA region of SEQ ID NO: 39, and respective 0.01 µM TE buffer solutions were prepared.

### <Oligonucleotide comprising target DNA region>

Z:5′-
TAGGGAGTGCCAGACAGTGGGCGCAGGCCAGTGTGTGTGCGCACCGTGCGCGAGCCGAAGCA
GGGCGAGGCATTGCCTCACCTGGGAAGCGCAAGGGGTCAGGGAGTTCCCTTTCTGAGTCAAA
GAAAGGGGTGACGGTCGCACCTGGAAAATCGGGTCACTCCCACCCGAATATTGCGCTTTTCA
GACCGGCTTAAGAAACGGCGCACCACGAGACTATATCCCACACCTGGCTCGGAGGGTCCTAC
GCCCACGGAATCTCGCTGATTGC -3′ (SEQ ID NO: 37)

### <Counter oligonucleotides>

C15:5'- CAGTGTGTGTGCGCACCGTGCGCGAGCCGA -3' (SEQ ID NO: 55)
C16:5'- GGCGAGGCATTGCCTCACCTGGGAAGCGCA -3' (SEQ ID NO: 56)
C17:5'- GGTGACGGTCGCACCTGGAAAATCGGGTCA -3' (SEQ ID NO: 57)
C18:5'- ACCCGAATATTGCGCTTTTCAGACCGGCTT -3' (SEQ ID NO: 58)
C19:5'- TCGGAGGGTCCTACGCCCACGGAATCTCGC -3' (SEQ ID NO: 59)

Each obtained reaction liquid was subjected to the following treatments.

In a PCR tube, 40 µL of the reaction liquid prepared in the above, 10 µL of the 5'-end FITC-labeled oligonucleotide solution, 10 µL of the counter oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂ PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 9 minutes, 50 µL of a stop solution (2N H₂ SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 14. In Solution A, Solution B, and Solution C, increase in absorbance was observed compared to Solution D. The intensity increased depending on the concentration of genomic DNA. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 15

A commercially available methylcytosine antibody (available from Aviva Systems Biology) was labeled with biotin using a commercially available biotinylating kit (Biotin Labeling Kit-NH2, available from DOJINDO LABORATORIES) according to the method described in the catalogue. The obtained biotin-labeled methylcytosine antibody was refrigerated as a 0.25 µg/ µL solution in 0.1% BSA-containing phosphate buffer (1 mM KH₂ PO₄, 3 mM Na₂HPO 7H₂O, 154 mM NaCl pH7.4).

A 0.5 µg/mL solution of the synthetically obtained biotin-labeled methylcytosine antibody in 0.1% BSA-containing phosphate buffer (1 mM KH₂ PO₄, 3 mM Na₂HPO 7H₂O, 154 mM NaCl pH 7.4) was prepared, and each 100 µL of this was added to an 8-well strip coated with streptavidin (available from PerkinElmer), and left still at room temperature for about 1 hour to immobilize it to the wells. Thereafter, the solution was removed by pipetting, and 200 µL of a washing buffer [0.05% Tween20-containing phosphate buffer (1 mM KH₂ PO₄, 3 mM Na₂HPO 7H₂O, 154 mM NaCl pH7.4)] was added, and then the buffer was removed by decantation. This operation was repeated two more times (the above corresponds to preparation of an immobilized methylated DNA antibody used in the present method) .

Using genomic DNA derived from human blood purchased from Clontech, the following solutions were prepared respectively in duplicate.
Solution A: Genomic DNA derived from human blood 100 ng/5 µL TE buffer solution
Solution B: Genomic DNA derived from human blood 10 ng/5 µL TE buffer solution
Solution C: Genomic DNA derived from human blood 1 ng/5 µL TE buffer solution
Solution D: TE buffer solution (negative control solution)

Five (5) µL of each obtained solution, 4 U of restriction enzyme MspI, and 2 µL of 10x buffer optimum for MspI (100 mM Tris-HCl pH 8.5, 100 mM MgCl₂, 10 mM Dithiothreitol, 500 mM NaCl) were mixed, and added with sterilized ultrapure water to prepare a reaction liquid having a liquid amount of 20 µL. The reaction liquid was incubated at 37°C for 1 hour.

Twenty (20) µL of each obtained solution, 0.5 µL of SssI methylase (available from NEB Inc.), 5 µL of 10xNEBuffer2 (available from NEB Inc.), and 0.5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes (the foregoing process corresponds to First step of the present method).

Synthesized was 5'-end FITC-labeled oligonucleotide F6 having the nucleotide sequence of SEQ ID NO: 40 capable of binding by complementation with oligonucleotide W (region corresponding to the nucleotide numbers 178-262 shown in Genbank Accession No. AF458110) comprising a target DNA region of SEQ ID NO: 39 designed in Alu region known as human transposon, and a 0.02 µM solution in Tris-HCl buffer (10 mM) was prepared.

### <Oligonucleotide comprising target DNA region>

W:5'-

CGGGCGCGGTGGCTCACGCCTGTAATCCCAGCACTTTGGGAGGCCGAGGTGGGCGGATCACG

AGGTCAGGAGATCGAGACCATCC -3′ (SEQ ID NO: 39)

### <5'-end FITC-labeled oligonucleotide>

F6:5'- GGATGGTCTCGATCTCCTGAC -3' (SEQ ID NO: 40)

Synthesized were counter oligonucleotides C20 and C21 having the nucleotide sequences of SEQ ID NO: 60 and SEQ ID NO: 61, respectively capable of binding by complementation with a minus strand of DNA fragment W comprising the target DNA region of SEQ ID NO: 39, and respective 0.01 µM TE buffer solutions were prepared.

### <Oligonucleotide comprising target DNA region>

W:5′-

CGGGCGCGGTGGCTCACGCCTGTAATCCCAGCACTTTGGGAGGCCGAGGTGGGCGGATCACG

AGGTCAGGAGATCGAGACCATCC -3′ (SEQ ID NO: 39)

### <Counter oligonucleotides>

C20:5'- CGGGCGCGGTGGCTCACGCCTGTAATCCCA -3' (SEQ ID NO: 60)
C21:5'- TTTGGGAGGCCGAGGTGGGCGGATCACGAG -3' (SEQ ID NO: 61)

Each obtained reaction liquid was subjected to the following treatments.

In a PCR tube, 40 µL of the reaction liquid prepared in the above, 10 µL of the 5'-end FITC-labeled oligonucleotide solution, 10 µL of the counter oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the resultant mixture was added with sterilized ultra pure water to make the liquid amount 100 µL and mixed. Then the PCR tube was heated at 95°C for 10 minutes, cooled rapidly to 70°C, and retained for 10 minutes at this temperature. Then the mixture was cooled to 50°C and retained at the temperature for 10 minutes, and further retained at 37°C for 10 minutes, and returned to room temperature, to promote formation of a conjugate of the 5'-end FITC-labeled oligonucleotide and the DNA fragment (the foregoing process corresponds to Second step of the present method).

To an 8-well strip coated with streptavidin to which the biotin-labeled methylcytosine antibody has been immobilized, 100 µL of the reaction liquid of the DNA fragment prepared above was added, and left still at room temperature for 1 hour. Then the solution was removed by pipetting, and 200 µL of a washing buffer [0. 05% Tween20-containing phosphate buffer (1 mM KH₂ PO₄, 3 mM Na₂HPO 7H₂O, 154 mM NaCl pH7.4)] was added, and then the buffer was removed by pipetting. This operation was repeated two more times (the foregoing process corresponds to Third step of the present method).

Then 100 µL of a HRP-labeled FITC antibody solution [available from Jackson ImmunoResearch Laboratories, 0.005 µg/100 µL solution in 0.1% BSA-containing phosphate buffer (1 mM KH₂ PO₄, 3 mM Na₂HPO 7H₂O, 154 mM NaCl pH7.4)] was added to each well, and left still at room temperature for 1 hour. After leaving still, each well was added with 200 µL of a washing buffer [0.05% Tween20-containing phosphate buffer (1 mM KH₂ PO₄, 3 mM Na₂HPO 7H₂O, 154 mM NaCl pH7.4)], and the buffer was removed by decantation. This operation was repeated two more times.

One hundred (100) µL of a substrate (available from R&D, #DY999) was added to each well and mixed, to start the reaction. After leaving still at room temperature for about 8 minutes, 50 µL of a stop solution (2N H₂ SO₄ aqueous solution) was added to each well to stop the reaction. Within 30 minutes after stopping of the reaction, absorbance at 450 nm was measured (the foregoing process corresponds to Fourth step of the present method).

The result is shown in Fig. 15. In Solution A, Solution B, and Solution C, increase in absorbance was observed compared to Solution D. The intensity increased depending on the concentration of genomic DNA. In this experiment, it was revealed that the DNA fragment can be detected and quantified by forming and selecting a complex of the methylcytosine antibody, the methylated DNA fragment, and the immobilized 5'-end biotin-labeled oligonucleotide, and quantifying or detecting FITC in the complex by its function.

### Example 16

As a serum sample, mixed liquids of a TE buffer solution of genomic DNA derived from human blood DNA (Human Genomic DNA, #636401, Clontech) and serum collected from rat (Wistar Hannover) were prepared respectively in quadruplicate as follows.

Serum sample A: Genomic DNA derived from human blood 10 ng/10 µL TE buffer solution + rat serum 10 µL

Serum sample B: Genomic DNA derived from human blood 1 ng/10 µL TE buffer solution + rat serum 10 µL

Serum sample C: Genomic DNA derived from human blood 0.1 ng/10 µL TE buffer solution + rat serum 10 µL

Serum sample D: 0 ng/10 µL TE buffer solution + rat serum 10 µL (negative control)

For Serum samples A to D prepared in the above, Treatment 1 or Treatment 2 was conducted respectively in duplicate. Treatment 1:

Twenty (20) µL of a serum sample and 4 µL of a buffer (500 mM Tris-HCl (pH 7.5), 100 mM MgCl₂, 10 mM DTT, 1000 mM NaCl) were mixed, and the mixture was added with sterilized ultrapure water to make a liquid amount 40 µL, and mixed. Then, the PCR tube was retained at 95°C for 10 minutes, retained at 4°C for 10 minutes, and then returned to room temperature. After centrifugation at 9100xg for 10 minutes, the supernatant was collected.

### Treatment 2:

Twenty (20) µL of a serum sample and 4 µL of a buffer (330 mM Tris-Acetate (pH 7.9), 100 mM Mg(OAe)₂, 5 mM DTT, 660 mM KOAc) were mixed, and the mixture was added with sterilized ultrapure water to make a liquid amount 40 µL, and mixed. Then, the PCR tube was retained at 95°C for 10 minutes, retained at 4°C for 10 minutes, and then returned to room temperature. After centrifugation at 9100xg for 10 minutes, the supernatant was collected.

Twenty (20) µL of each solution prepared by Treatment 1 or Treatment 2, 2U of restriction enzyme MspI, and 5 µL of 10x buffer optimum for MspI (100 mM Tris-HCl pH 7.5, 100 mM MgCl₂, 10 mM Dithiothreitol, 500 mM NaCl) were mixed, and added with sterilized ultrapure water to prepare a reaction liquid having a liquid amount of 50 µL. The reaction liquid was incubated at 37°C for 1 hour.

Thirty (30) µL of the solution obtained by the above enzyme treatment, 0.5 µL of SssI methylase (available from NEB Inc.), 5 µL of 10xNEBuffer2 (available from NEB Inc.), and 0.5 µL of 3.2 mM S-adenosyl methionine (available from NEB Inc.) were mixed, and added with sterilized ultrapure water to prepare a reaction liquid having a liquid amount of 50 µL. The reaction liquid was incubated at 37°C for 30 minutes.

As a specific oligonucleotide used for obtaining a target DNA region (W, SEQ ID NO: 39, region corresponding to the nucleotide number 178-262 shown in Genbank Accession No. AF458110) designed in Alu region known as human transposon and having the nucleotide sequence of SEQ ID NO: 39, was synthesized 5'-end biotin-labeled oligonucleotide F1 comprising the nucleotide sequence of SEQ ID NO: 40 that binds by complementation with a plus strand of the target DNA region W, a 0.02 µM solution in Tris-HCl buffer (10 mM) was prepared

### <Target DNA region>

W:5'-
CGGGCGCGGTGGCTCACGCCTGTAATCCCAGCACTTTGGGAGGCCGAGGTGGGCGGATCACG
AGGTCAGGAGATCGAGACCATCC -3'(SEQ ID NO: 39)

### <5'-end FITC-labeled oligonucleotide>

F1:5'- GGATGGTCTCGATCTCCTGAC -3' (SEQ ID NO: 40)

Fifty (50) µL of the reaction liquid obtained in the above, 10 µL of the 5'-end FITC-labeled oligonucleotide solution, 10 µL of a buffer (330 mM Tris-Acetate pH 7.9, 660 mM KOAc, 100 mM MgOAc₂, 5 mM Dithiothreitol), 10 µL of a 100 mM MgCl₂ solution, 10 µL of a 1 mg/mL BSA solution were added, and the mixture was further added with sterilized ultrapure water to make a liquid amount 100 µL, and mixed. Then for forming a double strand between the target DNA region and the 5'-end FITC-labeled oligonucleotide, the PCR tube was retained at 95°C for 10 minutes, rapidly cooled to 70°C, and retained at this temperature for 10 minutes. Then the PCR tube was cooled to 50°C and retained for 10 minutes, and further retained at 37°C for 10 minutes, and returned to room temperature.

A commercially available methylcytosine antibody (available from Aviva Systems Biology) was labeled with biotin by using a commercially available biotinylating kit (Biotin Labeling Kit-NH2, available from DOJINDO LABORATORIES) according to the method described in the protocol. The obtained biotin-labeled methylcytosine antibody was refrigerated as a 0.25 µg/µL solution in 0.1% BSA-containing phosphate buffer (1 mM KH₂PO₄, 3 mM Na₂HPO 7H₂O, 154 mM NaCl pH 7.4).

100 µL of the reaction liquid obtained by the above heat treatment was added with 1 µL of a x5 diluted solution of the biotin-labeled methylcytosine antibody (0.05 µg/µL solution in 0.1% BSA-containing phosphate buffer (1 mM KH₂PO4, 3 mM Na₂HPO 7H₂O, 154 mM NaCl pH 7.4)), and left still at room temperature for 1 hour, to form a detection complex comprising the target DNA region, the 5'-end FITC-labeled oligonucleotide, and the biotin-labeled methylcytosine antibody.

The reaction liquid obtained in the above was transferred to an 8-well strip coated with streptavidin (StreptaWell, #11645692001, available from Roche), and left still at room temperature for about 60 minutes, to immobilize the detection complex comprising the target DNA region, the 5'-end FITC-labeled oligonucleotide and the biotin-labeled methylcytosine antibody via a biotin-streptavidin bond. Thereafter, the solution was removed by decantation, and each well was washed three times with 200 µL of a washing buffer [0.05%Tween20-containing phosphate buffer (1 mM KH₂PO₄, 3 mM Na₂HPO₄·7H2O, 154 mM NaCl pH 7.4)].

Thereafter, 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H2O, 154 mM NaCl pH 7.4)] was added to each well, and left still at room temperature for 1 hour. After leaving still, each well was washed with 200 µL of a washing buffer [0.05% Tween20-containing phosphate buffer (1 mM KH₂PO₄, 3 mM Na₂HPO 7H₂O, 154 mM NaCl pH 7.4)], and the buffer was removed by decantation. This operation was repeated two more time.

One hundred (100) µL of a substrate (available from R&D, #DY999) was added to each well and mixed, to start the reaction.

The reaction was left still at room temperature for about 30 minutes, and 50 µL of a stop solution (2N H₂SO₄ aqueous solution) was added to each well to stop the reaction. Within 30 minutes after stopping of the reaction, absorbance at 450 nm was measured, and an average value was calculated in duplicate for the obtained measured values.

The results are shown in Fig. 16 and Fig. 17. In Treatment 1, absorbance increased depending on the concentration in Solution A (10 ng), Solution B (1 ng), and Solution C (0.1 ng) of genomic DNA derived from human blood, compared to Solution D (0 ng: control solution) (Fig. 16). On the other hand, in Treatment 2, absorbance increased in Solution A (10 ng) of genomic DNA derived from human blood, compared to Solution D (0 ng: control solution), however, in Solution B (1 ng) and Solution C (0.1ng), increase in absorbance was not observed (Fig. 17).

In the present experiment, it was revealed that human genomic DNA in serum can be detected and quantified with high sensitivity by forming and selecting a complex of an immobilized biotin-labeled methylcytosine antibody, a methylated DNA fragment, and a 5'-end FITC-labeled oligonucleotide, and quantifying and detecting FITC in the complex according to its function. In Treatment 1, human genomic DNA in serum was detected with better sensitivity than in Treatment 2.

### Example 17

As a serum sample, mixed liquids of a TE buffer solution of genomic DNA derived from human blood (Human Genomic DNA, #636401, Clontech) and a human serum purchased from Kohjin Bio Co., Ltd (individual human serum) were prepared respectively in duplicate as follows.
Serum sample A: Genomic DNA derived from human blood 4 ng/10 µL TE buffer solution + human serum 40 µL
Serum sample B: Genomic DNA derived from human blood 2 ng/10 µL TE buffer solution + human serum 40 µL
Serum sample C: Genomic DNA derived from human blood 1 ng/10 µL TE buffer solution + human serum 40 µL
Serum sample D: Genomic DNA derived from human blood 0 ng/10 µL TE buffer solution + human serum 40 µL (negative control solution)
For Serum samples A to D prepared above, the following treatments were conducted respectively in duplicate.

In a PCR tube, 50 µL of a serum sample, and 20 µL of a buffer (500 mM Tris-HCl (pH 7.5), 100 mM MgCl₂, 10 mM DTT, 1000 mM NaCl) were charged, and the result mixture was added with sterilized ultra pure water to make the liquid amount 100 µL, and mixed. Then the PCR tube was retained at 95°C for 10 minutes, and cooled to 4°C, and then returned to room temperature. After centrifugation at 9100xg for 10 minutes, 20 µL of the supernatant was collected.

Twenty (20) µL of the solution prepared by the above treatment, 2 U of restriction enzyme MspI, and 5 µL of a 10xbuffer optimum for MspI (100 mM Tris-HCl pH 7.5, 100 mM Mg_{C}l2, 10 mM Dithiothreitol, 500 mM NaCl) were mixed, and further added with sterilized ultra pure water to make the liquid amount 50 µL. The reaction liquid was incubated at 37°C for 1 hour.

Thirty (30) µL of the solution obtained by the above enzyme treatment, 0.5 µL of SssI methylase (available from NEB Inc.), 5 µL of 10xNEBuffer2 (available from NEB Inc.), and 0.5 µL of 3.2 mM S-adenosyl methionine (available from NEB Inc.) were mixed, and added with sterilized ultra pure water to make the liquid amount 50 µL. The reaction liquid was incubated at 37°C for 30 minutes.

Synthesized was 5'-end FITC-labeled oligonucleotide F1 comprising the nucleotide sequence of SEQ ID NO: 40 capable of binding by complementation with the target DNA region (W, SEQ ID NO: 39, the region corresponding to base number 178-262 shown in Genbank Accession No. AF458110) designed in an Alu region known as human transposon, and a 0.02 µM solution in Tris-HCl buffer (10 mM) was prepared.

### <Target DNA region>

W:5′-

CGGGCGCGGTGGCTCACGCCTGTAATCCCAGCACTTTGGGAGGCCGAGGTGGGCGGATCACG
AGGTCAGGAGATCGAGACCATCC -3′ (SEQ ID NO: 39)

### <5'-end biotin-labeled oligonucleotide>

F6:5'- GGATGGTCTCGATCTCCTGAC -3' (SEQ ID NO: 40)

Fifty (50) µL of the reaction liquid obtained in the above, 10 µL of the 5'-end FITC-labeled oligonucleotide solution, 10 µL of a buffer (330 mM Tris-Acetate pH 7. 9, 660 mM KOAc, 100 mM MgOAc₂, 5 mM Dithiothreitol), 10 µL of a 100 mM MgCl₂ solution, 10 µL of a 1 mg/mL BSA solution were added, and the mixture was further added with sterilized ultrapure water to make a liquid amount 100 µL, and mixed. Then for forming a double strand between the target DNA region and the 5'-end FITC-labeled oligonucleotide, the PCR tube was retained at 95°C for 10 minutes, rapidly cooled to 70°C, and retained at this temperature for 10 minutes. Then the PCR tube was cooled to 50°C and retained for 10 minutes, and further retained at 37°C for 10 minutes, and returned to room temperature.

A commercially available methylcytosine antibody (available from Aviva Systems Biology) was labeled with biotin by using a commercially available biotinylating kit (Biotin Labeling Kit-NH2, available from DOJINDO LABORATORIES) according to the method described in the protocol. The obtained biotin-labeled methylcytosine antibody was refrigerated as a 0.25 µg/µL solution in 0.1% BSA-containing phosphate buffer (1 mM KH₂PO₄, 3 mM Na₂HPO 7H₂O, 154 mM NaCl pH 7.4).

100 µL of the reaction liquid obtained by the above heat treatment was added with 1 µL of a x5 diluted solution of the biotin-labeled methylcytosine antibody (0.05 µg/µL solution in 0.1% BSA-containing phosphate buffer (1 mM KH₂PO₄, 3 mM Na₂HPO 7H₂O, 154 mM NaCl pH 7.4)), and left still at room temperature for 1 hour, to form a detection complex comprising the target DNA region, the 5'-end FITC-labeled oligonucleotide, and the biotin-labeled methylcytosine antibody.

The reaction liquid obtained in the above was transferred to an 8-well strip coated with streptavidin (StreptaWell, #11645692001, available from Roche), and left still at room temperature for about 60 minutes, to immobilize the detection complex comprising the target DNA region, the 5'-end FITC-labeled oligonucleotide and the biotin-labeled methylcytosine antibody via a biotin-streptavidin bond. Thereafter, the solution was removed by decantation, and each well was washed three times with 200 µL of a washing buffer [0.05%Tween20-containing phosphate buffer (1 mM KH₂PO₄, 3 mM Na₂HPO₄·7H₂O, 154 mM NaCl pH 7.4)].

Thereafter, 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H2O, 154 mM NaCl pH 7.4)] was added to each well, and left still at room temperature for 1 hour. After leaving still, each well was washed with 200 µL of a washing buffer [0.05% Tween20-containing phosphate buffer (1 mM KH₂PO₄, 3 mM Na₂HPO 7H₂O, 154 mM NaCl pH 7.4)], and the buffer was removed by decantation. This operation was repeated two more time.

One hundred (100) µL of a substrate (available from R&D, #DY999) was added to each well and mixed, to start the reaction.

The reaction was left still at room temperature for about 20 minutes, and 50 µL of a stop solution (2N H₂SO₄ aqueous solution) was added to each well to stop the reaction. Within 30 minutes after stopping of the reaction, absorbance at 450 nm was measured.

The results are shown in Fig. 18. In Solution A (4 ng), Solution B (2 ng), and Solution C (1 ng) of genomic DNA derived from human blood, absorbance increased depending on the concentration, compared to Solution D (0 ng: control solution).

In the present experiment, it was revealed that human genomic DNA in human serum can be detected and quantified by forming a detection complex made up of a target DNA region, a 5'-end FITC-labeled oligonucleotide, and a biotin-labeled methylcytosine antibody, using the DNA sample extracted by the present inventive method, and selecting by immobilization via a biotin-streptavidin bond, and detecting FITC in the complex according to its function.

### Example 18

As a serum sample, the following human serums were used. Human serums purchased from Kohjin Bio Co., Ltd (individual human serums)
Lot No.:
N51438 (healthy subject)
N51439 (healthy subject)
N51441 (healthy subject)
Human serums purchased from ProMedDx (individual human serums) Lot No.:
11171268 (healthy subject, age 56, male)
11171292 (healthy subject, age 62, male)
11171297 (healthy subject, age 67, male)
11202510 (healthy subject, age 67, female)
11202522 (healthy subject, age 64, female)
11202527 (healthy subject, age 52, female)
11202615 (healthy subject, age 75, female)
11202618 (healthy subject, age 78, female)
10958886 (healthy subject, age 56, male)
10958979 (healthy subject, age 39, male)
10958980 (healthy subject, age 45, male)
10960268 (healthy subject, age 37, male)
10960272 (healthy subject, age 50, male)
10960276 (healthy subject, age 30, male)
10960285 (healthy subject, age 39, male)
11003457 (healthy subject, age 38, male)
11003479 (healthy subject, age 51, male)
11003480 (healthy subject, age 48, male)
11324997 (healthy subject, age 59, male)
11325001 (healthy subject, age 61, male)
10325022 (healthy subject, age 61, male)
10870623 (breast cancer patient, age 33, female)
10929521 (breast cancer patient, age 55, female)
10989644 (breast cancer patient, age 45, female)
11209430 (breast cancer patient, age 80, female)
10929514 (breast cancer patient, age 57, female)
10843055 (breast cancer patient, age 59, female)
10984680 (breast cancer patient, age 64, female)
10840414 (lung cancer patient, age 54, female)
10929506 (lung cancer patient, age 55, male)
11091955 (lung cancer patient, age 76, female)
11103346 (lung cancer patient, age 66, female)
11142322 (lung cancer patient, age 62, female)
11152564 (lung cancer patient, age 67, male)
11152571 (lung cancer patient, age 67, male)
11153198 (lung cancer patient, age 69, female)
11209435 (lung cancer patient, age 61, male)
11230621 (lung cancer patient, age 71, female)
11153192 (lung cancer patient, age 59, male)
10715942 (lung cancer patient, age 64, male)
10840422 (lung cancer patient, age 78, female)
10935547 (prostate cancer patient, age 83, male)
11000243 (prostate cancer patient, age 78, male)
11071226 (prostate cancer patient, age 84, male)

For the above Serum samples, the following treatments were conducted respectively in duplicate.

### Treatment 1:

In a PCR tube, 40 µL of a serum sample, and 20 µL of a buffer (500 mM Tris-HCl (pH 7.5), 100 mM MgCl₂, 10 mM DTT, 1000 mM NaCl) were charged, and the result mixture was added with sterilized ultra pure water to make the liquid amount 100 µL, and mixed. Then the PCR tube was retained at 95°C for 10 minutes, and cooled to 4°C, and then returned to room temperature. After centrifugation at 9100xg for 10 minutes, 20 µL of the supernatant was collected.

Twenty (20) µL of the solution prepared by the above treatment, 2 U of restriction enzyme MspI, and 5 µL of a 10xbuffer optimum for MspI (100 mM Tris-HCl pH 7.5, 100 mM MgCl₂, 10 mM Dithiothreitol, 500 mM NaCl) were mixed, and further added with sterilized ultra pure water to make the liquid amount 50 µL. The reaction liquid was incubated at 37°C for 1 hour.

Thirty (30) µL of the solution obtained by the above enzyme treatment, 0.5 µL of SssI methylase (available from NEB Inc.), 5 µL of l0xNEBuffer2 (available from NEB Inc.), and 0.5 µL of 3.2 mM S-adenosyl methionine (available from NEB Inc.) were mixed, and added with sterilized ultra pure water to make the liquid amount 50 µL. The reaction liquid was incubated at 37°C for 30 minutes.

Synthesized was 5'-end FITC-labeled oligonucleotide F6 comprising the nucleotide sequence of SEQ ID NO: 40 capable of binding by complementation with the target DNA region (W, SEQ ID NO: 39, the region corresponding to base number 178-262 shown in Genbank Accession No. AF458110) designed in an Alu region known as human transposon, and a 0.02 µM solution in Tris-HCl buffer (10 mM) was prepared.

### <Target DNA region>

W:5'-
CGGGCGCGGTGGCTCACGCCTGTAATCCCAGCACTTTGGGAGGCCGAGGTGGGCGGATCACG
AGGTCAGGAGATCGAGACCATCC -3' (SEQ ID NO: 39)

### <5'-end biotin-labeled oligonucleotide>

F6:5'- GGATGGTCTCGATCTCCTGAC -3' (SEQ ID NO: 40)

Fifty (50) µL of the reaction liquid obtained in the above, 10 µL of the 5'-end FITC-labeled oligonucleotide solution, 10 µL of a buffer (330 mM Tris-Acetate pH 7. 9, 660 mM KOAc, 100 mM MgOAc₂, 5 mM Dithiothreitol), 10 µL of a 100 mM MgCl₂ solution, 10 µL of a 1 mg/mL BSA solution were added, and the mixture was further added with sterilized ultrapure water to make a liquid amount 100 µL, and mixed. Then for forming a double strand between the target DNA region and the 5'-end FITC-labeled oligonucleotide, the PCR tube was retained at 95°C for 10 minutes, rapidly cooled to 70°C, and retained at this temperature for 10 minutes. Then the PCR tube was cooled to 50°C and retained for 10 minutes, and further retained at 37°C for 10 minutes, and returned to room temperature.

A commercially available methylcytosine antibody (available from Aviva Systems Biology) was labeled with biotin by using a commercially available biotinylating kit (Biotin Labeling Kit-NH2, available from DOJINDO LABORATORIES) according to the method described in the protocol. The obtained biotin-labeled methylcytosine antibody was refrigerated as a 0.25 µg/µL solution in 0.1% BSA-containing phosphate buffer (1 mM KH₂PO₄, 3 mM Na₂HPO 7H₂O, 154 mM NaCl pH 7.4).

100 µL of the reaction liquid obtained by the above heat treatment was added with 1 µL of a x5 diluted solution of the biotin-labeled methylcytosine antibody (0.05 µg/µL solution in 0.1% BSA-containing phosphate buffer (1 mM KH₂PO₄, 3 mM Na₂HPO 7H₂O, 154 mM NaCl pH 7.4)), and left still at room temperature for 1 hour, to form a detection complex comprising the target DNA region, the 5'-end FITC-labeled oligonucleotide, and the biotin-labeled methylcytosine antibody.

The reaction liquid obtained in the above was transferred to an 8-well strip coated with streptavidin (StreptaWell, #11645692001, available from Roche), and left still at room temperature for about 60 minutes, to immobilize the detection complex comprising the target DNA region, the 5'-end FITC-labeled oligonucleotide and the biotin-labeled methylcytosine antibody via a biotin-streptavidin bond. Thereafter, the solution was removed by decantation, and each well was washed three times with 200 µL of a washing buffer [0.05%Tween20-containing phosphate buffer (1 mM KH2PO₄, 3 mM Na₂HPO₄·7H₂O, 154 mM NaCl pH 7.4)].

Thereafter, 100 µL of a HRP-labeled FITC antibody solution [available from Jackson ImmunoResearch Laboratories, 0.005 µg/100 µL solution in 0.1% BSA-containing phosphate buffer (1 mM KH₂PO₄, 3 mM Na₂HPO 7H2O, 154 mM NaCl pH 7.4)] was added to each well, and left still at room temperature for 1 hour. After leaving still, each well was washed with 200 µL of a washing buffer [0.05% Tween20-containing phosphate buffer (1 mM KH₂PO₄, 3 mM Na₂HPO 7H₂O, 154 mM NaCl pH 7.4)], and the buffer was removed by decantation. This operation was repeated two more time.

One hundred (100) µL of a substrate (available from R&D, #DY999) was added to each well and mixed, to start the reaction.

The reaction was left still at room temperature for about 25 minutes, and 50 µL of a stop solution (2N H₂SO₄ aqueous solution) was added to each well to stop the reaction. Within 30 minutes after stopping of the reaction, absorbance at 450 nm was measured, and an average value was calculated in duplicate for the obtained measured values.

On the other hand, DNA in the solution obtained by the above enzyme treatment (MspI treatment) was quantified by real time PCR.

As a standard sample for measuring concentration, an MspI-treated human genomic DNA solution was prepared in the following manner. A 5 ng/µL TE buffer solution of genomic DNA derived from human blood (Human Genomic DNA, #636401, available from Clontech) in a TE buffer solution was prepared, and 20 µL of this solution, 2 U of restriction enzyme MspI, and 5 µL of a 10xbuffer optimum for MspI (100 mM Tris-HCl pH 7.5, 100 mM MgCl₂, 10 mM Dithiothreitol, 500 mM NaCl) were mixed, and added with sterilized ultra pure water to make the liquid amount 50 µL. This was prepared for each treatment. The reaction liquid was incubated at 37°C for 1 hour. For the obtained reaction liquid, 10⁻⁵, 10⁻⁴, 10⁻¹, 10⁻², 10⁻¹, 1, 10 ng/5 µL solutions were prepared by dilution with TE buffer.

For amplifying the target DNA region (W, SEQ ID NO: 39, the region corresponding to base number 178-262 shown in Genbank Accession No. AF458110) designed in an Alu region known as human transposon and quantifying it by real time PCR, Forward primer (F) comprising the nucleotide sequence of SEQ ID NO: 62 and Reverse primer (R) comprising the nucleotide sequence of SEQ ID NO: 63 were designed.

### <Target DNA region>

W:5'-

CGGGCGCGGTGGCTCACGCCTGTAATCCCAGCACTTTGGGAGGCCGAGGTGGGCGGATCACG

AGGTCAGGAGATCGAGACCATCC -3' (SEQ ID NO: 39)

### <Forward primer>

F:5'- GGTGGCTCACGCCTGTAATC -3' (SEQ ID NO: 62)

### <Reverse primer>

R:5'- GGATGGTCTCGATCTCCTGAC -3' (SEQ ID NO: 63)

As a reaction liquid of PCR, a liquid prepared by mixing 5 µL of an MspI-treated human genomic DNA solution prepared in the above which is to be a template, or a standard sample for concentration measurement prepared in the above, and each 1.5 µL of 5 µM solutions of primers comprising the nucleotide sequences of SEQ ID NO: 62 and SEQ ID NO: 63, O.lx amount of SYBR® Green I (available from Lonza), each 2. 5 µL of 2 mM dNTPs, 2. 5 µL of a 10xPCR buffer (100 mM Tris-HCl pH 8.3, 500 mM KCl, 15 mM MgCl₂, 0.01% Gelatin), and 0.125 µL of heat-resistant DNA polymerase (AmpliTaq Gold, 5 U/µL, available from ABI) and adding with sterilized ultra pure water to make the liquid amount 25 µL was used. Real-time PCR was conducted using Mx3005P (Stratagene). After retaining the reaction liquid at 95°C for 10 minutes, PCR was conducted by 40 cycles each consisting of 30 seconds at 95°C, 30 seconds at 61°C and 45 seconds at 72°C, to amplify the target DNA region. According to a result of the real-time PCR, DNA in a serum sample was quantified.

The results are shown in Fig. 19 and Fig. 20. A measured value by the present method, and a value quantified by the real-time PCR were compared, to reveal that there is a correlation (coefficient of correlation: R = 0.62)(Fig. 19). A comparison between cancer patients and healthy subjects was made for the result of quantification for human serum samples aged 57 or younger, to reveal that serum DNA concentration increased in cancer patient compared to healthy subjects (Fig. 20).

In the present experiment, it was revealed that free DNA in human serum can be detected and quantified with high sensitivity by forming and selecting a complex of a methylcytosine antibody, a methylated DNA fragment, and a 5'-end biotin-labeled oligonucleotide, and detecting the methylcytosine antibody in the complex according to its function. It was also revealed that serum DNA concentration differs between cancer patients and healthy subjects at age 57 or younger, and the difference can be detected in a simple manner by the present method.

### INDUSTRIAL APPLICABILITY

According to the present invention, it becomes possible to provide a method for quantifying or detecting DNA having a target DNA region in a simple and convenient manner. Further, it becomes possible to provide a method for selecting a specimen derived from a cancer patient by using a specimen derived from a test subject (preferably serum) and comparing a result in the specimen and a result in a specimen derived from a healthy subject, and so on.

### Free Text in Sequence Listing

### SEQ ID NOs:17 to 63

### Designed oligonucleotide

## Claims

1. A method for quantifying or detecting DNA comprising a target DNA region contained in a specimen comprising:
(1) First step of preparing from the specimen DNA for which the target DNA region is to be detected;
(2) Second step of treating the DNA prepared in First step with a DNA methylation enzyme;
(3) Third step of preparing single-stranded methylated DNA from the DNA treated in Second step, and making a detection oligonucleotide bind with the single-stranded methylated DNA to obtain a test DNA complex;
(4) Forth step of making an immobilized methylated-DNA antibody bind with the test DNA complex obtained in Third step to obtain a detection complex; and
(5) Fifth step of quantifying or detecting DNA comprising a target DNA region in the single-stranded DNA by quantifying or detecting the detection oligonucleotide contained in the detection complex obtained in Fourth step by its identification function.

2. The method according to claim 1, wherein a counter oligonucleotide is added in obtaining a test DNA complex in Third step.

3. The method according to claim 1 or 2, wherein the immobilized methylated-DNA antibody is a methylcytosine antibody.

4. The method according to any one of claims 1 to 3, wherein the DNA methylation enzyme is a cytosine methylation enzyme or SssI methylase.

5. The method according to any one of claims 1 to 4, wherein the DNA comprising a target DNA region contained in the specimen is DNA comprising a target DNA region in DNA generated from RNA by a reverse transcriptase.

6. The method according to any one of claims 1 to 5, wherein the specimen is any of the following biological specimen:
(a) mammalian blood, body fluid, excreta, body secretion, cell lysate, or tissue lysate,
(b) DNA extracted from one selected from the group consisting of mammalian blood, body fluid, excreta, body secretion, cell lysate, and tissue lysate,
(c) DNA prepared by using as a template RNA extracted from one selected from the group consisting of mammalian tissue, cell, tissue lysate and cell lysate,
(e) DNA extracted from bacterium, fungus or virus, or
(f) DNA prepared by using as a template RNA extracted from cell, fungus or virus.

7. The method according to any one of claims 1 to 6, wherein the DNA comprising a target DNA region obtained in First step is DNA digested in advance with a restriction enzyme recognition cleavage site for which is not present in the target DNA region, a synthesized oligonucleotide, or DNA purified in advance.

8. The method according to any one of claims 1 to 7, wherein the identification function of the detection oligonucleotide is any of the following identification function:
(a) fluorescence detection of FITC, or
(b) detection by FITC antibody.

9. The method according to any one of claims 1 to 8, wherein the detection oligonucleotide comprises a repetitive sequence or a nucleotide sequence of an overlapping gene or a pseudo gene in human genome or a nucleotide sequence capable of complementarily binding with a part thereof.

10. The method according to claim 9, wherein the repetitive sequence in human genome is LINE or SINE;

11. The method according to any one of claims 1 to 10, wherein the detection oligonucleotide comprises a nucleotide sequence capable of complementarily binding with any one of the following nucleotide sequences:
(1) the nucleotide sequence of SEQ ID NO: 37 or a nucleotide sequence having 80% or more sequence identity to the same,
(2) a complementary sequence of the nucleotide sequence of SEQ ID NO: 37 or a nucleotide sequence having 80% or more sequence identity to the same,
(3) the nucleotide sequence of SEQ ID NO: 39 or a nucleotide sequence having 80% or more sequence identity to the same, or
(4) a complementary sequence of the nucleotide sequence of SEQ ID NO: 39 or a nucleotide sequence having 80% or more sequence identity to the same.

12. The method according to any one of claims 1 to 10, wherein the detection oligonucleotide comprises any one of the following nucleotide sequences:
(1) the nucleotide sequence of SEQ ID NO: 38 or a nucleotide sequence having 80% or more sequence identity to the same,
(2) a complementary sequence of the nucleotide sequence of SEQ ID NO: 38 or a nucleotide sequence having 80% or more sequence identity to the same,
(3) the nucleotide sequence of SEQ ID NO: 40 or a nucleotide sequence having 80% or more sequence identity to the same, or
(4) a complementary sequence of the nucleotide sequence of SEQ ID NO: 40 or a nucleotide sequence having 80% or more sequence identity to the same.

13. The method according to any one of claims 1 to 12, wherein concentration of a sodium salt in a solution used in a DNA extracting operation for preparing DNA from a specimen in First step is 100 mM or more and 1000 mM or less.

14. The method according to any one of claims 1 to 12, wherein concentration of a sodium salt in a solution used in a DNA extracting operation for preparing DNA from a specimen in First step is 100 mM or more and 200 mM or less.

15. A method for selecting a specimen from a cancer patient comprising the step of evaluating that a specimen from a test subject is a specimen from a cancer patient when there is significant difference between a quantification result or a detection result of DNA quantified or detected by using the specimen from the test subject according to the method of any one of claims 1 to 14 and a quantification result or a detection result of DNA quantified or detected by using a specimen from a healthy subject according to the same method, and identifying a specimen from a cancer patient based on a result of the evaluation.

16. The method according to claim 15, wherein the specimen is mammalian serum.

17. The method according to claim 15 or 16, wherein DNA comprising a target DNA region is free DNA comprising the target DNA region in a mammalian serum.
